# EUROPEAN PATENT SPECIFICATION

(11) **EP 1 102 769 B1**
(45) Date of publication and mention of the grant of the patent: **16.04.2003**
(21) Application number: 99941505.2
(22) Date of filing: 03.08.1999
(51) Int. Cl.: C07D 471/04, A61K 31/437, A61K 31/55, C07D 455/04, C07D 487/04, C07D 471/14, A61P 25/00

(54) **TRICYCLIC CARBOXAMIDES**
TRICYCLISCHE CARBOXAMIDE
NOUVEAUX COMPOSES

(30) Priority: 05.08.1998 GB 9817028
(43) Date of publication of application: 30.05.2001
(73) Proprietor: SmithKline Beecham plc, Brentford, Middlesex TW8 9GS (GB)
(72) Inventor: COULTON, Steven, SmithKline Beecham Pharm., Harlow, Essex CM19 5AW (GB); NOVELLI, Riccardo, SmithKline Beecham Pharm., Harlow, Essex CM19 5AW (GB); PORTER, Roderick Alan, SmithKline Beecham Pharm., Harlow, Essex CM19 5AW (GB); THOMPSON, Mervyn, SmithKline Beecham Pharm., Harlow, Essex CM19 5AW (GB); WARD, Robert William, SmithKline Beecham Pharm., Harlow, Essex CM19 5AW (GB)
(74) Representative: West, Vivien
(86) International application number: EP9905584
(87) International publication number: WO00008020

(56) References cited:
- EP-A- 0 067 565

## Description

This invention relates to novel compounds, to processes for preparing them, and to their use as therapeutic agents.

It has now been surprisingly found that tricyclic/carboxamide compounds of formula (I) below possess anti-convulsant activity and are therefore believed to be useful in the treatment and/or prevention of anxiety, mania, depression, panic disorders and/or aggression, disorders associated with a subarachnoid haemorrhage or neural shock, the effects associated with withdrawal from substances of abuse such as cocaine, nicotine, alcohol and benzodiazepines, disorders treatable and/or preventable with anti-convulsive agents, such as epilepsy including post-traumatic epilepsy, Parkinson's disease, psychosis, migraine, cerebral ischaemia, Alzheimer's disease and other degenerative diseases such as Huntingdon's chorea, schizophrenia, obsessive compulsive disorders (OCD), neurological deficits associated with AIDS, sleep disorders (including circadian rhythm disorders, insomnia & narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, multiple sclerosis (MS) and motor neurone disease, ataxias, muscular rigidity (spasticity), temporomandibular joint dysfunction, and amyotrophic lateral sclerosis (ALS).

Accordingly, the present invention provides a compound of formula (I) or salt thereof or solvate thereof: 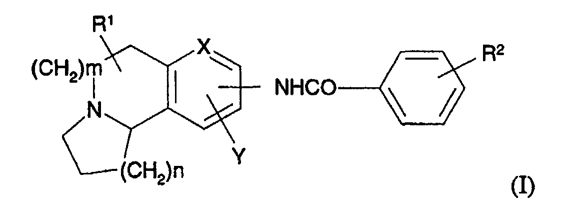 in which;
m is 1 or 2;
n is 1 or 2;
X is CH or N;
Y is selected from hydrogen, halogen, cyano, CF₃, alkyl or alkoxy
R¹, which may be at any position within the saturated ring system, is hydrogen or up to two substituents which may be the same or different and each of which is selected from fluoro and C₁₋₆-alkyl;
R² is hydrogen or up to four substituents selected from halogen, NO₂, CN, N₃, CF₃O-, CF₃S-, CF₃CO-, C₁₋₆alkyl, C₁₋₆alkenyl, C₁₋₆alkynyl,
C₁₋₆perfluoroalkyl, C₃₋₆cycloalkyl, C₃₋₆cycloalkyl-C₁₋₄alkyl-, C₁₋₆alkylO-, C₁₋₆alkylCO-, C₃₋₆cycloalkylO-, C₃₋₆cycloalkylCO-,
C₃₋₆cycloalkyl-C₁₋₄alkylO-, C₃₋₆cycloalkyl-C₁₋₄alkylCO-, phenyl, phenoxy, benzyloxy, benzoyl, phenyl-C₁₋₄alkyl-, C₁₋₆alkylS-, C₁₋₆alkylSO₂-,
(C₁₋₄alkyl)₂NSO₂-, (C₁₋₄alkyl)NHSO₂-, (C₁₋₄alkyl)₂NCO-, oxazolyl, (C₁₋₄alkyl)NHCO-, CONH₂;
or R⁴CONH-or -NR⁴R⁵
wherein R⁴ is hydrogen or C₁₋₄ alkyl, and;
R⁵ is hydrogen, C₁₋₄alkyl, formyl, -CO₂C₁₋₄alkyl or -COC₁₋₄alkyl;
or two R² groups are linked together form a carbocyclic or heterocyclic ring that is saturated or unsaturated and unsubstituted or substituted by -OH or =O.

When two R² groups are linked to form a ring, this is typically a 5-7 membered ring, so that the resultant bicyclic fused ring may be a naphthalene or an indane or indanone or indolyl ring system.

In the formula (I), alkyl groups, including alkyl groups that are part of another moiety, may be straight chain or branched. Aromatic rings, especially phenyl groups, including rings that are part of another moiety, may optionally be substituted with one or more independently selected halogen, C₁₋₆ alkyl, C₁₋₆ alkoxy or C₁₋₆ alkylcarbonyl groups. Suitable halo substituents include fluoro, chloro, iodo and bromo. Suitable C₃₋₆ cycloalkyl groups include cyclopropyl, cyclobutyl, cyclopentyl, and cyclohexyl groups.

When used herein the terms "heterocyclyl" and "heterocyclic" suitably include, unless otherwise defined, aromatic and non-aromatic, single and fused, rings suitably containing up to four heteroatoms in each ring, each of which is selected from oxygen, nitrogen and sulphur, which rings, may be unsubstituted or substituted by, for example, up to three substituents. Each heterocyclic ring suitably has from 4 to 7, preferably 5 or 6, ring atoms. A fused heterocyclic ring system may include carbocyclic rings and need include only one heterocyclic ring.

Preferably a substituent for a heterocyclyl group is selected from halogen, (C₁₋₆)alkyl, aryl(C₁₋₆)alkyl, (C₁₋₆)alkoxy, (C₁₋₆)alkoxy(C₁₋₆)alkyl, halo(C₁₋₆)alkyl, hydroxy, amino, mono- and di-N-(C₁₋₆)alkyl-amino, acylamino, carboxy, carboxy salts, carboxy esters, carbamoyl, mono- and di-N-(C₁₋₆)alkylcarbonyl, aryloxycarbonyl, (C₁₋₆)alkoxycarbonyl(C₁₋₆)alkyl, aryloxy groups, ureido, guanidino, sulphonylamino, aminosulphonyl, (C₁₋₆)alkylthio, (C₁₋₆)alkylsulphinyl, (C₁₋₆)alkylsulphonyl, heterocyclyl and heterocyclyl(C₁₋₆)alkyl.

It should be appreciated that the compounds of formula (I) have one or more chiral carbon atoms and therefore may exist as enantiomers. The present invention extends to each enantiomer and to mixtures thereof including racemates and diastereomers.

A suitable group of compounds of formula (I) have;
R¹ as hydrogen, fluoro, methyl, ethyl or propyl;
R² as hydrogen or one or more of methyl, ethyl, n-butyl, phenyl, *iso*-propyl, *t*-butyl, methoxy, ethoxy, n-propoxy, *iso*-propoxy, *n*-butoxy, phenoxy, benzyloxy, bromo, chloro, iodo, fluoro, nitro, cyano, acetyl, pivaloyl, *iso*-butyroyl, benzoyl, trifluoromethyl, trifluoromethoxy, trifluoroacetyl, amino, acetylamino, methylthio, oxazolo, methylsulfonyl, *n*-propylsulfonyl, isopropylsulfonyl or dimethylsulfamoyl;
Y is hydrogen

In a particular group of compounds of formula (I),
R¹ is hydrogen,
R² is one or more of ethyl, methoxy, isopropoxy, trifluoromethyl, cyano, chloro, fluoro.

Particularly preferred compounds are those where R² is 3-fluoro-4-methoxy, 3-fluoro-4-ethoxy or 3-fluoro-4-isopropoxy, or where R² is 3-cyano-4-methoxy, 3-cyano-4-ethoxy or 3-cyano-4-isopropoxy.

Examples of compounds of formula (I) are:
3-cyano-N-(1,2,3,5,6,10b-hexahydropyrrolo[2,1-a]isoquinolin-9-yl)-4-methoxybenzamide;
3-bromo-4-ethyl-N-( 1,2,3,5,6,10b-hexahydropyrrolo[2,1-a]isoquinolin-9-yl)benzamide;
N-(1,2,3,5,6,10b-hexahydropyrrolo[2,1-a]isoquinolin-9-yl)-4-methoxy-3-trifluoromethylbenzamide;
3-bromo-4-ethoxy-N-( 1,2,3,5,6,10b-hexahydropyrrolo[2,1-a]isoquinolin-9-yl)benzamide;
3-bromo-4-ethoxy-N-(1,3,4,6,7,11b-hexahydro-2H-pyrido[2,1-a]isoquinolin-10-yl)benzamide;
N-( 1,2,3,5,6,10b-hexahydropyrrolo[2,1-a]isoquinolin-9-yl)-4-methoxy-3-propionylbenzamide;
N-( 1,2,3,5,6,10b-hexahydropyrrolo[2,1-a]isoquinolin-9-yl)-4-ethoxy-3-propionylbenzamide;
3-acetyl-N-(1,2,3,5,6,10b-hexahydropyrrolo[2,1-a]isoquinolin-9-yl)benzamide;
3-acetyl-N-(1,2,3,5,6,10b-hexahydropyrrolo[2,1-a]isoquinolin-9-yl)-4-methoxybenzamide;
3-fluoro-N-(1,2,3,5,6,10b-hexahydropyrrolo[2,1-a]isoquinolin-9-yl)-4-methoxybenzamide;
3,5-dichloro-4-ethoxy-N-(1,2,3,5,6,10b-hexahydropyrrolo[2,1-a]isoquinolin-9-yl)benzamide;
3-acetyl-N-(1,2,3,5,6,10b-hexahydropyrrolo[2,1-a]isoquinolin-9-yl)-4-propoxybenzamide;
3-butyryl-N-(1,2,3,5,6,10b-hexahydropyrrolo[2,1-a]isoquinolin-9-yl)-4-methoxybenzamide;
3-*iso*-butyryl-N-(1,2,3,5,6,10b-hexahydropyrrolo[2,1-a]isoquinolin-9-yl)-4-*iso*propoxybenzamide;
3-cyano-N-(1,2,3,5,6,10b-hexahydropyrrolo[2,1-a]isoquinolin-9-yl)-4-*iso*propylbenzamide;
4-oxo-chroman-6-carboxylic acid (1,2,3,5,6,10b-hexahydropyrrolo[2,1-a]isoquinolin-9-yl)amide;
N-(1,2,3,5,6,10b-hexahydropyrrolo[2,1-a]isoquinolin-9-yl)-4-isopropoxy-3-propionylbenzamide;
N-(1,2,3,5,6,10b-hexahydropyrrolo[2,1-a]isoquinolin-9-yl)-3-isobutyryl-4-methoxybenzamide;
N-(1,2,3,5,6,10b-hexahydropyrrolo[2,1-a]isoquinolin-9-yl)-4-ethoxy-3-isobutyrylbenzamide;
N-( 1,2,3,5,6,10b-hexahydropyrrolo[2,1-a]isoquinolin-9-yl)-4-ethoxy-3-fluorobenzamide;
N-(1,2,3,5,6,10b-hexahydropyrrolo[2,1-a]isoquinolin-9-yl)-4-isopropoxy-3-fluorobenzamide;
3-cyano-N-( 1,2,3,5,6,10b-hexahydropyrrolo[2,1-a]isoquinolin-9-yl)-4-isopropoxybenzamide;
3-cyano-4-ethoxy-N-(1,2,3,5,6,10b-hexahydropyrrolo[2,1-a]isoquinolin-9-yl)benzamide;
N-(1,2,3,5,6,10b-hexahydropyrrolo[2,1-a]isoquinolin-9-yl)-3-propionyl-4-propoxybenzamide;
3-acetyl-4-ethyl-N-(1,2,3,5,6,10b-hexahydropyrrolo[2,1-a]isoquinolin-9-yl)benzamide;
3-acetyl-4-chloro-N-(1,2,3,5,6,10b-hexahydropyrrolo[2,1-a]isoquinolin-9-yl)benzamide;
3-acetyl-4-bromo-N-(1,2,3,5,6,10b-hexahydropyrrolo[2,1-a]isoquinolin-9-yl)benzamide;
3-acetyl-5-bromo-N-(1,2,3,5,6,10b-hexahydropyrrolo[2,1-a]isoquinolin-9-yl)benzamide;
3-cyano-4-ethoxy-N-(1,2,3,5,6,10b-hexahydropyrrolo[2,1-a]isoquinolin-9-yl)benzamide;
3-cyano-N-( 1,2,3,5,6,10b-hexahydropyrrolo[2,1-a]isoquinolin-9-yl)-4-isopropoxybenzamide;
3-acetyl-4-acetylamino-N-(1,2,3,5,6,10b-hexahydropyrrolo[2,1-a]isoquinolin-9-yl)benzamide;
3-acetyl-N-(1,2,3,5,6,10b-hexahydropyrrolo[2,1-a]isoquinolin-9-yl)-4-isopropylbenzamide;
N-( 1,2,3,5,6,10b-hexahydropyrrolo[2,1-a]isoquinolin-9-yl)-3-propionyl-4-isopropoxybenzamide;
3-acetyl-4-ethoxy-N-( 1,2,3,5,6,10b-hexahydropyrrolo[2,1-a]isoquinolin-9-yl)benzamide;
3-cyano-N-( 1,2,3,5,6,10b-hexahydropyrrolo[2,1-a]isoquinolin-9-yl)-4-methoxybenzamide;
N-( 1,2,3,5,6,10b-hexahydropyrrolo[2,1-a]isoquinolin-9-yl)-4-methoxy-3-trifluoromethylbenzamide;
3-fluoro-N-(1,2,3,5,6,10b-hexahydropyrrolo[2,1-a]isoquinolin-9-yl)-4-methoxybenzamide;
4-ethoxy-3-fluoro-N- (1,2,3,5,6,10b-hexahydropyrrolo[2,1-a]isoquinolin-9-yl)benzamide;
3-butyryl-N-(1,2,3,5,6,10b-hexahydropyrrolo[2,1-a]isoquinolin-9-yl)-4-propoxybenzamide;
3-butyryl-N-(1,2,3,5,6,10b-hexahydropyrrolo[2,1-a]isoquinolin-9-yl)-4-*iso*propoxybenzamide;
3-acetyl-N-(1,2,3,5,6,10b-hexahydropyrrolo[2,1-a]isoquinolin-9-yl)-4-propoxybenzamide;
3-butyryl-4-ethoxy-N-(1,2,3,5,6,10b-hexahydropyrrolo[2,1-a]isoquinolin-9-yl)benzamide;
3-*iso*-butyryl-N-(1,2,3,5,6,10b-hexahydropyrrolo[2,1-a]isoquinolin-9-yl)-4-propoxybenzamide;
3-acetyl-N-(1,2,3,5,6,10b-hexahydropyrrolo[2,1-a]isoquinolin-9-yl)-4-*iso*propoxybenzamide;
3-chloro-N-( 1,2,3,5,6,10b-hexahydropyrrolo[2,1-a]isoquinolin-9-yl)-4-*iso*propoxybenzamide;
3-cyano-N-(1,2,3,5,6,10b-hexahydropyrrolo[2,1-a]isoquinolin-9-yl)-4-*iso*propylbenzamide;
3-bromo-N-(1,2,3,5,6,10b-hexahydropyrrolo[2,1-a]isoquinolin-9-yl)-4-*iso*propoxybenzamide;
5-acetyl-N-( 1,2,3,5,6,10b-hexahydropyrrolo[2,1-a]isoquinolin-9-yl)-2-methoxy-4-isopropoxybenzamide;
N-(1,2,3,5,6,10b-hexahydropyrrolo[2,1-a]isoquinolin-9-yl)-3-pivaloylbenzamide;
N-(1,2,3,5,6,10b-hexahydropyrrolo[2,1-a]isoquinolin-9-yl)-2-methoxy-4-isopropyl-5-trifluoromethylbenzamide;
naphthalene-2-carboxylic acid (1,2,3,5,6,10b-hexahydropyrrolo[2,1-a]isoquinolin-9-yl)amide;
benzothiazole-5-carboxylic acid (1,2,3,5,6,10b-hexahydropyrrolo[2,1-a]isoquinolin-9-yl)amide;
2,3-dihydrobenzofuran-5-carboxylic acid (1,2,3,5,6,10b-hexahydropyrrolo[2,1-a]isoquinolin-9-yl)carboxamide;
3-acetyl-N-(1,2,3,5,6,10b-hexahydropyrrolo[2,1-a]isoquinolin-9-yl)-4-isopropoxybenzamide;
3-chloro-4-ethoxy-N-(1,2,3,5,6,10b-hexahydropyrrolo[2,1-a]isoquinolin-9-yl)benzamide;
N-3-(N, N-dimethylcarboxamido)-(1,2,3,5,6,10b-hexahydropyrrolo[2,1-a]isoquinolin-9-yl)-4-isopropoxybenzamide;
N-(6,6-dimethyl-1,2,3,5,6,10b-hexahydropyrrolo[2,1-a]isoquinolin-9-yl)-4-methoxy-3-trifluoromethylbenzamide;
3-bromo-N-(6,6-dimethyl-1,2,3,5,6,10b-hexahydropyrrolo[2,1-a]isoquinolin-9-yl)-4-ethoxybenzamide;
3-bromo-N-(6,6-dimethyl-1,2,3,5,6,10b-hexahydropyrrolo[2,1-a]isoquinolin-9-yl)-4-*iso*propoxybenzamide;
3-cyano-N-(6,6-dimethyl-1,2,3,5,6,10b-hexahydropyrrolo[2,1-a]isoquinolin-9-yl)-4-*iso*propylbenzamide;
3-acetyl-N- (6,6-dimethyl-1,2,3,5,6,10b-hexahydropyrrolo[2,1-a]isoquinolin-9-yl)-4-*iso*propoxybenzamide;
N-(6,6-dimethyl-1,2,3,5,6,10b-hexahydropyrrolo[2,1-a]isoquinolin-9-yl)-3-fluoro-4-methoxybenzamide;
N-(6,6-dimethyl-1,2,3,5,6,10b-hexahydropyrrolo[2,1-a]isoquinolin-9-yl)-3-fluoro-4-ethoxybenzamide;
N-(6,6-dimethyl-1,2,3,5,6,10b-hexahydropyrrolo[2,1-a]isoquinolin-9-yl)-3-fluoro-4-*iso*propoxybenzamide;
3-cyano-N-(6,6-dimethyl-1,2,3,5,6,10b-hexahydropyrrolo[2,1-a]isoquinolin-9-yl)-4-methoxybenzamide;
3-acetyl-N-(6,6-dimethyl-1,2,3,5,6,10b-hexahydropyrrolo[2,1-a]isoquinolin-9-yl)-4-methoxybenzamide;
N-(6,6-dimethyl-1,2,3,5,6,10b-hexahydropyrrolo[2,1-a]isoquinolin-9-yl)-3-fluoro-4-methoxybenzamide;
N-(7-chloro-1,2,3,5,6,10b-hexahydropyrrolo[2,1-a]isoquinolin-9-yl)-4-methoxy-3-trifluoromethylbenzamide;
10b-methyl-N-(1,2,3,5,6,10b-hexahydropyrrolo[2,1-a]isoquinolin-9-yl)-4-methoxy-3-trifluoromethylbenzamide;
3-bromo-N-4-ethoxy(6-methyl-1,2,3,5,6,10b-hexahydropyrrolo[2,1-a]isoquinolin-9-yl)-4-*iso*propoxybenzamide;
N-(6-methyl-1,2,3,5,6,10b-hexahydropyrrolo[2,1-a]isoquinolin-9-yl)-4-methoxy-3-trifluoromethylbenzamide;
N-(5-methyl-1,2,3,5,6,10b-hexahydropyrrolo[2,1-a]isoquinolin-9-yl)-4-methoxy-3-trifluoromethylbenzamide;
3-acetyl-N-(5-methyl-1,2,3,5,6,10b-hexahydropyrrolo[2,1-a]isoquinolin-9-yl)-4-isopropoxybenzamide;
3-acetyl-N-(2,3,5,6,7,11b-hexahydro-1H-benzo[c]pyrrolo[1,2-a]azepin-10-yl)-4-isopropoxybenzamide;
N-(2,3,5,6,7,11b-hexahydro-1H-benzo[c]pyrrolo[1,2-a]azepin-10-yl)-4-methoxy-3-trifluoromethylbenzamide;
4*-tert*-butyl-2-methoxy-N-(1,2,3,5,6,10b-hexahydropyrrolo[2,1-a]isoquinolin-7-yl)benzamide
5-cyano-2-ethoxy-4-*iso*propyl-N-(2,3,5,6,6a,10a,10b-octahydro-1H-pyrrolo[2,1-a]isoquinolin-7-yl)benzamide, and;
N-(5,6,8,9,10,10a-hexahydropyrrolo[2,1-f][1,6]naphthridin-2-yl)-4-methoxy-3-trifluoromethylbenzamide.

When synthesised, these compounds may be isolated in salt form, such as the hydrochloride or trifluoroacetate, and such salts also form part of this invention. Such salts may be used in preparing pharmaceutically acceptable salts. The compounds and their salts may be obtained as solvates, such as hydrates, and these also form part of this invention.

The above compounds and pharmaceutically acceptable salts thereof, especially the hydrochloride, and pharmaceutically acceptable solvates, especially hydrates, form a preferred aspect of the present invention.

The administration of such compounds to a mammal may be by way of oral, parenteral, sub-lingual, nasal, rectal or transdermal administration.

An amount effective to treat the disorders hereinbefore described depends on the usual factors such as the nature and severity of the disorders being treated and the weight of the mammal. However, a unit dose will normally contain 1 to 1000 mg, suitably 1 to 500 mg, for example an amount in the range of from 2 to 400 mg such as 2, 5, 10, 20, 30, 40, 50, 100, 200, 300 and 400 mg of the active compound. Unit doses will normally be administered once or more than once per day, for example 1, 2, 3, 4, 5 or 6 times a day, more usually 1 to 4 times a day, such that the total daily dose is normally in the range, for a 70 kg adult of 1 to 1000 mg, for example 1 to 500 mg, that is in the range of approximately 0.01 to 15 mg/kg/day, more usually 0.1 to 6 mg/kg/day, for example 1 to 6 mg/kg/day.

It is greatly preferred that the compound of formula (I) is administered in the form of a unit-dose composition, such as a unit dose oral, including sub-lingual, rectal, topical or parenteral (especially intravenous) composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colorants, flavourings, and wetting agents. The tablets may be coated according to well known methods in the art. Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl *p*-hydroxybenzoate or sorbic acid, and if desired conventional flavouring or colouring agents. Oral formulations also include conventional sustained release formulations, such as tablets or granules having an enteric coating.

For parenteral administration, fluid unit dose forms are prepared containing the compound and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing.
Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

As is common practice, the compositions will usually be accompanied by written or printed directions for use in the medical treatment concerned.

Accordingly, the present invention further provides a pharmaceutical composition for use in the treatment and/or prophylaxis of anxiety, mania, depression, panic disorders and/or aggression, disorders associated with a subarachnoid haemorrhage or neural shock, the effects associated with withdrawal from substances of abuse such as cocaine, nicotine, alcohol and benzodiazepines, disorders treatable and/or preventable with anti-convulsive agents, such as epilepsy including post-traumatic epilepsy, Parkinson's disease, psychosis, migraine, cerebral ischaemia, Alzheimer's disease and other degenerative diseases such as Huntingdon's chorea, schizophrenia, obsessive compulsive disorders (OCD), neurological deficits associated with AIDS, sleep disorders (including circadian rhythm disorders, insomnia & narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, multiple sclerosis (MS) and motor neurone disease, ataxias, muscular rigidity (spasticity), temporomandibular joint dysfunction, and amyotrophic lateral sclerosis (ALS) which comprises a compound of formula (I), or a pharmaceutically acceptable salt or solvate thereof, and a pharmaceutically acceptable carrier.

The present invention also provides a method of treatment and/or prophylaxis of anxiety, mania, depression, panic disorders and/or aggression, disorders associated with a subarachnoid haemorrhage or neural shock, the effects associated with withdrawal from substances of abuse such as cocaine, nicotine, alcohol and benzodiazepines, disorders treatable and/or preventable with anti-convulsive agents, such as epilepsy including post-traumatic epilepsy, Parkinson's disease, psychosis, migraine, cerebral ischaemia, Alzheimer's disease and other degenerative diseases such as Huntingdon's chorea, schizophrenia, obsessive compulsive disorders (OCD), neurological deficits associated with AIDS, sleep disorders (including circadian rhythm disorders, insomnia & narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, multiple sclerosis (MS) and motor neurone disease, ataxias, muscular rigidity (spasticity), temporomandibular joint dysfunction, and amyotrophic lateral sclerosis (ALS), comprising administering to the sufferer in need thereof an effective or prophylactic amount of a compound of formula (I), or a pharmaceutically acceptable salt or solvate thereof.

In a further aspect the invention provides the use of a compound of formula (I), or a pharmaceutically acceptable salt or solvate thereof, for the manufacture of a medicament for the treatment and/or prophylaxis of anxiety, mania, depression, panic disorders and/or aggression, disorders associated with a subarachnoid haemorrhage or neural shock, the effects associated with withdrawal from substances of abuse such as cocaine, nicotine, alcohol and benzodiazepines, disorders treatable and/or preventable with anti-convulsive agents, such as epilepsy including post-traumatic epilepsy, Parkinson's disease, psychosis, migraine, cerebral ischaemia, Alzheimer's disease and other degenerative diseases such as Huntingdon's chorea, schizophrenia, obsessive compulsive disorders (OCD), neurological deficits associated with AIDS, sleep disorders (including circadian rhythm disorders, insomnia & narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, multiple sclerosis (MS) and motor neurone disease, ataxias, muscular rigidity (spasticity), temporomandibular joint dysfunction, and amyotrophic lateral sclerosis (ALS).

In a further aspect the invention provides the use of a compound of formula (I), or a pharmaceutically acceptable salt or solvate, thereof as a therapeutic agent, in particular for the treatment and/or prophylaxis of anxiety, mania, depression, panic disorders and/or aggression, disorders associated with a subarachnoid haemorrhage or neural shock, the effects associated with withdrawal from substances of abuse such as cocaine, nicotine, alcohol and benzodiazepines, disorders treatable and/or preventable with anti-convulsive agents, such as epilepsy including post-traumatic epilepsy, Parkinson's disease, psychosis, migraine, cerebral ischaemia, Alzheimer's disease and other degenerative diseases such as Huntingdon's chorea, schizophrenia, obsessive compulsive disorders (OCD), neurological deficits associated with AIDS, sleep disorders (including circadian rhythm disorders, insomnia & narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, multiple sclerosis (MS) and motor neurone disease, ataxias, muscular rigidity (spasticity), temporomandibular joint dysfunction, and amyotrophic lateral sclerosis (ALS).

The present invention also provides a process for the preparation of compounds of formula (I), or salts thereof or solvates thereof, which comprises reacting a compound of formula (II) 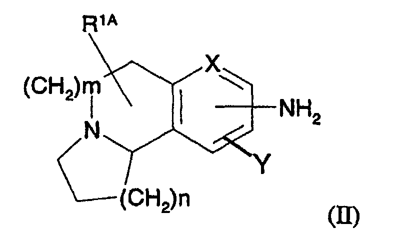 with a compound of formula (III) 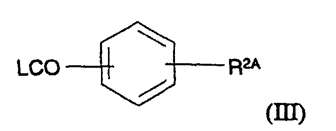 where R^{1A} and R^{2A} are R¹ and R² respectively as defined for formula (I) or a group or groups convertible to R¹ or R² groups; and L is OH, acyloxy, or a halogen,
and where required;
converting an R^{1A} or R^{2A} group to an R¹ or R² group;
converting one R¹ or R² group to another R¹ or R² group;
converting a salt product to the free base or another pharmaceutically acceptable salt;
or converting a free base product to a pharmaceutically acceptable salt.

Conventional conditions for condensation of amines with carboxylic acids or active derivatives thereof, such as acid chlorides, may be used. For example the amines and acids may be reacted in the presence of a mixture of ethyl(dimethylaminopropyl)-carbodiimide/hydroxybenzotriazole in a suitable solvent such as dimethyl formamide, and amines and acid chlorides may be reacted together in a suitable solvent such as ethyl acetate or tetrahydrofuran. Alternatively the acid may be treated in solution with oxalyl chloride and then reacted with the amine or its hydrochloride.

Reaction of a compound of formula (III) which is an acid chloride (L=Cl) with a compound of formula (II) will lead, in the absence of a suitable base such as triethylamine, to formation of the hydrochloride salt of the compound of formula (I). Hydrochloride salts can also be obtained by passing HCl gas into a solution of the free base, or adding a solution of HCl in ether.

Conversions of an R^{1A} or R^{2A} group to an R¹ or R² group typically arise when a protecting group is needed during the above coupling reaction or during the preparation of the reactants by the procedures described below.

Interconversion of one R¹ or R² group to another typically arises when one compound of formula (I) is used as the precursor of another compound of formula (I) or when it is easier to introduce a more complex or reactive substituent at the end of a synthetic sequence.

The compounds of formula (II) have chiral carbon atoms and therefore may exist as enantiomers. Accordingly the above process may produce compounds of formula (I) that are racemic mixtures. These mixtures may be separated or resolved by conventional procedures if individual enantiomers are required. Alternatively the starting materials may be selected to achieve a stereospecific reaction.

Compounds of formula (II) may be prepared from the corresponding desamino analogues, firstly forming a nitro compound and then hydrogenating the nitro group to the amine. The nitro group may be introduced by treating the desamino compound with concentrated sulfuric acid and adding potassium nitrate. Hydrogenation of the nitro compound may be carried out by reaction with hydrogen at 50 psi in the presence of palladium/charcoal in a suitable solvent such as ethanol.

Hexahydro-pyrido-isoquinoline starting materials may be prepared by analogous methods to those described in J. Pharm Bull, 1960, 8, 14.

Hexahydro-pyrrolo-isoquinolinylamines may be prepared by analogy to the methods disclosed in International Application Publication Number WO 97/17344 (Astra Aktiebolag).

Substituted phenyl compounds of formula (III) may be prepared by further substitution of commercially available benzoic acid derivatives using conventional procedures, or by oxidation of corresponding substituted benzyl alcohols.

Where the above described intermediates are novel compounds, they also form part of this invention.

The preparation of compounds of formulae (II) is illustrated by the following **Descriptions**; the preparation of compounds of this invention is illustrated by the following **Examples**. The utility of compounds of this invention is shown by the **Pharmacological Data** that follow the Examples.

### Description 1

### (+/-)-10-vitro-1,3,4,6,7,11b-hexahydro-2H-pyrido[2,1-a]isoquinoline

(+/-)-1,3,4,6,7,11b-hexahydro-2H-pyrido[2,1-a]isoquinoline (1.0g) in conc sulphuric acid at -10°C was stirred for 1h. Potassium nitrate (0.54g) was added portionwise over 15min. The reaction was stirred at -10°C overnight warmed to 0°C stirred 1h and quenched with ice/water (100g/100ml). The mixture was neutralised with ammonia, extracted with ethyl acetate (3 x 30ml), the combined organics dried (MgSO4) and solvent removed at reduced pressure. The residue was column chromatographed (silica gel, 98% diethyl ether/methanol) to give the title compound (0.36g).
MS m/_{z} (API): 232 (MH⁺; 100%)

### Description 2

### (+/-)-1,3,4,6,7,11b-hexahydro-2H-pyrido[2,1-a]isoquinolin-10-ylamine

A solution of (+/-)-10-nitro-1,3,4,6,7,11b-hexahydro-2H-pyrido[2,1-a]isoquinoline (0.36g) in ethanol (20ml) and palladium on charcoal (5% w/w, 0.1g) was hydrogenated at 50psi at room temperature for 2h. The reaction mixture was filtered through a celite pad and the filtrate evaporated to dryness to give the title compound (0.3g) as an oil.
MS m/_{z} (API): 202 (MH⁺; 100%).

### Description 3

### (+/-)-7-nitro-1,2,3,5,6,10b-hexahydropyrrolo[2,1-a]isoquinoline and (+/-)-9-nitro-1,2,3,5,6,10b-hexahydropyrrolo[2,1-a]isoquinoline

From the nitration of 1,2,3,5,6,10b-hexahydropyrrolo[2,1-a]isoquinoline (3.5g) according to the method of WO 97/17344 (+/-)-7-nitro-1,2,3,5,6,10b-hexahydropyrrolo[2,1-a]isoquinoline (0.35g) and (+/-)-9-nitro-1,2,3,5,6,10b-hexahydropyrrolo[2,1-a]isoquinoline (3.6g) were isolated by column chromatography (silica gel, 10% methanol:diethyl ether).
Characterisation of (+/-)-7-nitro-1,2,3,5,6,10b-hexahydropyrrolo[2,1-a]isoquinoline (D3a)
¹H NMR (400MHz, CDCl₃) δ:1.70-1.81 (1H, m), 1.86 - 2.05 (2H, m), 2.37 - 2.43 (1H, m), 2.52 - 2.64 (2H, m), 3.07 - 3.47 (5H, m), 7.24 - 7.35 (2H, m) and 7.77 (1H, d, 7.16Hz).
Characterisation of (+/-)-9-nitro-1,2,3,5,6,10b-hexahydropyrrolo[2,1-a]isoquinoline (D3b)
¹H NMR (250MHz, CDCl₃) δ:1.72 - 2.01 (3H, m), 2.47 - 2.70 (3H, m), 2.90 - 3.10 (1H, m), 3.10 - 3.32 (3H, m), 3.39 (1H, t), 7.27 (1H, d, J = 8.3Hz), 7.95 (1H, s) and 7.99 (1H, dd, J = 2.3, 8.3Hz)
m/_{z} (API): 219 (MH⁺; 100%).

### Description 4

### (+/-)-1,2,3,5,6,10b-hexahydropyrrolo[2,1-a]isoquinolin-7-ylamine

From (+/-)-7-nitro-1,2,3,5,6,10b-hexahydropyrrolo[2,1-a]isoquinoline (D3a) (0.30g) the title compound (0.22g) was prepared according to the method of Description 2.
¹H NMR (250MHz, CDCl₃) δ: 1.67 - 2.00 (3H, m), 2.27 - 2.82 (5H, m), 3.12 (1H, dt, J = 2.62 and 7.99Hz), 3.25 - 3.34 (2H, m), 6.54 (2H, d, J = 7.75Hz) and 6.98 (1H, t, J = 7.69).

### Description 5

### (+/-)-1,2,3,5,6,10b-hexahydropyrrolo[2,1-a]isoquinolin-9-ylamine

From (+/-)-9-nitro-1,2,3,5,6,10b-hexahydropyrrolo[2,1-a]isoquinoline (D3b) (4.0g) the title compound (3.5g) was prepared according to the method of Description 2.
¹H NMR (250MHz, CDCl₃) 1.63 - 2.03 (3H, m), 2.23 - 2.38 (1H, m), 2.49 - 2.78 (3H, m), 2.93 - 3.24 (3H, m), 3.39 (1H, t), 6.42 (1H, d, J = 2.3Hz), 6.50 (1H, dd, J = 2.4, 7.9Hz) and 6.90 (1H, d, J = 7.9Hz).
MS m/_{z} (API): 189 (MH⁺; 100%).

### Description 6

### Ethyl 4-isobutyryloxybenzoate

*Iso*butyryl chloride (19.235g) was added to a mixture of ethyl 4-hydroxybenzoate (30.0g) and triethylamine (35ml) in tetrahydrofuran (250ml) over 10min. The mixture was stirred for 2h, filtered and solvent removed from the filtrate at reduced pressure to give the title compound (42.5g)
¹H NMR (250MHz, CDCl₃) δ 1.32 (6H, d, J = 7.0Hz), 1.39 (3H, t, J = 7.2Hz), 2.82 (1H, septet, J = 7.0Hz), 4.37(2H, q, J = 7.0Hz), 7.16 (2H, d, J = 8.7Hz) and 8.07 (2H, d, J = 8.7Hz).

### Description 7

### Ethyl 3-isobutyryl-4-hydroxybenzoate

Ethyl 4-*iso*butyryloxybenzoate (44.0g) was treated with aluminium chloride (27.0g) and heated at 160°C for 1h. After cooling to room temperature ice/water (∼200g) was added and the mixture stirred for 2h. The precipitated solid was separated by filtration, dried *in vacuo*, added to ethanol (250ml) containing conc. sulphuric acid (18ml) and the mixture refluxed through 4A molecular sieves for 48h. The reaction mixture was cooled to room temperature, filtered (celite pad) and solvent removed at reduced pressure. The residue was dissolved in dichloromethane and washed with aqueous sodium hydrogen carbonate, the organic phase dried (MgSO₄) and solvent removed at reduced pressure. The residue was column chromatographed (silica gel, dichloromethane/petroleum ether mixtures) to give the title compound (7.5g)
¹H NMR (250MHz, CDCl₃) 8 1.25 (6H, d, J = 6.8Hz), 1.38(3H, t, J = 6.9Hz), 3.72 (1H, septet, J = 6.8Hz), 4.38 (2H, q, J = 6.9Hz), 7.02 (1H, d, J = 8.8Hz), 8.13 (1H, dd, J = 2.0, 8.8Hz) and 8.54 (1H, d, J = 2.0Hz).

### Description 8

### Ethyl 3-isobutyryl-4-propoxybenzoate

Ethyl 3-*iso*butyryl-4-hydroxybenzoate (1.2g), 1-iodopropane (1.87g) and potassium carbonate (1.3g) were combined in dimethylformamide (10ml) and stirred for 16h. The mixture was diluted with dichloromethane and washed with water and brine, the organic phase was dried (MgSO₄) and solvent removed at reduced pressure to give the title compound (1.4g)
¹H NMR (250MHz, CDCl₃) δ1.04 (3H, t, J = 7.3Hz), 1.16 (6H, d, J = 6.9Hz), 1.38 (3H, t, J = 7.2Hz), 1.86 (2H, m), 3.48 (1H, septet, J = 6.9Hz), 4.07 (2H, t, J = 6.4Hz), 4.36 (2H, q, J = 7.2Hz), 6.96 (1H, d, J = 8.7Hz), 8.10 (1H, dd, J = 2.3, 8.7Hz) and 8.16 (1H, d, J = 2.3Hz)

### Description 9

### 3-iso-Butyryl-4-propoxybenzoic acid

Ethyl 3-*iso*-butyryl-4-propoxybenzoate (1.4g) was added to methanol (50ml) containing 2N aqueous sodium hydroxide (30ml). The mixture was stirred for 15min at 50°C, solvent removed and water (50ml) added followed by 2N hydrochloric acid (40ml). The mixture was extracted with ethyl acetate and the combined organic extracts evaporated to dryness to give the title compound (1.1g) as an oil.
¹H NMR (250MHz, CDCl₃) δ 1.00 (3H, t, J = 7.4Hz), 1.08 (6H, d, J = 6.9Hz), 1.80 (2H, m), 3.45 (1H, septet, J = 6.9Hz), 4.13 (2H, t, J = 6.3Hz), 7.23 (1H, d, J = 8.8Hz), 7.97 (1H, d, J = 2.3Hz), 8.05 (1H, dd, J = 8.8, 2.3Hz).

### Description 10

### Ethyl 3-isobutyryl-4-isopropoxybenzoate

The title compound (1.4g) was prepared from compound D7 (1.2g) and *iso*propyl iodide according to the method of Description 8.
¹H NMR (250MHz, CDCl₃) δ 1.16 (6H, d, J = 6.9Hz), 1.38 (3H, t, J = 7.2Hz), 1.49 (6H, d, J = 6.1Hz), 3.45 (1H, septet, J = 6.9Hz), 4.35 (2H, q, J = 7.2Hz), 4.74 (1H, septet, J = 6.1Hz), 6.95 (1H, d, J = 8.8Hz), 8.08 (1H, dd, J = 2.3, 8.8Hz) and 8.15 (1H, d, J = 2.3Hz).

### Description 11

### 3-isoButyryl-4-isopropoxybenzoic acid

The title compound (1.1g) was prepared from D10 (1.4g) according to the method of Description 9
¹H NMR (250MHz, CDCl₃) δ 1.17 (6H, d, J = 6.9Hz), 1.41 (6H, d, J = 6.0Hz), 3.44 (1H, septet, J = 6.9Hz), 4.77 (1H, septet, J = 6.0Hz), 6.98 (1H, d, J = 8.9Hz), 8.14 (1H, dd, J = 2.2, 8.7Hz) and 8.22 (1H, d, J = 2.2Hz).

### Description 12

### (+)-9-nitro-1,2,3,5,6,10b-hexahydropyrrolo[2,1-a]isoquinoline and (-)-9-nitro-1,2,3,5,6,10b-hexahydropyrrolo[2,1-a]isoquinoline.

(+/-)-9-nitro-1,2,3,5,6,10b-hexahydropyrrolo[2,1-a]isoquinoline (D3b) (50g) was separated into the two enantiomers by simulated moving bed chromatography using eight columns packed with 30g of Chiralpak AD and 10% ethanol in hexane (containing 0.1% diethylamine) as the eluant with the following system parameters: recycle flow rate = 101.64ml/min, feed = 1.04ml/min, eluent = 20.21ml/min, raffinate = 5.78ml/min, extract = 15.48ml/min, feed concentration = 9g/l, switch period = 1.18min. 19g of each enantiomer (e.e. > 95%) was obtained. First eluting component (+)-9-nitro-1,2,3,5,6,10b-hexahydropyrrolo[2,1-a]isoquinoline (D12a)
Second eluting component (-)-9-nitro-1,2,3,5,6,10b-hexahydropyrrolo[2,1-a]isoquinoline (D12b)

### Description 13

### (-)-1,2,3,5,6,10b-hexahydropyrrolo[2,1-a]isoquinolin-9-ylamine

From (-)-9-nitro-1,2,3,5,6,10b-hexahydropyrrolo[2,1-a]isoquinoline (D12b) (0.5g) the title compound (0.454g) was prepared according to the method of Description 2 using 5%pd/C (0.2g) and hydrogenating for 45min at room temperature.. Spectral data identical to the compound of Description 5.
[α]_{D}²⁵ -111° (c 1.0, MeOH).

### Desciption 14

### (+)-1,2,3,5,6,10b-hexahydropyrrolo[2,1-a]isoquinolin-9-ylamine

From (+)-9-nitro-1,2,3,5,6,10b-hexahydropyrrolo[2,1-a]isoquinoline (D12a) (0.25g) the title compound (0.183g) was prepared according to the method of Description 2 using 5%pd/C (0.15g) and hydrogenating for 45min at room temperature..
Spectral data identical to the compound of Description 5.
[α]_{D}²⁵ +123° (c 0.5, MeOH).

### Description 15

### Dimethylphenylacetonitrile

Phenylacetonitrile (11.7g) was dissolved in dimethyl sulphoxide/water (96ml 80:16). Sodium hydroxide (16.0g) was added to the rapidly stirred mixture. Iodomethane (25ml) was added over 30 min (exotherm). The mixture was stirred for 1h, partitioned between diethyl ether:water and the ether layer separated. The ether layer was washed with water and brine, dried (MgSO₄) and solvent removed at reduced pressure to give the title compound (13.8g).
¹H NMR (250MHz, CDCl₃) δ 1.73 (6H, s), 7.29 - 7.50 (5H, m).

### Description 16

### 4-Chloro-N-2-methyl-2-phenylpropane

Lithium aluminium hydride (5.42g) was stirred in diethyl ether (250ml). Dimethylphenylacetonitrile (13.8g) in diethyl ether (50ml) was added over 30 min at room temperature. When the addition was complete the reaction mixture was warmed to reflux for 3h, cooled (ice bath) and wet tetrahydrofuran CAREFULLY added. The mixture was subsequently quenched with water and 2N sodium hydroxide(11ml). The suspension was stirred for 30min, filtered, the organic phase isolated and dried (MgSO4). Solvent was removed at reduced pressure to give the title compound (13.8g) as an oil.
m/z (API): 150 (MH+; 100%).

### Description 17

### 4-Chloro-N-(2-methyl-2-phenylpropyl)butyramide

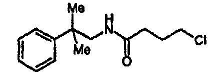

4-Chloro-N-2-methyl-2-phenylpropane (D16) in tetrahydrofuran (250ml) containing triethylamine (12.88ml) was treated with 4-chlorobutyryl chloride (10.37ml) with stirring. After stirring overnight, the reaction mixture was partitioned between diethyl ether/water, the organic phase separated washed with water, dried (MgSO₄) and solvent removed at reduced pressure to give the title compound (23.08g) as an oil.
m/z (API): 254, 256 (MH⁺; 100%)

### Description 18

### 6,6-Dimethyl-2,3,5,6-tetrahydro-1H-pyrrolo[2,1-a]isoquinolinium nitrate

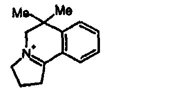

The amide D17 (10.0g) in xylene (200ml) was treated with phosphorous pentoxide (25g) and phosphorous oxychloride (25g) subsequently added carefully. The mixture was boiled for 7h. cooled and the solvent decanted. The residue was dissolved in water, acidified with conc. HCl and the aqueous phase washed with toluene. The aqueous phase was basified with excess potassium carbonate, washed with toluene and treated with potassium iodide (20g). The aqueous mixture was extracted with dichloromethane (x2), the combined organic phase dried (MgSO4) and solvent removed at reduced pressure to give after trituration with acetone the title compound as a brown solid (8.47g).
The iodide salt was converted to the nitrate salt by dissolving the iodide (9.4g) was dissolved in acetonitrile (250ml). Silver nitrate (4.89g) in acetonitrile (100ml) was added dropwise over 30 min. The mixture was stirred for a further 30 min, filtered and solvent removed at reduced pressure to give after trituration with acetone the title compound (6.48g) as a colourless solid.
¹H NMR (250MHz, CDCl₃) δ 1.40 (6H, s), 2.52 (2H, m), 3.69 - 3.77 (2H, m), 3.94 (2H, s), 4.52 (2H, m), 7.45 - 7.53 (2H, m) and 7.74 - 7.80 (2H, m).

### Description 19

### 6,6-Dimethyl-2,3,5,6-tetrahydro-1H-pyrrolo[2,1-a]-9-nitro-isoquinolinium iodide

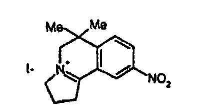

The nitrate salt D18 (6.48g) was added as a solid in portions over 1h to conc. sulphuric acid pre-cooled to -10 - -15°C **internal** temperature. After addition was complete the mixture was warmed to 0°C (internal temperature) and stirred for 1h. The reaction mixture was adjusted to pH10 by addition to sat. potassium hydroxide, pH adjusted to 3 with conc. HCl excess potassium iodide added and extracted with dichloromethane (10 x 100ml). The combined organic phase was dried (MgSO4) and solvent removed at reduced pressure to give the title compound (7.83g) after trituration with acetone.
¹H NMR (250MHz, CDCl₃) δ 1.50 (6H, s), 2.65 (2H, m), 3.98 - 4.05 (2H, m), 4.11 (2H, s), 4.50 - 4.57 (2H, m), 7.74 (1H, d, J = 7.6Hz) and 8.57 - 8.61 (2H, m).

### Description 20

### (+/-)-6,6-Dimethyl-1,2,3,5,6,10b-hexahydropyrrolo[2,1-a]-9-nitro-isoquinoline

The compound D19 (7.0g) was suspended in methanol and sodium borohydride (1.43g) added over 1h. The mixture was stirred for a further 1h after addition was complete, saturated potassium carbonate added and solvent removed at reduced pressure. The residue was extracted with dichloromethane (3 x 100ml), the organic phases were combined, dried (MgSO₄) and solvent removed at reduced pressure to give the title compound (5.17g) as a colourless solid.
¹H NMR (250MHz, CDCl₃) δ 1.32 (3H, s), 1.39 (3H, s), 1.64 - 2.00 (3H, m), 2.34 - 2.49 (3H, m), 2.88 (1H, d, J = 11Hz), 3.08 - 3.24 (2H, m), 7.44 (1H, d, J = 8.7Hz), 7.90 (1H, d, J = 2.4Hz) and 8.03 (1H, dd, J = 2.4, 8.7Hz).

### Description 21

### (+/-)-6,6-Dimethy-1,2,3,5,6,10b-hexahydropyrrolo[2,1-a]isoquinolin-9-ylamine

The title compound (4.31g) was prepared from D20 (5.0g) according to the procedure of D2.
¹H NMR (250MHz, CDCl₃) δ 1.24 (3H, s), 1.30 (3H, s), 1.63 - 1.95 (4H, m), 2.18 - 2.30 (2H, m), 2.38 (q, J = 8.7Hz), 2.80 (1H, d, J = 10.9Hz), 3.02 - 3.18 (2H, m), 3.53 (2H, br. s.)6.39 (1H, d, J = 2.3Hz), 6.55 (1H, dd, J = 2.5, 8.3Hz) and 7.08 (1H, d, J = 8.3Hz).
MS m/z (API): 217 (MH⁺; 100%)

### Description 22

### (+/-)7-Iodo-1,2,3,5,6,10b-hexahydropyrrolo[2,1-a]-9-Nitro-isoquinoline

Compound D3b (1.308g) was dissolved in trifluoromethanesulphonic acid (10ml) and cooled to 5°C. N-Iodosuccinimide (1.35g) was added in portions over 30min and the reaction stirred for 16h after completion of the addition. The mixture was poured into water adjusted to pH10 with 40% sodium hydroxide maintaining the internal temperature below 15°C. The basic solution was extracted with ethyl acetate, the organic phase washed with sodium thiosulphate and brine, dried (MgSO4) and solvent removed at reduced pressure. The residue was column chromatographed (silica gel, 0 - 10% (9:1 methanol/ammonium hydroxide) in dichloromethane) to give the title compound (1.29g) as a brown oil.
m/z (API): 345 (MH+; 100%)

### Description 23

### (+/-)7-Chloro-1,2,3,5,6,10b-hexahydropyrrolo[2,1-a]-9-Nitro-isoquinoline

Compound D22 (1.21g) was dissolved in dimethylformamide (20ml), copper(I)chloride (1.08g) added and the mixture heated to 125°C for 24h. Solvent was removed at reduced pressure and partitioned between ethyl acetate/water. A brown solid was separated by filtration, the organic phase separated and washed with water, sodium thiosulphate and brine, dried (MgSO4) and solvent removed at reduced pressure. Silica gel chromatography (silica gel, 0 - 5% (9:1 methanol/ammonium hydroxide) in dichloromethane) gave the title compound (0.17g)
MS m/z (API): 253, 255 (MH⁺; 100%)

### Description 24

### (+/-)-9-amino-7-Chloro-1,2,3,5,6,10b-hexahydropyrrolo[2,1-a]isoquinoline

Compound D23 (0.17g) was suspended in ethanol (13ml) and tin(II)chloride dihydrate (0.622g) added at 5°C. Tetrahydrofuran (2ml) was added and stirring continued for 16h. Conc. hydrochloric acid (1.3ml) was added and the reaction partitioned between dichloromethane/water. Sodium hydroxide was added to basify the mixture and the organic phase separated. The organic phase was washed with brine dried (MgSO4) and solvent removed at reduced pressure to give the title compound (0.15g) as a brown semi-solid
m/z (API): 223, 225 (MH⁺; 100%)

### Description 25

### (+/-) 10b-Methyl-1,2,3,5,6,10b-hexahydropyrrolo[2,1-a]isoquinoline

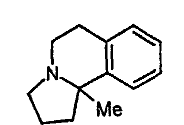

2,3,5,6-tetrahydro-1H-pyrrolo[2,1-a]isoquinolinium iodide was dissolved in tetrahydrofuran (25ml) and cooled to -78°C. Methylmagnesium chloride (0.4ml, 3.0M solution in THF) was added and the mixture warmed to room temperature and stirred for 3h. The mixture was recooled to -78°C and additional methyl magnesium chloride (0.8ml, 3.0M solution in THF) added and then stirred at room temperature for 16h. After quenching with saturated ammonium chloride the mixture was extracted with ethyl acetate, the organic phase washed with brine, dried (MgSO₄) and solvent removed at reduced pressure. The residue was column chromatographed (silica gel, (0-10% (9:1) methanol/ammonia)/dichloromethane) to give the title compound (0.13g) as a colourless oil.
MS m/z (API): 188 (MH+; 100%)

### Description 26

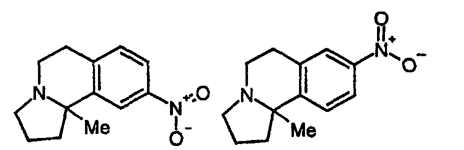

### 10b-Methyl-1,2,3,5,6,10b-hexahydropyrrolo[2,1-a]-8-nitroquinoline and 10b-Methyl-1,2,3,5,6,10b-hexahydropyrrolo[2,1-a]-9-nitroquinoline

The amine of D25 (2.85g) was dissolved in conc. sulphuric acid ( 40ml) and cooled to -10°C. Solid potassium nitrate (1.54g) was added over 30min. Stirring was continued at -10°C for 1h and at 10°C for 2h. The mixture was carefully basified with aqueous ammonia, extracted with dichloromethane and the organic phase dried (MgSO₄) and solvent removed at reduced pressure. The residue was column chromatographed silica gel, (0-5% (9:1)
methanol/ammonia)/dichloromethane eluant) to give the title inseparable mixture MS m/z (API): 233 (MH⁺; 100%)

### Description 27

### 8-Amino-10b-methyl-1,2,3,5,6,10b-hexahydropyrrolo[2,1-a]quinoline and 9-Amino-10b-methyl-1,2,3,5,6,10b-hexahydropyrrolo[2,1-a]quinoline

The title compounds (0.41g) were prepared according to the method of description D2 as an inseparable mixture.
MS m/z (API): 203 (MH⁺; 100%)

### Description 28

### 4-Chloro-N-(2-phenylpropyl)butyramide

The title compound (40.5g) was prepared according to the method of D17 from 2-phenylpropylamine (25.0g).
MS m/z (API): 240, 242 (MH⁺; 100%)

### Description 29

### 6-Methyl-2,3,5,6-tetrahydro-1H-pyrrolo[2,1-a]isoquinolinium iodide

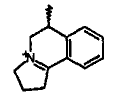

The title compound (8.47g) was prepared from D28 (10g) according to the method of example D18.
MS m/z (API): 186 (MH⁺; 100%)

### Description D30

### (+/-)-6-Methyl-1,2,3,5,6,10b-hexahydropyrrolo[2,1-a]isoquinoline

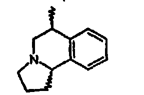

The salt D29 (5.0g) was dissolved in ethanol (100ml) containing platinum oxide (1.0g). The mixture was shaken under a hydrogen atmosphere (50 psi) for 4h, filtered and solvent removed at reduced pressure. The residue was dissolved in dichloromethane and washed with potassium carbonate and brine, dried (MgSO4) and solvent removed at reduced pressure to give the title compound (1.62g) as a brown oil.
MS m/z (API): 188 (MH⁺; 100%)

### Description 31

### (+/-)-6-Methyl-1,2,3,5,6,10b-hexahydropyrrolo[2,1-a]-7-nitro-isoquinoline and (+/-)-6-Methyl-1,2,3,5,6,10b-hexahydropyrrolo[2,1-a]-9-nitro-isoquinoline

The title compound (1.3g) isolated as a regioisomeric mixture of diastereoisomers was prepared according to the method of D26 from D30 (1.62g).
MS m/z (API): 233 (MH⁺; 100%)

### Description 32

### (+/-)-9-Amino-6-Methyl-1,2,3,5,6,10b-hexahydropyrrolo[2,1-a]isoquinoline

The title compound (0.6g) isolated as a mixture of diastereoisomers after chromatography (silica gel, 0-10% (9:1 methanol/ammonia)/dichloromethane eluant) was prepared according to the method of D2 from D31 (1.62g).
MS m/z (API): 203 (MH⁺; 100%)

### Description 33

### 4-Chloro-N-(3-phenylpropyl)butyramide

The title compound (44.0g) was prepared according to the method of D17 from 1-phenylpropylamine (25.0g) and 4-chlorobutyryl chloride.
MS m/z (API): 240, 242 (MH⁺; 100%)

### Description 34

### 1,2,3,5,6,7-hexahydrobenzo[c]pyrrolo[1,2-a]azepine nitrate salt and 5-methyl-2,3,5,6-tetrahydro-1H-pyrrolo[2,1-a]isoquinolinium nitrate

The title compound (1.2g) isolated as a mixture was prepared from D33 (44.0g) according to the method of example D18.
MS m/z (API): 186 (MH⁺; 100%)

### Description 35

### 10-Nitro-2,3,5,6,7,11b-hexahydro-1H-benzo[c]pyrrolo[1,2-a]azepine and (+/-)-5-Methyl-1,2,3,5,6,10b-hexahydropyrrolo[2,1-a]-9-nitro-isoquinoline

10-Nitro-1,2,3,5,6,7-hexahydrobenzo[c]pyrrolo[1,2-a]azepine iodide salt and 5-methyl-2,3,5,6-tetrahydro-1H-pyrrolo[2,1-a]isoquinolinium iodide (0.86g) isolated as a mixture was prepared from D34 (1.2g) according to the method of D19.

The title compounds were obtained by treating a mixture of 10-Nitro-1,2,3,5,6,7-hexahydrobenzo[c]pyrrolo[1,2-a]azepine iodide salt and 5-methyl-2,3,5,6-tetrahydro-1H-pyrrolo[2,1-a]isoquinolinium iodide (0.86g) with sodium borohydride according to the method of D20 after chromatography (silica gel, petroleum ether/acetone) to give
10-Nitro-2,3,5,6,7,11b-hexahydro-1H-benzo[c]pyrrolo[1,2-a]azepine (0.09g).
¹H NMR (250MHz, CDCl₃) δ: 1.59 - 1.79 (1H, m), 1.84 - 2.02 (3H, m), 2.20 - 2.34 (1H, m), 2.45 - 2.61 (2H, m), 2.75 (1H, t), 2.91 - 3.07 (2H, m), 3.24 - 3.34 (2H, m), 3.85 (1H, t), 7.26 (1H, m), 7.99 (1H, d) and 8.18 (1H, d). (+/-)-5-Methyl-1,2,3,5,6,10b-hexahydropyrrolo[2,1-a]-9-nitro-isoquinoline (0.245g)
MS (API): 233 (MH+; 100%)

### Description 36

### (+/-)-5-Methyl-1,2,3,5,6,10b-hexahydropyrrolo[2,1-a]isoquinolin-9-ylamine

The title compound (0.18g) was prepared from (+/-)-5-Methyl-1,2,3,5,6,10b-hexahydropyrrolo[2,1-a]-9-nitro-isoquinoline (0.23g, D36) according to the method of D2.

### Description 37

### 2,3,5,6,7,11b-hexahydro-1H-benzo[c]pyrrolo[1,2-a]azepin-10-ylamine.

The title compound (0.08g) was prepared from the compound of D36 according to the method of D2

### Description 38

### Hexahydroindolizin-7-one

Methyl vinylketone (3.5g) was added dropwise to 4-aminobutyraldehyde (6.45g) in diethyl ether (20ml) at 0°C. Stirring was continued for 1h, the cooling bath removed and the mixture stirred for 2h at room temperature. The organic phase was extracted with 2M hydrochlo0ric acid (100ml) and the extract warmed to 100°C for 2h. Solvent was removed at reduced pressure and excess aqueous potassium carbonate added. The basic solution was extracted with dichloromethane, extracts dried (MgSO4) and solvent removed at reduced pressure. The residue was distilled (145°C 3mbar). The distillate was further purified by silica gel column chromatography to give the title compound (1.66g) as a colourless oil
MS m/_{z} (API): 348 (MH⁺; 100%) 140

### Description 39

### 2-Nitro-5,6,8,9,10,10a-hexahydropyrrolo[2,1-f][1,6]naphthyridine

3,5-Dinitro-1-methylpyridin-2-one (1.97g) in methanol ammonia (70ml, 6:1 methanol:0.88SG ammonia) was treated with compound D38 (1.66g) and heated to 70°C for 5.5h. After cooling to room temperature solvent was removed at reduced pressure, the residue extracted with hot dichloromethane (x 2) and combined extracts evaporated to dryness. The residue was column chromatographed (silica gel, 2-5% (9:1 methanol/ammonia) in dichloromethane). Fractions 11-15 were rechromatographed (silica gel, 2% (9:1 methanol/ammonia) in dichloromethane) to give the title compound (0.09g)
MS m/_{z} (API): 348 (MH⁺; 100%) 220

### Description 40

### 5,6,8,9,10,10a-Hexahydropyrrolo[2,1-f][1,6]naphthyridine-2-ylamine

The title compound (0.07g) was prepared from D39 (0.09g) according to the procedure of D2
MS m/_{z} (API): 348 (MH⁺; 100%) 190

### Example 1

### (+/-) 3-cyano-N-(1,2,3,5,6,10b-hexahydropyrrolo[2,1-a]isoquinolin-9-yl)-4-methoxybenzamide hydrochloride

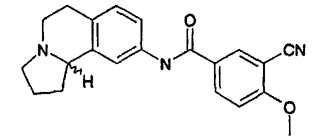

A solution of amine D5 (0.188g) 3-cyano-4-methoxybenzoic acid (0.179g) and N-hydroxybenzotriazole (0.05g) in dimethylformamide was treated with ethyldimethylaminopropyl carbodiimide HCl salt (0.19g) and stirred for 16h. The mixture was diluted with ethyl acetate, washed with water (x 5) dried (MgSO4) and solvent removed at reduced pressure. The residue was column chromatographed (silica gel, 95% dichloromethane/methanol) to give the title compound as a free base (0.24g). The hydrochloride salt was prepared from the free base (0.18g) in methanol (5ml) by addition of ethereal HCl (1M, 2ml). Solvent was removed at reduced presSure and the residue triturated to give the salt (0.09g).
¹H NMR (250MHz, CDCl₃) δ: 1.88 - 2.14 (3H, m), 2.47 - 2.62 (1H, m), 2.82 - 2.98 (1H, m), 3.08 - 3.14 (3H, m), 3.30 - 3.40 (1H, m), 3.55 - 3.69 (1H, m), 3.96 (3H, s), 4.36 (1H, t), 7.00 - 7.04 (2H, m), 7.67 - 7.73 (2H, m), 8.38 - 8.42 (m, 2H) and 9.76 (1H, br. s).
MS m/_{z} (API): 348 (MH⁺; 100%)

### Example 2

### (+/-) 3-Bromo-4-ethyl-N-(1,2,3,5,6,10b-hexahydropyrrolo[2,1-a]isoquinolin-9-yl)-benzamide

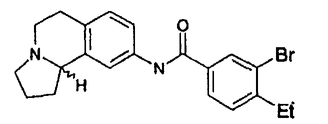

The title compound (0.06g) was prepared from amine D5 (0.188g) and 3-bromo-4-ethylbenzoic acid (0.228g) according to the method of Example 1.
¹H NMR (250MHz, CDCl₃) δ: 1.25 (3H, t), 1.67 - 2.02 (3H, m), 2.32 - 2.45 (1H, m), 2.52 (1H, t), 2.68(1H, dt), 2.76 - 2.86 (1H, m), 2.83 (2H, q), 3.02 - 3.27 (3H, m), 3.43 (1H, m), 7.11 (1H, d), 7.31 - 7.46 (3H, m), 7.69 - 7.76 (2H, m) and 8.03 (1H, s).
MS m/_{z} (API): 399, 401 (MH⁺; 100%)

### Example 3

### (+/-)N-(1,2,3,5,6,10b-hexahydropyrrolo[2,1-a]isoquinolin-9-yl)-4-methoxy-3-trifluoromethylbenzamide and (+/-)N-(1,2,3,5,6,10b-hexahydropyrrolo[2,1-a] isoquinolin-9-yl)-4-methoxy-3-trifluoromethylbenzamide hydrochloride

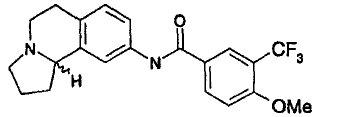

The free base of the title compound (0.38g) was prepared from amine D5 (0.188g) and 4-methoxy-3-trifluoromethylbenzoic acid (0.22g) according to the method of Example 1.
MS m/_{z} (API): 391 (MH⁺; 100%)
Free base ¹H NMR (250MHz, CDCl₃) δ 1.67 - 1.80 (1H, m), 1.82 - 2.01 (2H, m), 2.29 - 2.37 (1H, m), 2.52 (1H, q, J = 8Hz), 2.60 - 2.67 (1H, m), 2.81 (1H, m), 3.04 - 3.12 (2H, m), 3.15 - 3.24 (1H, m), 3.40 (1H, t), 7.04 (1H, d, J = 8.4Hz), 7.08 (1H, d, J = 8Hz), 7.34 - 7.41(2H, m), 7.99(1H, s), 8.05 (1H, d) and 8.08 (1H, s). The HCl salt (0.07g), isolated as a colourless solid was prepared by treating a solution of the free base (0.1g) in methanol (10ml) with ethereal HCI, removal of solvent at reduced pressure and recrystallisation from methanol/ethyl acetate/hexane. Hydrochloride salt ¹H NMR (250MHz, d⁶-DMSO) δ: 1.99 - 2.07 (3H, m), 2.52 - 2.61 (1H, m), 2.89 - 3.32 (4H, m), 3.42 - 3.50 (1H, m), 3.57 - 3.71 (1H, m), 4.74 (1H, t), 7.25 (1H, d, J = 8.36Hz), 7.43 (1H, d, J = 8.79Hz), 7.64 (1H, d, J = 8.29Hz), 7.70 (1H, s), 8.26 (1H, s) and 8.30 (1H, d, J = 8.79Hz), 10.43 (1H, s) and 11.50 (1H, br. s)

### Example 4

### (+/-) 3-Bromo-4-ethoxy-N-(1,2,3,5,6,10b-hexahydropyrrolo[2,1-a]isoquinolin-9-yl)benzamide, (+) 3-Bromo-4-ethoxy-N-(1,2,3,5,6,10b-hexahydropyrrolo[2,1-a]isoquinolin-9-yl)benzamide and (-) 3-Bromo-4-ethoxy-N-(1,2,3,5,6,10b-hexahydropyrrolo[2,1-a]isoquinolin-9-yl)benzamide

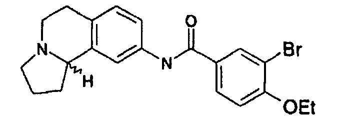

The title racemic compound (0.23g) was prepared from (+/-)-1,2,3,5,6,10b-hexahydropyrrolo[2,1-a]isoquinolin-9-amine (0.188g) and 3-bromo-4-ethoxybenzoic acid (0.25g) according to the method of Example 1. The HCl salt (0.13g) was prepared from the free base (0.18g) according to the method of Example 1. Hydrochloride salt ¹H NMR (250MHz, CDCl₃) δ: 1.48 (3H, t), 1.79 - 2.07 (3H, m), 1.89 - 2.11 (3H, m), 2.51 - 2.60 (1H, m), 2.79 - 2.87 (1H, m), 3.03 - 3.18 (3H, m), 3.34 - 3.43 (1H, m), 3.71 m(1H, m), 4.11 (2H, q), 4.47 (1H, t), 6.87 (1H, d, J = 4.64Hz), 6.98 (1H, d, J = 8.32Hz), 7.63 - 7.70 (2H, m), 8.11 (1H, dd, J = 2, 8.6Hz) and 9.55 (1H, s).
MS m/_{z} (API): 415, 417 (MH⁺; 100%)
Chiral HPLC separated the racemate into the two enantiomers, first eluting enantiomer (0.025g) and second eluting enantiomer (0.023g).

### Example 5

### (+/-) 3-Bromo-4-ethoxy-N-(1,3,4,6,7,11b-hexahydro-2H-pyrido[2,1-a]isoquinolin-10-yl)benzamide,

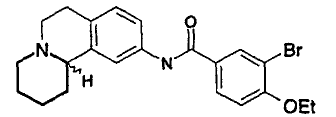

The title compound (0.09g) was prepared from amine D2 (0.165g) and 3-bromo-4-ethoxybenzoic acid (0.2g) according to the method of Example 1.
¹H NMR (250MHz, CDCl₃) δ: 1.50 (3H, t), 1.44 - 1.53 (1H, m), 1,65 - 1.73 (1H, m), 1.90 (1H, m), 2.20 - 2.42 (2H, m), 2.58 (1H, dt, J = 11.29 and 3.98Hz), 2.70 (1H, d), 2.96 - 3.19 (4H, m), 4.15 (2H, q), 6.90 (1H, d, J = 8.64Hz), 7.04 (1H, d, J = 8.25Hz), 7.30 (1H, dd, J = 2.01, 8.18Hz), 7.55 1H, d, J = 1.68Hz)7.80 (2H, m) and 8.05 (1H, d, J = 2.26Hz).
MS m/_{z} (API): 429, 431 (MH⁺)

### Example 6

### (+/-) N-(1,2,3,5,6,10b-hexahydropyrrolo[2,1-a]isoquinolin-9-yl)-4-methoxy-3-propionylbenzamide hydrochloride.

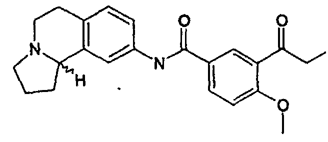

The title compound (0.126g) was prepared from compound D5 (0.094g) and 4-methoxy-3-propionylbenzoic acid (0.115g) according to the method of example 1. ¹H NMR (250MHz, CDCl₃) δ (free base) 1.84 (3H, t, J = 7.2Hz), 1.75 - 1.96 (3H, m), 2.31 - .68 (3H, m) 2.78 - 2.84 (1H, m), 3.03 (2H, q, J = 7.2Hz), 3.07 - 3.23 (3H, m), 3.42 (1H, m), 3.98 (3H, s), 7.06 - 7.13 (2H, m), 7.37 - 7.41 (2H, m),7.88 (1H, br. s.) and 8.14 (2H, m)
MS m/z (API): 379 (MH⁺; 100%)

### Example 7

### (+/-) N-(1,2,3,5,6,10b-hexahydropyrrolo[2,1-a]isoquinolin-9-yl)-4-ethoxy-3-propionylbenzamide hydrochloride.

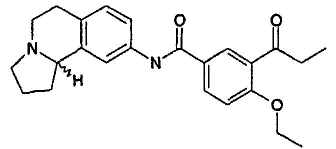

The title compound (0.177g) was prepared from compound D5 (0.094g) and 4-ethoxy-3-propionylbenzoic acid (0.122g) according to the method of example 1. ¹H NMR (250MHz, CDCl₃) δ (free base) 1.19 (3H, t, J = 7.2Hz), 1.55 (3H, t, J = 7.0Hz), 1.75 - 1.95 (3H, m), 2.31 - 2.68 (3H, m) 2.78 - 2.84 (1H, m), 3.03 (2H, q, J = 7.2Hz), 3.04 - 3.23 (3H, m), 3.42 (1H, m), 4.21 (2H, q, J = 6.9Hz), 7.05 (1H, d, J = 8.4Hz), 7.10(1H, d, J = 8.9Hz), 7.38 - 7.41(2H, m), 7.87 (1H, br. s.) and 8.12 (2H, m)
MS m/z (API): 393 (MH⁺; 100%)

### Example 8

### (+/-) 3-Acetyl-N-(1,2,3,5,6,10b-hexahydropyrrolo[2,1-a]isoquinolin-9-yl)benzamide hydrochloride.

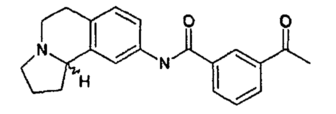

The title compound (0.235g) was prepared from compound D5 (0.188g) and 3-acetylbenzoic acid (0.164g) according to the method of example 1.
¹H NMR (250MHz, CDCl₃) δ (free base) 1.73 - 1.95 (3H, m), 2.31 - 2.69 (3H, m), 2.67 (3H, s), 2.78 - 2.85 (1H, m), 3.04 - 3.24 (3H, m), 3.42 (1H, m), 7.13 (1H, d, J = 8.0Hz), 7.37 - 7.42 (2H, m), 7.61 (1H, t, J = 7.7Hz), 7.94 (1H, br. s.), 8.12 (2H, m) and 8.44 (1H, m).
MS m/z (API): 335 (MH⁺; 100%)

### Example 9

### (+/-) 3-Acetyl-N-(1,2,3,5,6,10b-hexahydropyrrolo[2,1-a]isoquinolin-9-yl)-4-methoxybenzamide hydrochloride.

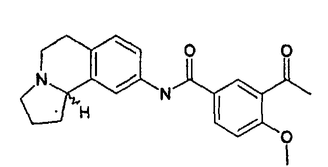

The title compound (0.227g) was prepared from compound D5 (0.188g) and 3-acetyl-4-methoxybenzoic acid (0.194g) according to the method of example 1.
¹H NMR (250MHz, CDCl₃) δ (free base) 1.76 - 1.96 (3H, m), 2.31 - 2.68 (3H, m), 2.66 (3H, s), 2.78 - 2.85 (1H, m), 3.04 - 3.24 (3H, m), 3.42 (1H, m), 4.00 (3H, s), 7.08-7.13 (2H, m), 7.39-7.41 (2H, m), 7.90 (1H, br. s.) and 8.15-8.19 (2H, m).
MS m/z (API): 365 (MH⁺; 90%)

### Example 10

### (+/-) 3-Fluoro-N-(1,2,3,5,6,10b-hexahydropyrrolo[2,1-a]isoquinolin-9-yl)-4-methoxybenzamide hydrochloride.

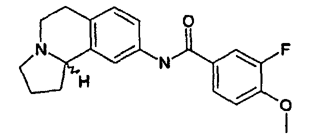

The title compound (0.228g) was prepared from compound D5 (0.188g) and 3-fluoro-4-methoxybenzoic acid (0.170g) according to the method of example 1.
¹H NMR (250MHz, CDCl₃) δ (free base) 1.71 - 1.97 (3H, m), 2.35 - 2.68 (3H, m), 2.78 - 2.85 (1H, m), 3.07 - 3.22 (3H, m), 3.41 (1H, m), 3.96 (3H, s), 7.02 (1H, t, J = 8.2Hz), 7.11 (1H, d, J = 8.2Hz), 7.33 (1H, dd, J = 2.1, 8.2Hz), 7.39 (1H, s), 7.63 (2H, m), 7.69 (1H, br. s.).
MS m/z (API): 341 (MH+; 100%)

### Example 11

### (+/-) 3,5-Dichloro-4-ethoxy-N-(1,2,3,5,6,10b-hexahydropyrrolo[2,1-a]isoquinolin-9-yl)benzamide hydrochloride.

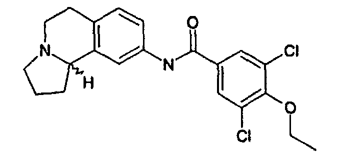

The title compound (0.352g) was prepared from compound D5 (0.188g) and 3,5-dichloro-4-ethoxybenzoic acid (0.235g) according to the method of example 1.
'H NMR (250MHz, CDCl₃) δ (free base) 1.49 (3H, t, J = 7.0Hz), 1.70 - 1.95 (3H, m), 2.29 - 2.68 (3H, m), 2.78 - 2.85 (1H, m), 3.07 - 3.22 (3H, m), 3.39 (1H, m), 4.17(2H, q, J = 7.0Hz), 7.10 (1H, d, J = 9.0Hz), 7.26 - 7.35 (2H, m), 7.70 (1H, br. s.) and 7.80 (2H, s).7.02 (1H, t, J = 8.2Hz), 7.11 (1H, d, J = 8.2Hz), 7.33 (1H, dd, J = 2.1, 8.2Hz), 7.39 (1H, s), 7.63 (2H, m), 7.69 (1H, br. s.).
MS m/z (API): 405,407 (MH+; 100%)

### Example 12

### (+/-) 3-Acetyl-N-(1,2,3,5,6,10b-hexahydropyrrolo[2,1-a]isoquinolin-9-yl)-4-propoxybenzamide hydrochloride.

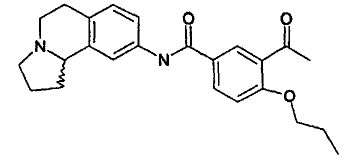

The title compound (0.340g) was prepared from compound D5 (0.188g) and 3-acetyl-4-propoxybenzoic acid (0.235g) according to the method of example 1.
¹H NMR (250MHz, CDCl₃) δ (free base) 1.10 (3H, t, J = 7.4Hz), 1.66 - 1.95 (5H, m), 2.28 - 2.63 (3H, m), 2.66 (3H, s), 2.78 - 2.84 (1H, m), 3.05 - 3.22 (3H, m), 3.39 (1H, m), 4.10 (2H, q, J = 7.4Hz), 6.99 - 7.11 (2H, m), 7.39 - 7.42 (2H, m), 8.12 - 8.17 (2H, m) and 8.22 (d, J = 2.4Hz).
MS m/z (API): 393 (MH+; 100%)

### Example 13

### (+/-) 3-Butyryl-N-(1,2,3,5,6,10b-hexahydropyrrolo[2,1-a]isoquinolin-9-yl)-4-methoxybenzamide hydrochloride.

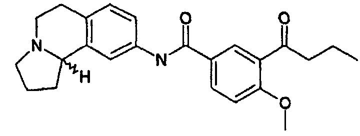

The title compound (0.280g) was prepared from compound D5 (0.188g) and 3-butyryl-4-methoxybenzoic acid (0.222g) according to the method of example 1. ¹H NMR (250MHz, CDCl₃) δ (free base) 0.93 (3H, t, J = 7.3Hz), 1.58 - 1.91 (5H, m), 2.20 - 2.65 (3H, m), 2.78 - 2.84 (1H, m), 2.91 (2H, d, J = 7.3Hz), 3.00 - 3.18 (3H, m), 3.34 (1H, m), 3.90 (3H, s), 6.95 (1H, d, J = 8.7Hz), 7.04 (1H, d, J = 8.3Hz), 7.38 - 7.47 (2H, m), 8.05 (1H, dd, J = 2.3, 8.7Hz), 8.15 (1H, d, J = 2.3Hz) and 8.58 (1H, br. s.)
MS m/z (API): 393 (MH+; 100%)

### Example 14

### (+/-) 3-isoButyryl-N-(1,2,3,5,6,10b-hexahydropyrrolo[2,1-a]isoquinolin-9-yl)-4-isopropoxybenzamidehydrochloride.

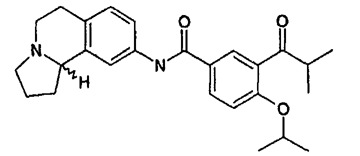

The title compound (0.275g) was prepared from compound D5 (0.188g) and 3-isobutyryl-4-*iso*propoxybenzoic acid - compound D11 - (0.275g) according to the method of example 1.
¹H NMR (250MHz, CDCl₃) δ 1.17 (6H, d, J = 6.9Hz), 1.42 (6H, d, J = 6.1Hz), 1.68 - 2.00 (3H, m), 2.31 - 2.68 (3H, m), 2.78 - 2.84 (1H, m), 3.02 - 3.26 (3H, m), 3.42 (1H, m), 3.54 (1H, septet, J = 6.8Hz), 4.76 (1H, septet, 6.0Hz), 7.02 (1H, d, J= 8.9Hz), 7.10 (1H, d, 8.8Hz), 7.37 - 7.41 (2H, m), 7.92 (1H, br. s.), 7.97 (1H, dd, J = 2.4Hz), 8.07 (1H, dd, J = 2.4, 8.7Hz)
MS m/z (API): 421 (MH+; 100%)

### Example 15

### (-) 3-Cyano-N-(1,2,3,5,6,10b-hexahydropyrrolo[2,1-a]isoquinolin-9-yl)-4-isopropylbenzamide hydrochloride.

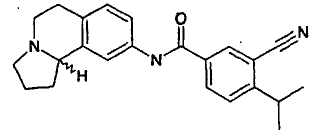

The title compound (0.25g) was prepared from compound D13 (0.188g) and 3-cyano-4-*iso*propylbenzoic acid ― (0.189g) according to the method of example 1. ¹H NMR (250MHz, CDCl₃) δ (free base) 1.32 (6H, d, J = 6.9Hz), 1.67 - 2.01 (3H, m), 2.31 (1H, m), 2.47 - 2.68 (2H, m), 2.76 - 2.82 (1H, m), 3.02 - 3.27 (3H, m), 3.33 - 3.48 (2H, m), 7.07 (1H, d, J= 8.6Hz), 7.35 - 7.39 (2H, m), 7.47 (1H, d, J = 8.5Hz), 8.06 (1H, dd, J = 1.9, 8.3Hz), 8.13 (1H, m) and 8.28 (1H, br. s.).
MS m/z (API): 360 (MH+; 100%)

### Example 16

### (+) 3-Cyano-N-(1,2,3,5,6,10b-hexahydropyrrolo[2,1-a]isoquinolin-9-yl)-4-isopropylbenzamide hydrochloride.

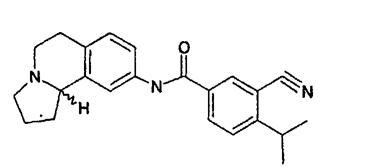

The title compound (0.213g) was prepared from (+) amine compound D14 (0.193g) and 3-cyano-4-*iso*propylbenzoic acid ― (0.189g) according to the method of example 1.
¹H NMR (250MHz, CDCl₃) δ (free base) 1.35 (6H, d, J = 6.9Hz), 1.71-1.99 (3H, m), 2.38 (1H, m), 2.47 - 2.68 (2H, m), 2.78 - 2.85 (1H, m), 3.03 - 3.28 (3H, m), 3.38 - 3.50 (2H, m), 7.12 (1H, d, J = 8.0Hz), 7.34 - 7.38 (2H, m), 7.52 (1H, d, J = 8.2Hz), 7.82 (1H, br. s.),8.05 (1H, dd, J = 1.9, 8.2Hz and 8.11 (1H, d, J = 1.9Hz). MS m/z (API): 360 (MH⁺; 100%)

### Example 17

### (+/-)4-Oxo-chroman-6-carboxylic acid (1,2,3,5,6,10b-hexahydropyrrolo[2,1-a]isoquinolin-9-yl)amide.

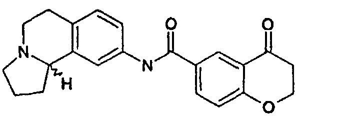

The title compound (0.078g) was prepared from compound D5 (0.188g) and 4-Oxo-chroman-6-carboxylic acid (0.192g) according to the method of example 1. ¹H NMR (250MHz, d⁶DMSO) δ (free base) 1.53 - 1.68 (1H, m), 1.76 - 1.87 (2H, m), 2.31 (1H, m), 2.46 - 2.58 (2H, m), 2.70 - 3.14 (5H, m)3.24 - 3.45 (2H, m), 4.63 (2H, t, J = 6.4Hz), 7.08 (1H, d, J = 8.3Hz), 7.17 (1H, d, J = 8.7Hz), 7.51 (1H, s), 7.61 (1H, d, J = 8.3Hz), 8.14 (1H, dd, J = 2.3, 8.7Hz), 8.45 (1H, d, J = 2.3Hz and 10.25 (1H, s).
MS m/z (API): 363 (MH⁺; 100%)

### Example 18

### (+/-) N-(1,2,3,5,6,10b-hexahydropyrrolo[2,1-a]isoquinolin-9-yl)-4-isopropoxy-3-propionylbenzamide hydrochloride.

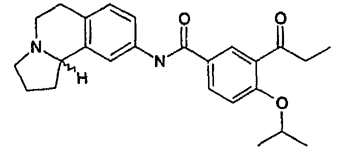

The title compound (0.174g) was prepared from compound D5 (0.094g) and 4-isopropoxy-3-propionylbenzoic acid (0.130mg) according to the method of example 1.
¹H NMR (250MHz, CDCl₃) δ (free base) 1.19 (3H, t, J = 7.2Hz), 1.44 (6H, d, J = 6.0Hz), 1.75 - 1.95 (3H, m), 2.31 - 2.68 (3H, m) 2.78 - 2.84 (1H, m), 3.04 (2H, q, J = 7.2Hz), 3.04 - 3.23 (3H, m), 3.42 (1H, m), 4.79 (1H, m), 7.05 (1H, d, J = 8.4Hz), 7.10 (1H, d, J = 8.9Hz), 7.38 - 7.41 (2H, m), 7.85 (1H, br. s.) and 8.09 - 8.12 (2H, m)
MS m/z (API): 407 (MH⁺; 100%).

### Example 19

### (+/-) N-(1,2,3,5,6,10b-hexahydropyrrolo[2,1-a]isoquinolin-9-yl)-3-isobutyryl-4-methoxybenzamide hydrochloride.

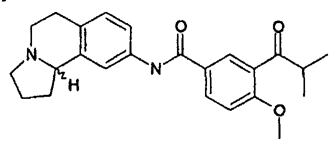

The title compound (0.045g) was prepared from compound D5 (0.094g) and 4-methoxy-3-isobutyrylbenzoic acid (0.111mg) according to the method of example 1.
¹H NMR (250MHz, CDCl₃) δ (free base) 1.17 (6H, d, J = 6.9Hz), 1.75-2.05 (3H, m), 2.32 - 2.69 (3H, m) 2.78 - 2.84 (1H, m), 3.02 - 3.25 (3H, m), 3.43 (1H, m), 3.49 (1H, m), 3.96 (1H, s), 7.06 (1H, d, J = 8.8Hz), 7.11 (1H, d, J = 8.1Hz), 7.37 - 7.40 (2H, m), 7.84 (1H, br. s.) 7.98 (1H, d, J = 2.4Hz) and 8.09 (1H, dd, J = 2.4, 8.7Hz)
MS m/z (API): 393 (MH⁺; 100%).

### Example 20

### (+/-) N-(1,2,3,5,6,10b-hexahydropyrrolo[2,1-a]isoquinolin-9-yl)-4-ethoxy-3-isobutyrylbenzamide hydrochloride.

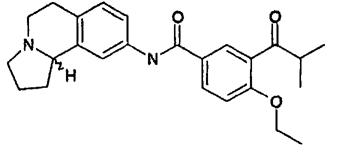

The title compound (0.04g) was prepared from compound D5 (0.094g) and 4-ethoxy-3-isobutyrylbenzoic acid (0.117mg) according to the method of example 1. ¹H NMR (250MHz, CDCl₃) δ (free base) 1.16 (6H, d, J = 6.9Hz), 1.49 (3H, t, J = 6.9Hz), 1.75 - 1.96 (3H, m), 2.31 - 2.69 (3H, m) 2.78 - 2.84 (1H, m), 3.02 - 3.25 (3H, m), 3.42 (1H, m), 3.56 (1H, m), 4.20 (2H, q, J = 7.04Hz), 7.03 (1H, d, J = 8.7Hz), 7.11 (1H, d, J = 8.6Hz), 7.37 - 7.40 (2H, m), 7.84 (1H, br. s.) 7.98 (1H, d, J = 2.4Hz) and 8.08 (1H, dd, J = 2.4, 8.6Hz)
MS m/z (APT): 407 (MH⁺; 100%).

### Example 21

### (+/-) N-(1,2,3,5,6,10b-hexahydropyrrolo[2,1-a]isoquinolin-9-yl)-4-ethoxy-3-fluorobenzamide hydrochloride.

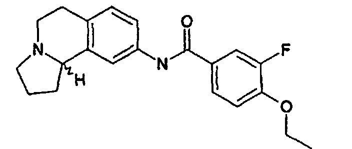

The title compound (0.151g) was prepared from compound D5 (0.094g) and 4-ethoxy-3-fluorobenzoic acid (0.101mg) according to the method of example 1.
¹H NMR (250MHz, CDCl₃) δ (free base) 1.52 (3H, t, J = 7.1Hz), 1.74 - 1.95 (3H, m), 2.31 - 2.68 (3H, m) 2.78 - 2.84 (1H, m), 3.01 - 3.24 (3H, m), 3.41 (1H, m), 4.17 (2H, q, J = 7.0Hz), 7.01 (1H, t, J = 8.2Hz), 7.10 (1H, d, J = 8.2Hz), 7.34 (1H, dd, J = 8.1, 2.1Hz), 7.39 (1H, s), 7.59 - 7.65 (2H, m) and 7.71 (1H, s).
MS m/z (API): 355 (MH⁺; 100%).

### Example 22

### (+/-) N-(1,2,3,5,6,10b-hexahydropyrrolo[2,1-a]isoquinolin-9-yl)-4-isopropoxy-3-fluorobenzamide hydrochloride.

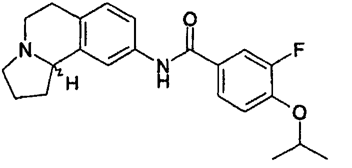

The title compound (0.166g) was prepared from compound D5 (0.094g) and 4-isopropoxy-3-fluorobenzoic acid (0.109mg) according to the method of example 1.
¹H NMR (250MHz, CDCl₃) δ (free base) 1.52 (6H, d, J = 6.0Hz), 1.74 - 1.95 (3H, m), 2.31 - 2.68 (3H, m) 2.78 - 2.84 (1H, m), 3.02 - 3.23 (3H, m), 3.42 (1H, m), 4.66 (1H, m), 7.02 (1H, t, J = 8.2Hz), 7.10 (1H, d, J = 8.2Hz), 7.33 (1H, dd, J = 8.1, 2.1Hz), 7.39 (1H, s), 7.59 - 7.65 (2H, m) and 7.70 (1H, s).
MS m/z (API): 369 (MH⁺; 100%).

### Example 23

### (+) 3-Cyano-N-(1,2,3,5,6,10b-hexahydropyrrolo[2,1-a]isoquinolin-9-yl)-4-isopropoxybenzamide hydrochloride.

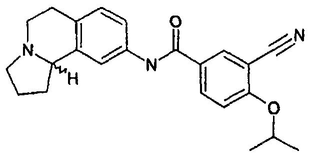

The title compound (0.167g) was prepared from compound D14 (0.139g) and 4-isopropoxy-3-cyanobenzoic acid (0.205g) according to the method of example 1. ¹H NMR (250MHz, CDCl₃) δ (free base) 1.44 (6H, d, J = 6.0Hz), 1.74 - 1.95 (3H, m), 2.31 - 2.68 (3H, m) 2.78 - 2.84 (1H, m), 3.02 - 3.23 (3H, m), 3.42 (1H, m), 4.75 (1H, m),. 7.04 (1H, t, J = 9.6Hz), 7.10 (1H, d, J = 8.0Hz), 7.35 - 7.38 (2H, m), 7.81 (1H, s). 8.05 - 8.10 (2H, m).
MS m/z (API): 376 (MH⁺; 100%).

### Example 24

### (+) 3-Cyano-4-ethoxy-N-(1,2,3,5,6,10b-hexahydropyrrolo[2,1-a]isoquinolin-9-yl)-benzamide hydrochloride.

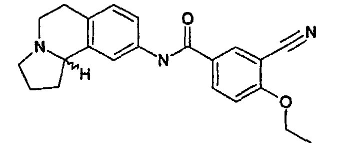

The title compound (0.167g), after recrystallisation from ethyl acetate, was prepared from compound D14 (0.139g) and 4-ethoxy-3-cyanobenzoic acid (0.191g) according to the method of example 1.
¹H NMR (250MHz, CDCl₃) δ (free base) 1.52 (3H, t, J = 6.9Hz), 1.66 - 1.95 (3H, m), 2.31 - 2.68 (3H, m), 2.78 - 2.84 (1H, m), 3.02 - 3.23 (3H, m), 3.40 (1H, m), 4.23 (2H, q, J = 7.0Hz), 7.04 (1H, d, J = 9.5Hz), 7.10(1H, d, J = 8.0Hz), 7.35 - 7.38 (2H, m), 7.81 (1H, s), 8.07 - 8.11 (2H, m)
MS m/z (API): 362 (MH⁺; 100%).

### Example 25

### (+/-)N-(1,2,3,5,6,10b-hexahydropyrrolo[2,1-a]isoquinolin-9-yl)-3-propionyl-4-propoxybenzamide hydrochloride.

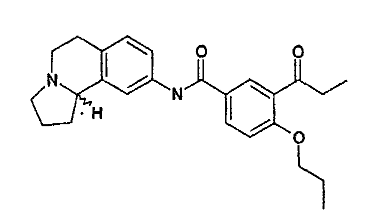

The title compound (0.135g) was prepared from compound D5 (0.094g) and 4-propoxy-3-propionylbenzoic acid (0.118g) according to the method of example 1. ¹H NMR (250MHz, CDCl₃) δ (free base) 1.10 (3H, t, J = 7.5Hz), 1.19 (3H, t, J = 7.2Hz), 1.75 - 1.99 (5H, m), 2.31 - 2.69 (3H, m), 2.78 - 2.84 (1H, m), 3.05 (2H, q, J = 7.2Hz), 3.02 - 3.24 (3H, m), 3.42 (1H, m), 4.10 (2H, q, J = 7.5Hz), 7.05 (1H, d, J = 8.5Hz), 7.11 (1H, d, J = 9.0Hz), 7.37 - 7.41 (2H, m), 7.88 (1H, s), 8.10 - 8.15 (2H, m)
MS m/z (API): 407 (MH⁺; 100%).

### Example 26

### (+/-) 3-Acetyl-4-ethyl-N-(1,2,3,5,6,10b-hexahydropyrrolo[2,1-a]isoquinolin-9-yl)benzamide hydrochloride.

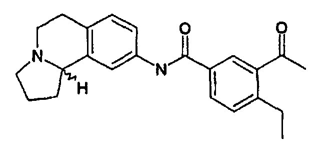

The title compound (0.146g) was prepared from compound D5 (0.094g) and 3-acetyl-4-ethylbenzoic acid (0.096g) according to the method of example 1.
¹H NMR (250MHz, CDCl₃) δ (free base) 1.24 (3H, t, J = 7.5Hz), 1.72 - 1.99 (3H, m), 2.31 - 2.68 (3H, m), 2.64 (3H, s), 2.78 - 2.84 (1H, m), 2.92 (2H, q, J = 7.5Hz), 3.02 - 3.23 (3H, m), 3.38 (1H, m), 7.12 (1H, d, J = 8.2Hz), 7.34 - 7.43 (3H, m), 7.83 (2H, m) and 8.17 (1H, d, J = 1.9Hz).
MS m/z (API): 363 (MH⁺; 100%).

### Example 27

### (+/-)3-Acetyl-4-chloro-N-(1,2,3,5,6,10b-hexahydropyrrolo[2,1-a]isoquinolin-9-yl)benzamide hydrochloride.

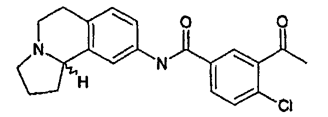

The title compound (0.160g) was prepared from compound D5 (0.094g) and 3-acetyl-4-chlorobenzoic acid (0.10g) according to the method of example 1.
¹H NMR (250MHz, CDCl₃) δ (free base) 1.71-1.99 (3H, m), 2.31 - 2.68 (3H, m), 2.64 (3H, s), 2.78 - 2.84 (1H, m), 3.02 - 3.23 (3H, m), 3.41 (1H, m), 7.12 (1H, d, J = 8.4Hz), 7.36 - 7.39 (2H, m), 7.54 (1H, d, J = 8.3Hz), 7.88 (1H, br. s.), 7.94 (1H, dd, J = 2.3, 8.3Hz) and 8.04 (1H, d, J= 2.3Hz).
MS m/z (API): 369, 371 (MH⁺; 100%).

### Example 28

### (+/-) 3-Acetyl-4-bromo-N-(1,2,3,5,6,10b-hexahydropyrrolo[2,1-a]isoquinolin-9-yl)benzamide hydrochloride.

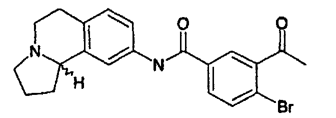

The title compound (0.075g) was prepared from compound D5 (0.047g) and 3-acetyl-4-bromobenzoic acid (0.061g) according to the method of example 1.
¹H NMR (250MHz, CDCl₃) δ (free base) 1.71-1.99 (3H, m), 2.31 - 2.68 (3H, m), 2.69 (3H, s), 2.78 - 2.84 (1H, m), 3.02 - 3.23 (3H, m), 3.41 (1H, m), 7.12 (1H, d, J = 8.4Hz), 7.35 - 7.38 (2H, m), 7.72 (1H, d, J = 8.2Hz), 7.80 (1H, dd, J = 2.2, 8.3Hz), 7.88 (1H, s) and 7.94 (1H, s, J = 2.2Hz).
MS m/z (API):413, 415 (MH⁺; 100%).

### Example 29

### (+/-) 3-Acetyl-5-bromo-N-(1,2,3,5,6,10b-hexahydropyrrolo[2,1-a]isoquinolin-9-yl)benzamide hydrochloride.

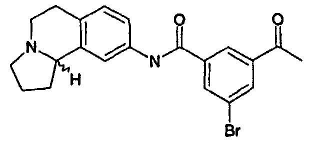

The title compound (0.051g) was prepared from compound D5 (0.047g) and 3-acetyl-5-bromobenzoic acid (0.061g) according to the method of example 1.
¹H NMR (250MHz, CDCl₃) δ (free base) 1.71-1.99 (3H, m), 2.31 - 2.68 (3H, m), 2.69 (3H, s), 2.78 - 2.84 (1H, m), 3.02 - 3.23 (3H, m), 3.41 (1H, m), 7.13 (1H, d, J = 8.8Hz), 7.38 - 7.40 (2H, m), 7.90 (1H, br. s.), 8.22 (2H, m) and 8.33 (1H,m).. MS m/z (API):413, 415 (MH⁺; 100%

### Example 30

### (+/-) 3-Cyano-4-ethoxy-N-(1,2,3,5,6,10b-hexahydropyrrolo[2,1-a]isoquinolin-9-yl)benzamide hydrochloride.

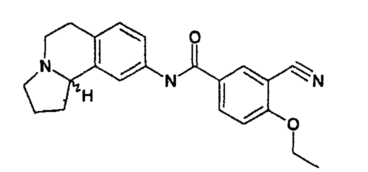

The title compound (0.158g), after recrystallisation from ethyl acetate, was prepared from compound D5 (0.094g) and 3-cyano-4-ethoxybenzoic acid (0.105g) according to the method of example 1.
¹H NMR (250MHz, d⁶DMSO) δ (free base) 1.33 (3H, t, J = 6.9Hz), 1.46 - 1.57 (1H, m), 1.67 - 1.75 (2H, m), 2.17 - 2.41 (3H, m), 2.61 - 2.67 (1H, m), 2.79 - 3.16 (3H, m), 3.23 (1H, m), 4.22 (2H, q, J = 6.9Hz), 7.01 (1H, d, J = 8.3Hz), 7.31 (1H, d, J= 9.0Hz), 7.39 (1H, s), 7.46 (1H, dd, J = 1.9, 8.2Hz),8.16 (1H, dd, J = 2.2, 8.9Hz), 8.29 (1H, d, J = 2.2Hz) and 10.07 (1H, s)
MS m/z (API): 362 (MH⁺; 100%

### Example 31

### (+/-) 3-Cyano-N-(1,2,3,5,6,10b-hexahydropyrrolo[2,1-a]isoquinolin-9-yl)-4-isopropoxybenzamide hydrochloride.

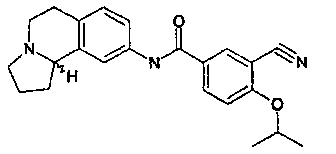

The title compound (0.135g) was prepared from compound D5 (0.094g) and 3-cyano-4-isopropoxybenzoic acid (0.113g) according to the method of example 1. ¹H NMR (250MHz, CDCl₃) δ (free base) 1.44 (6H, d, J = .6.1Hz), 1.71-1.99 (3H, m), 2.31 - 2.68 (3H, m), 2.69 (3H, s), 2.77 - 2.84 (1H, m), 3.02 - 3.23 (3H, m), 3.41 (1H, m), 4.75 (1H, m), 7.04 (1H, d, J = 9.6Hz), 7.11 (1H, d, J = 8.0Hz), 7.34 - 7.38 (2H, m), 7.79 (1H, br. s.) and 8.08 (2H, m).
MS m/z (API): 376 (MH⁺; 100%)

### Example 32

### (+/-) 3-acetyl-4-Acetylamino-N-(1,2,3,5,6,10b-hexahydropyrrolo[2,1-a]isoquinolin-9-yl)benzamide hydrochloride.

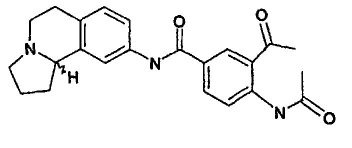

The title compound (0.060g) was prepared from compound D5 (0.094g) and 3-acetyl-4-acetylaminobenzoic acid (0.122g) according to the method of example 1. ¹H NMR (250MHz, CDCl₃) δ (free base) 1.68 - 1.97 (3H, m), 2.26 (3H, s), 2.31 - 2.69 (3H, m), 2.74 (3H, s), 2.77 - 2.84 (1H, m), 3.00 - 3.25 (3H, m), 3.41 (1H, m), 4.75 (1H, m), 7.13 (1H, d, J = 8.2Hz), 7.38 (1H, dd, J =2.1, 10.0Hz), 7.44 (1H, s), 7.95 (1H, dd, J = 2.1, 8.8Hz), 8.07 (1H, s), 8.58 (1H, d, J = 2.0Hz) and 8.84 (1H, d, J = 8.8Hz). 11.89 (1H, br. s.).
MS m/z (API): 414 (M+ Na⁺; 100%)

### Example 33

### (+) 3-Acetyl-N-(1,2,3,5,6,10b-hexahydropyrrolo[2,1-a]isoquinolin-9-yl)-4-isopropylbenzamide hydrochloride.

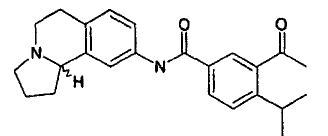

The title compound (0.177g) was prepared from compound D14 (0.168g) and 3-acetyl-4-isopropylbenzoic acid (0.206g) according to the method of example 1.
¹H NMR (250MHz, CDCl₃) δ (free base) 1.25 (6H, d, J = 6.8Hz), 1.67 - 1.97 (3H, m), 2.31 - 2.69 (3H, m), 2.61 (3H, s), 2.77 - 2.84 (1H, m), 3.04 - 3.22 (3H, m), 3.47 (1H, m), 7.11 (1H, d, J = 8.2Hz), 7.35 (1H, dd, J =2.1, 8.2Hz), 7.42 (1H, s), 7.50 (1H, d, J = 8.2Hz), 7.85 (12, m) and 8.01 (1H, d, J = 1.9Hz).
MS m/z (API): 377 (MH⁺; 100%)

### Example 34

### (+)-N-(1,2,3,5,6,10b-hexahydropyrrolo[2,1-a]isoquinolin-9-yl)-3-propionyl-4-isopropoxybenzamide hydrochloride.

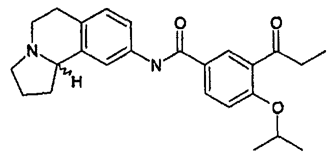

The title compound (0.078g) was prepared from compound D14 (0.047g) and 3-propionyl-4-isopropoxybenzoic acid (0.071g) according to the method of example 1.
¹H NMR (250MHz, CDCl₃) δ (free base)1.44 (6H, d, J = 6/1Hz), 1.71 - 1.96 (3H, m), 2.40 (1H, m), 2.48 - 2.69 (2H, m), 2.83 (1H, m), 3.01 - 3.27 (3H, m), 3.42 (1H, m), 4.79 (1H, m), 7.05 (1H, d, J = 9.6Hz), 7.11 (1H, d, J = 9.0Hz), 7.38 - 7.41 (2H, m), 7.86 (1H, br. s.) and 8.09 - 8.13 (2H, m).
MS m/z (API): 407 (MH⁺; 100%)

### Example 35

### (+) 3-Acetyl-4-ethoxy-N-(1,2,3,5,6,10b-hexahydropyrrolo[2,1-a]isoquinolin-9-yl)-benzamide.

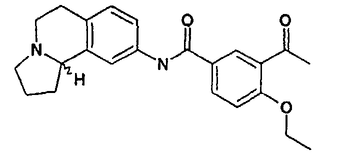

The title compound (0.19g) was prepared from compound D14 (0.168g) and 3-acetyl-4-ethoxybenzoic acid (0.208g) according to the method of example 1.
¹H NMR (250MHz, CDCl₃) δ (free base). 1.53 (3H, t, J = 7.1Hz), 1.69 - 1.97 (3H, m), 2.31 - 2.69 (3H, m), 2.68 (3H, s), 2.77 - 2.84 (1H, m), 3.02 - 3.23 (3H, m), 3.47 (1H, m), 4.22 (2H, q, J = 7.1Hz), 7.06 (1H, d, J = 8.7Hz), 7.11 (1H, d, J = 9.0Hz), 7.39 - 7.41 (2H, m). 7.89 (1H, s) and 8.17 (2H, m). m/z (API): 377 (MH+; 100%)
MS m/z (API): 379 (MH⁺; 100%)

### Example 36

### (+)3-Cyano-N-(1,2,3,5,6,10b-hexahydropyrrolo[2,1-a]isoquinolin-9-yl)-4-methoxybenzamide hydrochloride.

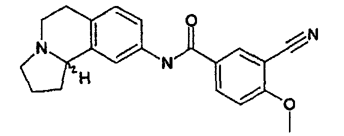

The title compound (0.205g) was prepared from compound D14 (0.168g) and 3-cyano-4-methoxybenzoic acid (0.177g) according to the method of example 1. ¹H NMR (250MHz, CDCl₃) δ (free base) 1.67 - 1.97 (3H, m), 2.31 - 2.69 (3H, m), 2.77 - 2.84 (1H, m), 3.01 - 3.24 (3H, m), 3.47 (1H, m), 4.01 (3H, s), 7.06 (1H, d, J = 8.7Hz), 7.11 (1H, d, J = 8.1Hz), 7.36 - 7.40 (2H, m), 7.95 (1H, s) and 8.11 (2H, m).
MS m/z (API): 348 (MH⁺; 100%)

### Example 37

### (+)-N-(1,2,3,5,6,10b-hexahydropyrrolo[2,1-a]isoquinolin-9-yl)4-methoxy-3-trifluoromethylbenzamide hydrochloride.

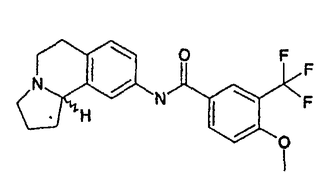

The title compound (0.187g) was prepared from compound D14 (0.177g) and 4-methoxy-3-trifluoromethylbenzoic acid (0.242g) according to the method of example 1.
¹H NMR (250MHz, CDCl₃) δ (free base) 1.70 - 1.95 (3H, m), 2.36 (1H, m), 2.47 - 2.68 (2H, m), 2.78 - 2.85 (1H, m), 3.01 - 3.23 (3H, m), 3.41 (1H, m), 3.98 (3H, s), 7.10 (2H, m), 7.33 - 7.40 (2H, m), 7.73 (1H, br. s.) and 8.06 (2H, m).
MS m/z (API): 391 (MH⁺; 100%)

### Example 38

### (-)-N-(1,2,3,5,6,10b-hexahydropyrrolo[2,1-a]isoquinolin-9-yl)-4-methoxy-3-trifluoromethylbenzamide hydrochloride.

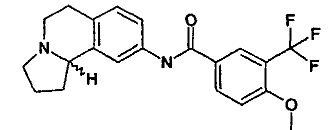

The title compound (0.23g) was prepared from compound D13 (0.188g) and 4-methoxy-3-trifluoromethylbenzoic acid (0.220g) according to the method of example 1.
¹H NMR (250MHz, CDCl₃) δ (free base) 1.70 - 1.95 (3H, m), 2.36 (1H, m), 2.47 - 2.68 (2H, m), 2.78 - 2.85 (1H, m), 3.01 - 3.23 (3H, m), 3.41 (1H, m), 3.98 (3H, s), 7.10 (2H, m), 7.33 - 7.40 (2H, m), 7.73 (1H, br. s.) and 8.06 (2H, m).
MS m/z (API): 391 (MH⁺; 100%)

### Example 39

### (+)-3-Fluoro-N-(1,2,3,5,6,10b-hexahydropyrrolo[2,1-a]isoquinolin-9-yl)-4-methoxy-benzamide hydrochloride.

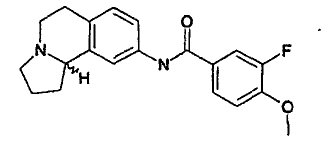

The title compound (0.236g) was prepared from compound D14 (0.188g) and 3-fluoro-4-methoxy-benzoic acid (0.204g) according to the method of example 1.
¹H NMR (250MHz, CDCl₃) δ (free base) 1.70 - 1.97 (3H, m), 2.35 (1H, m), 2.47 -2.68 (2H, m), 2.78-2.84(1H, m), 2.99- 3.23 (3H, m), 3.41 (1H, m), 3.96 (3H, s), 7.02 (1H, t, J = 8.2Hz), 7.11 (1H, d, J = 8.2Hz), 7.33 (1H, dd, J = 2.2, 8.2Hz), 7.39 (1H, s), 7.63 (2H, d, J = 9.3Hz) and 7.68 (1H, br. s.).
MS m/z (API): 341 (MH⁺; 100%)

### Example 40

### (+)-4-Ethoxy-3-fluoro-N-(1,2,3,5,6,10b-hexahydropyrrolo[2,1-a]isoquinolin-9-yl)benzamide hydrochloride.

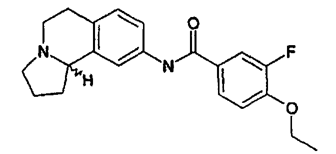

The title compound (0.282g) was prepared from compound D14 (0.188g) and 3-fluoro-4-methoxy-benzoic acid (0.22g) according to the method of example 1.
¹H NMR (250MHz, CDCl₃) δ (free base) 1.49 (3H, t, J = 7.1Hz), 1.70 - 1.94 (3H, m), 2.35 (1H, m), 2.47 - 2.68 (2H, m), 2.78 - 2.84 (1H, m), 2.99 - 3.23 (3H, m), 3.41 (1H, m), 4.17 (2H, q, J = 7.1Hz), 7.00 (1H, t, J = 8.2Hz), 7.10 (1H, d, J = 8.2Hz), 7.33 (1H, dd, J = 2.2, 8.2Hz), 7.39 (1H, s), 7.57 - 7.65 (2H, m) and 7.69 (1H, br. s.).
MS m/z (API): 355 (MH+; 100%)

### Example 41

### (+/-)-3-Butyryl-N-(1,2,3,5,6,10b-hexahydropyrrolo[2,1-a]isoquinolin-9-yl)-4-propoxybenzamide hydrochloride.

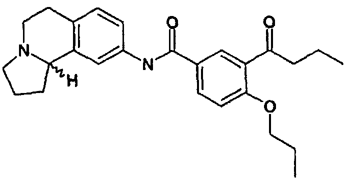

The title compound (0.3g) was prepared from compound D5 (0.188g) and 3-butyryl-4-propoxybenzoic acid (0.25g) according to the method of example 1. MS m/z (API): 421 (MH⁺; 100%).

### Example 42

### (+/-)-3-Butyryl-N-(1,2,3,5,6,10b-hexahydropyrrolo[2,1-a]isoquinolin-9-yl)-4-isopropoxybenzamide hydrochloride.

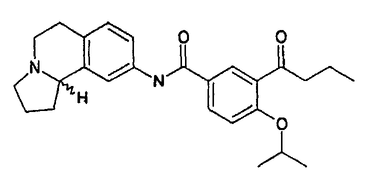

The title compound (0.32g) was prepared from compound D5 (0.188g) and 3-butyryl-4-isopropoxybenzoic acid (0.25g) according to the method of example 1. MS m/z (APT): 421 (MH⁺; 100%).

### Example 43

### (+/-)-3-Acetyl-N-(1,2,3,5,6,10b-hexahydropyrrolo[2,1-a]isoquinolin-9-yl)-4-propoxybenzamide hydrochloride.

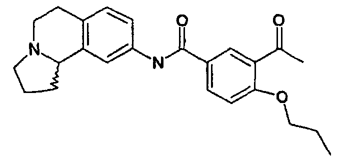

The title compound (0.34g) was prepared from compound D5 (0.188g) and 3-acetyl-4-propoxybenzoic acid (0.24g) according to the method of example 1.
MS m/z (API): 393 (MH⁺; 100%).

### Example 44

### (+/-)-3-Butyryl-4-ethoxy-N-(1,2,3,5,6,10b-hexahydropyrrolo[2,1-a]isoquinolin-9-yl)benzamide hydrochloride.

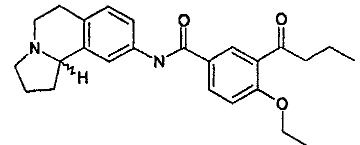

The title compound (0.247g) was prepared from compound D5 (0.188g) and 3-butyryl-4-ethoxybenzoic acid (0.24g) according to the method of example 1.
MS m/z (APT): 407 (MH⁺; 100%).

### Example 45

### (+/-)-3-isoButyryl-N-(1,2,3,5,6,10b-hexahydropyrrolo[2,1-a]isoquinolin-9-yl)-4-propoxybenzamide hydrochloride.

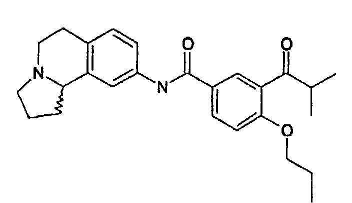

The title compound (0.30g) was prepared from compound D5 (0.188g) and 3-*iso*butyryl-4-propoxybenzoic acid (0.28g) according to the method of example 1. MS m/z (API): 421 (MH⁺; 100%).

### Example 46

### (+/-)-3-Acetyl-N-(1,2,3,5,6,10b-hexahydropyrrolo[2,1-a]isoquinolin-9-yl)-4-isopropoxybenzamide hydrochloride. SB-326909-A

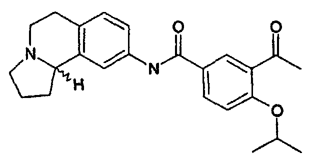

The title compound (0.162g) was prepared from compound D5 (0.223g) and 3-acetyl-4-*iso*propoxybenzoic acid (0.222g) according to the method of example 1. MS m/z (API): 393 (MH⁺, 100%)

### Example 47

### (+/-)-3-Chloro-N-(1,2,3,5,6,10b-hexahydropyrrolo[2,1-a]isoquinolin-9-yl)-4-isopropoxybenzamide hydrochloride.

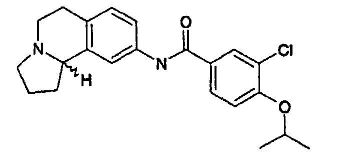

The title compound (0.162g) was prepared from compound D5 (0.223g) and 3-chloro-4-*iso*propoxybenzoic acid (0.212g) according to the method of example 1. MS m/z (API): 385, 387 (MH⁺; 100%).

### Example 48

### (+/-)-3-Cyano-N-(1,2,3,5,6,10b-hexahydropyrrolo[2,1-a]isoquinolin-9-yl)-4-isopropylbenzamide hydrochloride.

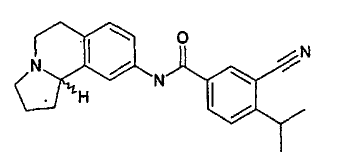

The title compound (0.145g) was prepared from compound D5 (0.223g) and 3-cyano-4-*iso*propylbenzoic acid (0.189g) according to the method of example 1. MS m/z (API): 360 (MH⁺; 100%).

### Example 49

### (+/-)-3-Bromo-N-(1,2,3,5,6,10b-hexahydropyrrolo[2,1-a]isoquinolin-9-yl)-4-isopropoxybenzamide hydrochloride.

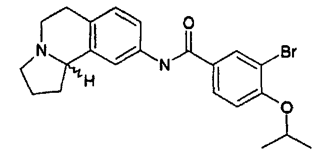

The title compound (0.086g) was prepared from compound D5 (0.223g) and 3-bromo-4-*iso*propoxybenzoic acid (0.259g) according to the method of example 1. MS m/z (API): 429, 431 (MH⁺; 100%).

### Example 50

### (+/-)-5-Acetyl-N-(1,2,3,5,6,10b-hexahydropyrrolo[2,1-a]isoquinolin-9-yl)-2-methoxy-4-isopropoxybenzamide hydrochloride.

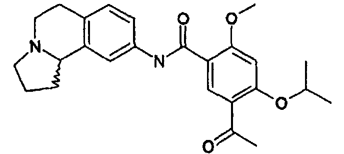

The title compound (0.197g) was prepared from compound D5 (0.188g) and 5-acetyl-2-methoxy-4-isopropoxybenzoic acid (0.259g) according to the method of example 1.
MS m/z (API): 422 (MH⁺; 100%).

### Example 51

### (+/-)-N-(1,2,3,5,6,10b-hexahydropyrrolo[2,1-a]isoquinolin-9-yl)-3-pivaloylbenzamide hydrochloride.

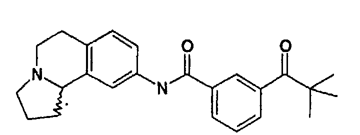

The title compound (0.312g) was prepared from compound D5 (0.188g) and 3-pivaloylbenzoic acid (0.210g) according to the method of example 1.
MS m/z (API): 377 (MH⁺; 100%).

### Example 52

### (+/-)-N-(1,2,3,5,6,10b-hexahydropyrrolo[2,1-a]isoquinolin-9-yl)-2-methoxy-4-isopropyl-5-trifluoromethylbenzamide hydrochloride.

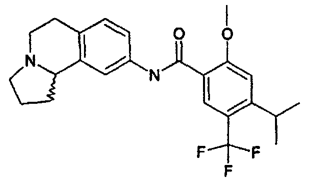

The title compound (0.250g) was prepared from compound D5 (0.188g) and 2-methoxy-4-isopropyl-5-trifluoromethylbenzoic acid (0.178g) according to the method of example 1.
MS m/z (API): 433 (MH⁺; 100%).

### Example 53

### (+/-)-Naphthalene-2-carboxylic acid (1,2,3,5,6,10b-hexahydropyrrolo[2,1-a]isoquinolin-9-yl)amide hydrochloride.

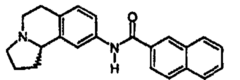

The title compound (0.265g) was prepared from compound D5 (0.188g) and 2-naphthoic acid (0.172g) according to the method of example 1.
MS m/z (API): 343 (MH⁺; 100%).

### Example 54

### (+/-)-Benzothiazole-5-carboxylic acid (1,2,3,5,6,10b-hexahydropyrrolo[2,1-a]isoquinolin-9-yl)amide hydrochloride.

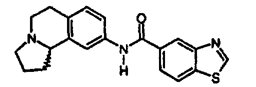

The title compound (0.215g) was prepared from compound D5 (0.188g) and 5-benzothiazolylcarboxylic acid (0.181g) according to the method of example 1.
MS m/z (API): 350 (MH⁺; 100%).

### Example 55

### (+/-)-2,3-Dihydrobenzofuran-5-carboxylic acid (1,2,3,5,6,10b-hexahydropyrrolo[2,1-a]isoquinolin-9-yl)carboxamide hydrochloride.

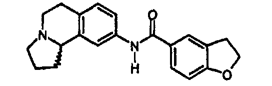

The title compound (0.280mg) was prepared from compound D5 (0.188g) and 5-(2,3-dihydrobenzofurancarboxylic acid (0.167g) according to the method of example 1.
MS m/z (API): 335 (MH⁺; 100%).

### Example 56

### (+)-3-Acetyl-N-(1,2,3,5,6,10b-hexahydropyrrolo[2,1-a]isoquinolin-9-yl)-4-isopropoxybenzamide hydrochloride.

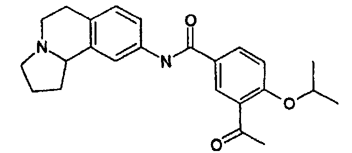

The title compound (0.276g) was prepared from compound D14 (0.211g) and 3-acetyl-4-isopropoxybenzoic acid (0.244g) according to the method of example 1. ¹H NMR (250MHz, CDCl₃) δ (free base) 1.46 (6H, d, J = .5.9Hz), 1.73 - 1.95 (3H, m), 2.31 - 2.68 (3H, m), 2.66 (3H, s), 2.77 - 2.84 (1H, m), 3.01 - 3.23 (3H, m), 3.44 (1H, m), 4.81 (1H, m), 7.06 (1H, d, J = 8.8Hz), 7.11(1H, d, J = 8.1Hz), 7.38 - 7.41 (2H, m), 7.88 (1H, br. s.) and 8.12 - 8.18 (2H, m).
MS m/z (API): 393 (MH⁺; 100%).

### Example 57

### (+)-3-Chloro-4-ethoxy-N-(1,2,3,5,6,10b-hexahydropyrrolo[2,1-a]isoquinolin-9-yl)benzamide hydrochloride.

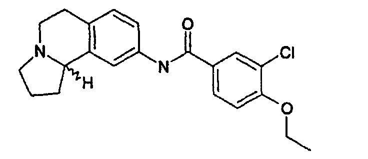

The title compound (0.184g) was prepared from compound D14 (0.193g) and 3-chloro-4-ethoxybenzoic acid (0.244g) according to the method of example 1.
¹H NMR (250MHz, CDCl₃) δ (free base) 1.53 (3H, t, J = 7.0Hz), 1.71 - 1.97 (3H, m), 2.29 - 2.68 (3H, m), 2.77- 2.84 (1H, m), 3.01 - 3.24 (3H, m), 3.41 (1H, m), 4.17 (2H, q, J = 7.0Hz), 6.97 (1H, d, J = 8.7Hz), 7.10 (1H, d, J = 8.2Hz), 7.35 (1H, dd, J = 2.1, 8.2Hz), 7.39 (1H, s), 7.68 (1H, s), 7.76 (1H, dd, J = 2.3, 8.5Hz) and 7.88 (1H, d, 2.2Hz).
MS m/z (API): 371, 373 (MH⁺; 100%).

### Example 58

### (+/-)-N-3-(N,N-Dimethylcarboxamido)-(1,2,3,5,6,10b-hexahydropyrrolo[2,1-a]isoquinolin-9-yl)-4-isopropoxybenzamide hydrochloride.

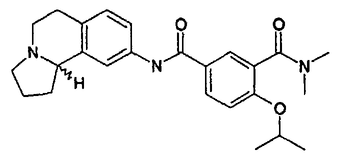

The title compound (0.184g) was prepared from compound D5 (0.188g) and 3-(N, N-dimethylcarboxamido)-4-isopropoxybenzoic acid (0.301g) according to the method of example 1.
MS m/z (API): 422 (MH⁺; 100%).

### Example 59

### (+/-)-N-(6,6-dimethyl-1,2,3,5,6,10b-hexahydropyrrolo[2,1-a]isoquinolin-9-yl)-4-methoxy-3-trifluoromethylbenzamide hydrochloride.

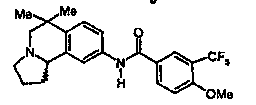

The title compound (0.340g) was prepared from compound D21 (0.216g) and 4-methoxy-3-trifluoromethylbenzoic acid (0.22g) according to the method of example 1.
MS m/z (API): 419 (MH⁺; 100%)

### Example 60

### (+/-)-3-Bromo-N-(6,6-dimethyl-1,2,3,5,6,10b-hexahydropyrrolo[2,1-a]isoquinolin-9-yl)-4-ethoxybenzamide hydrochloride.

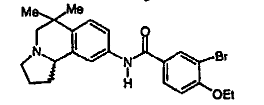

The title compound (0.355g) was prepared from compound D21 (0.216g) and 3-bromo-4-ethoxybenzoic acid (0.245g) according to the method of example 1.
MS m/z (API): 443, 445 (MH⁺; 100%)

### Example 61

### (+/-)-3-Bromo-N-(6,6-dimethyl-1,2,3,5,6,10b-hexahydropyrrolo[2,1-a]isoquinolin-9-yl)-4-isopropoxybenzamide hydrochloride.

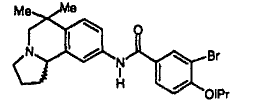

The title compound (0.21g) was prepared from compound D21 (0.216g) and 3-bromo-4-isopropoxybenzoic acid (0.259g) according to the method of example 1. MS m/z (API): 457, 459 (MH⁺; 100%)

### Example 62

### (+/-)-3-Cyano-N-(6,6-dimethyl-1,2,3,5,6,10b-hexahydropyrrolo[2,1-a]isoquinolin-9-yl)-4-isopropylbenzamide hydrochloride.

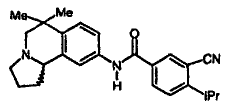

The title compound (0.21g) was prepared from compound D21 (0.216g) and 3-cyano-4-isopropylbenzoic acid (0.189g) according to the method of example 1. MS m/z (API): 388 (MH⁺; 100%)

### Example 63

### (+/-)-3-Acetyl-N-(6,6-dimethyl-1,2,3,5,6,10b-hexahydropyrrolo[2,1-a]isoquinolin-9-yl)-4-isopropoxybenzamide hydrochloride.

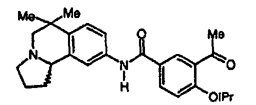

The title compound (0.313g) was prepared from compound D21 (0.216g) and 3-acetyl-4-isopropoxybenzoic acid (0.222g) according to the method of example 1. MS m/z (API): 421 (MH⁺; 100%)

### Example 64

### (+/-)-N-(6,6-dimethyl-1,2,3,5,6,10b-hexahydropyrrolo[2,1-a]isoquinolin-9-yl)-3-fluoro-4-methoxybenzamide hydrochloride.

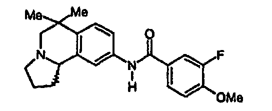

The title compound (0.125g) was prepared from compound D21 (0.108g) and 3-fluoro-4-methoxybenzoic acid (0.102g) according to the method of example 1.
¹H NMR (250MHz, CDCl₃) δ (free base) 1.28 (3H, s), 1.35 (3H, s), 1.68 - 2.00 (2H, m), 2.25 - 2.36 (2H, m), 2.40 (1H, q, J = 8.7Hz), 2.87 (2H, dd, J = 15, 22Hz), 3.08 (1H, dt, J = 2.6, 8.8Hz), 3.22 (1H, m), 3.96 (3H, s), 7.02 (1H, t, J = 8.2Hz), 7.26 - 7.43 (2H, m), 7.41 (1H, dd, J = 2.1, 8.3Hz), 7.60 - 7.69 (3H, m).
MS m/z (API): 369 (MH⁺; 100%)

### Example 65

### (+/-)-N-(6,6-dimethyl-1,2,3,5,6,10b-hexahydropyrrolo[2,1-a]isoquinolin-9-yl)-3-fluoro-4-ethoxybenzamide hydrochloride.

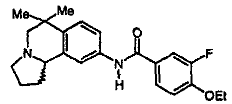

The title compound (0.129g) was prepared from compound D21 (0.108g) and 3-fluoro-4-ethoxybenzoic acid (0.11g) according to the method of example 1.
MS m/z (API): 383 (MH⁺; 100%)

### Example 66

### (+/-)-N-(6,6-dimethyl-1,2,3,5,6,10b-hexahydropyrrolo[2,1-a]isoquinolin-9-yl)-3-fluoro-4-isopropoxybenzamide hydrochloride.

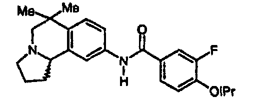

The title compound (0.138g) was prepared from compound D21 (0.108g) and 3-fluoro-4-isopropoxybenzoic acid (0.119g) according to the method of example 1. MS m/z (API): 397 (MH⁺; 100%)

### Example 67

### (+/-)-3-Cyano-N-(6,6-dimethyl-1,2,3,5,6,10b-hexahydropyrrolo[2,1-a]isoquinolin-9-yl)-4-methoxybenzamide hydrochloride.

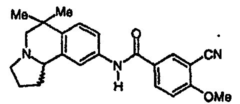

The title compound (0.114g) was prepared from compound D21 (0.108g) and 3-cyano-4-methoxybenzoic acid (0.106g) according to the method of example 1.
MS m/z (API): 376 (MH⁺; 100%)

### Example 68

### (+/-)-3-Acetyl-N-(6,6-dimethyl-1,2,3,5,6,10b-hexahydropyrrolo[2,1-a]isoquinolin-9-yl)-4-methoxybenzamide hydrochloride.

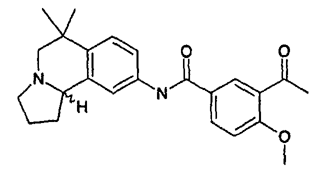

The title compound (0.122g) was prepared from compound D21 (0.108g) and 3-acetyl-4-methoxybenzoic acid (0.116g) according to the method of example 1.
MS m/z (APT): 393 (MH⁺; 100%)

### Example 69

### (+)-N-(6,6-dimethyl-1,2,3,5,6,10b-hexahydropyrrolo[2,1-a]isoquinolin-9-yl)-3-fluoro-4-methoxybenzamide hydrochloride and (-)-N-(6,6-dimethyl-1,2,3,5,6,10b-hexahydropyrrolo[2,1-a]isoquinolin-9-yl)-3-fluoro-4-methoxybenzamide hydrochloride.

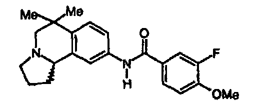

The compound of example 63 (0.1g) was resolved by chiral HPLC to give as the first eluting fraction (+)-N-(6,6-dimethyl-1,2,3,5,6,10b-hexahydropyrrolo[2,1-a]isoquinolin-9-yl)-3-fluoro-4-methoxybenzamide and as the second eluting fraction (-)-N-(6,6-dimethyl-1,2,3,5,6,10b-hexahydropyrrolo[2,1-a]isoquinolin-9-yl)-3-fluoro-4-methoxybenzamide as free bases. The free bases of the title compounds were converted to the hydrochloride salts according to the method of example 1.
Characterisation
(+)-N-(6,6-dimethyl-1,2,3,5,6,10b-hexahydropyrrolo[2,1-a]isoquinolin-9-yl)-3-fluoro-4-methoxybenzamide hydrochloride (0.026g). [α]²³_{D} = +66.4° (c 0.5, MeOH)
(-)-N-(6,6-dimethyl-1,2,3,5,6,10b-hexahydropyrrolo[2,1-a]isoquinolin-9-yl)-3-fluoro-4-methoxybenzamide hydrochloride (0.03g). [α]²³_{D} = -62.0° (c 0.5, MeOH)

### Example 70

### (+/-)-N-(7-Chloro-1,2,3,5,6,10b-hexahydropyrrolo[2,1-a]isoquinolin-9-yl)-4-methoxy-3-trifluoromethylbenzamide hydrochloride.

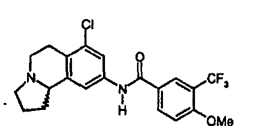

The title compound (0.04g) was prepared from compound D24 (0.15g) and 4-methoxy-3-trifluoromethylbenzoic acid (0.242g) according to the method of example 1.
¹H NMR (250MHz, CDCl₃) δ (free base) 1.66 - 1.99 (3H, m), 2.28 - 2.64 (3H, m), 2.82 - 3.17 (3H, m), 3.24 - 3.35 (2H, m), 3.98 (3H, s), 7.08 (1H, d, J = 9.3Hz), 7.30 (1H, s), 7.55 (1H, d, J = 1.9Hz), 7.84 (1H, s) and 8.05 - 8.07 (2H, m).
MS m/z (API): 425, 427 (MH⁺; 100%)

### Example 71

### (+/-)-10b-Methyl-N-(1,2,3,5,6,10b-hexahydropyrrolo[2,1-a]isoquinolin-9-yl)-4-methoxy-3-trifluoromethylbenzamide hydrochloride.

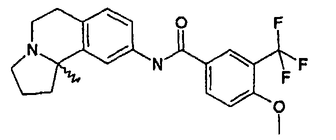

The title compound (0.035g) was prepared from mixture D27 (0.40g) and 4-methoxy-3-trifluoromethylbenzoic acid (0.435g) according to the method of example 1, after purification by silica gel chromatography.
MS m/z (API): 405 (MH⁺; 100%).

### Example 72

### (+/-)-3-Bromo-N-4-ethoxy(6-methyl-1,2,3,5,6,10b-hexahydropyrrolo[2,1-a]isoquinolin-9-yl)-4-isopropoxybenzamide hydrochloride.

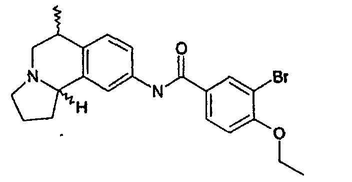

The title compound (0.105g) was prepared from compound D32 (0.3g) and 3-bromo-4-ethoxybenzoic acid (0.357g) according to the method of example 1. MS m/z (API): 429, 431 (MH⁺; 100%).

### Example 73

### (+/-)-N-(6-methyl-1,2,3,5,6,10b-hexahydropyrrolo[2,1-a]isoquinolin-9-yl)-4-methoxy-3-trifluoromethylbenzamide hydrochloride.

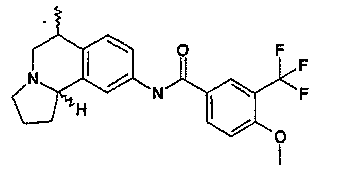

The title compound (0.153g) was prepared from compound D32 (0.3g) and 4-methoxy-3-trifluoromethylbenzoic acid (0.327g) according to the method of example 1.
MS m/z (API): 405 (MH⁺; 100%).

### Example 74

### (+/-)-N-(5-methyl-1,2,3,5,6,10b-hexahydropyrrolo[2,1-a]isoquinolin-9-yl)-4-methoxy-3-trifluoromethylbenzamide hydrochloride.

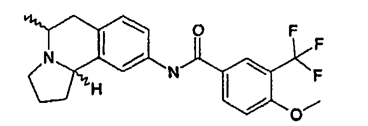

The title compound (0.13g) was prepared from amine D36 and 4-methoxy-3-trifluoromethylbenzoic acid according to the method of example 1.
MS m/z (API): 405 (MH⁺; 100%).

### Example 75

### (+/-)-3-Acetyl-N-(5-methyl-1,2,3,5,6,10b-hexahydropyrrolo[2,1-a]isoquinolin-9-yl)-4-isopropoxybenzamide hydrochloride.

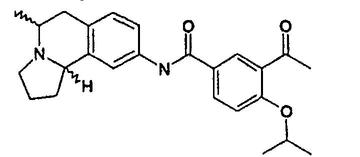

The title compound (0.13g) was prepared from amine D36 and 3-acetyl-4-isopropoxybenzoic acid, according to the method of example 1.
MS m/z (API): 407 (MH⁺; 100%).

### Example 76

### (+/-) 3-Acetyl-N-(2,3,5,6,7,11b-hexahydro-1H-benzo[c]pyrrolo[1,2-a]azepin-10-yl)-4-isopropoxybenzamide hydrochloride.

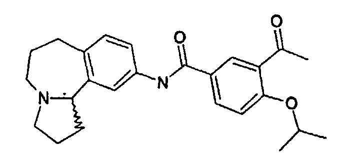

The title compound (0.042) was prepared from the amine D37 (0.135g) and 3-acetyl-4-isoporpoxybenzoic acid, according to the method of example 1.
MS m/z (API): 407 (MH⁺; 100%).

### Example 77

### (+/-)-N-(2,3,5,6,7,11b-hexahydro-1H-benzo[c]pyrrolo[1,2-a]azepin-10-yl)-4-methoxy-3-trifluoromethylbenzamide hydrochloride.

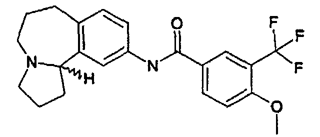

The title compound (0.026g) was prepared from the amine D37 (0.135g) and 4-methoxy-3-trifluoromethylbenzoic acid, according to the method of example 1. MS m/z (API): 405 (MH⁺; 100%)

### Example 78

### (+/-) 4-tert-Butyl-2-methoxy-N-(1,2,3,5,6,10b-hexahydropyrrolo[2,1-a]isoquinolin-7-yl)benzamide

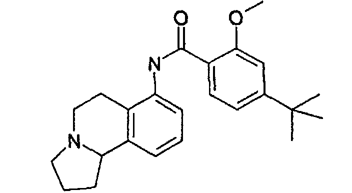

a). (+/-) 2-(2-Bromoethyl)-1,3-dioxane (1.08ml, 7.86mmol) and dry tetrahydrofuran (7ml) were added to magnesium turnings (0.257g,10.6 mmol) that had stirred overnight under argon. The mixture was heated to reflux to prepare the grignard reagent by a literature method. The resulting solution after allowing to cool to room temperature was added by syringe to a suspension of 5-amino-2-benzylisoquinolinium bromide² (1g, 3.17mmol) in dry tetrahydrofuran (40ml) with stirring under argon. After 2h at room temperature the resulting brown solution was poured into water (200ml), extracted with ethyl acetate (2 x 100ml) and dried over anhydrous sodium sulfate. The crude 2-benzyl-1-(2-[1,3]dioxan-2-ylethyl)-1, 2, 4a, 8a-tetrahydroyoquinoline-5-ylamine was obtained in quantitative yield after filtration and evaporation to dryness, as an orang-brown gum that solidified on standing.
   MS m/z (AP+): 351 (100%), MH⁺.
b). (+/-) Sodium borohydride (1.425g, 37.6mmol) was added in portions (1g in portions over 5-10 min, then allowed to stir for 20 min and the remainder added in one further portion) with stirring under argon to a solution of crude 2-Benzyl-1-(2-[1,3]dioxan-2-ylethyl)-1, 2, 4a, 8a-tetrahydroyoquinoline-5-ylamine (3.4g, 9.71mmol) in methanol (150ml) at room temperature. The mixture was concentrated under reduced pressure after stirring for 2 days, the residue diluted with water and acidified with 10% citric acid. After rebasifying with 10% sodium hydroxide the mixture was extracted with ethyl acetate (3 x 100ml). The combined organic extracts were dried over anhydrous sodium sulfate and evaporated to dryness to give a brown gum. Trituration with a mixture of diethyl ether, pentane and a small amount of dichloromethane gave 2-benzyl-1-(2-[1,3]dioxan-2-ylethyl)-1,2,3,4,4a,8a hexahydroisoquinolin-5-ylamine as a cream solid (2.114g, 62%)
   MS m/z (AP+): 353 (100%), MH⁺.
c). (+/-) 2-Benzyl-1-(2-[1,3]dioxan-2-ylethyl)-1, 2, 3, 4, 4a, 8a-hexahydroisoquinolin-5-ylamine(0.5g, 1.42mmol) and 2-methoxy-4-tert-butylbenzoic acid (0.295g, 1.54mmol) were coupled together under standard conditions [EDC/HOBT in dry dimethylformamide (5ml) at room temperature overnight]. Most of the solvent was then removed under reduced pressure and the residue partioned between water (20ml) and ethylacetate (20ml). The aqueous layer was further extracted with ethyl acetate (2 x 20ml) and the combined organics washed with dilute aqueous sodium carbonate (10ml) and brine (10ml) before drying over anhydrous sodium sulfate and evaporation to dryness. The residue was purified by column chromatography on silica gel with initially dichloromethane as eluant, then ethyl acetate, to give N-[2-benzyl-1-(2-[1,3]dioxan-2-ylethyl)-1,2,3,4,4a,8a-hexahydroisoquinolin-5-yl]-4-tertbutyl-2-methoxybenzamide as a pale yellow foam (0.669g, 87%)
   MS m/z (AP+): 543(100%), MH⁺.
d). (+/-) N-[2-benzyl-1-(2-[1,3]dioxan-2-ylethyl)-1,2,3,4,4a,8a-hexahydroisoquinolin-5-yl]-4-tertbutyl-2-methoxybenzamide (0.078g, 0.144mmol) was added to a suspension of 10% palladium on charcoal in tetrahydrofuran (20ml) and 5N hydrochloric acid (5ml) and the mixture hydrogenated at room temperature and atmospheric pressure for 2 days. After filtration through kieselguhr and evaporation to dryness the residue was chromatographed on silica gel with a gradient of 0-5% methanol in dichloromethane as eluant. The product, a white gum, was washed repeatedly with small volumes of ether and then converted to the free base by partition between dichloromethane (10ml) and saturated aqueous sodium carbonate (10ml). The aqueous layer was further extracted with dichloromethane (2 x 10ml) and the combined organic layers dried over anhydrous sodium sulfate and evaporated to dryness to give the title compound as a white crystalline solid (0.0307g, 56%). MS m/z (AP+): 379(100%), MH⁺.
   ¹H NMR (CDCl₃, 400MHz) δ: 1.36 (9H, s), 1.77 (1H, m), 2.50 (1H, q), 2.67 (1H, m), 2.73-2.83 (1H, br.m), 2.94-3.06 (1H, m), 3.15 (1H, m), 3.28-3.43 (2H, m), 4.06 (3H, s), 6.91 (1H, d), 7.03 (1H,s) 7.12-7.29 (2H, overlapping m), 8.15 (1H, d), 8.23 (1H, d) and 9.72 (1H, br.s).

### Example 79

### (+/-) 5-Cyano-2-ethoxy-4-iso-propyl-N-(2,3,5,6,6a,10a,10b-octahydro-1H-pyrrolo[2,1-a]isoquinolin-7-yl)benzamide

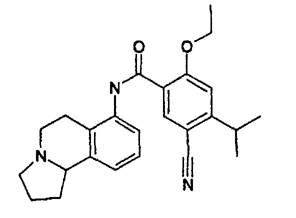

The title compound (0.05g) was prepared from (+/-)-1,2,3,5,6,10b-hexahydropyrrolo[2,1-a]isoquinolin-7-ylamine (0.10g) (Description 4) and 5-cyano-2-ethoxy-4-iso-propylbenzoic acid (0.12g) according to the method of Example 2.
MS m/z (AP+): 404 (100%), MH⁺.
¹H NMR (CDCl₃, 400MHz) δ: 1.35 (6H, d, J = 7Hz), 1.59 (3H, t, J = 7Hz, 1.7 - 2.0 (3H, m), 2.35 - 3.05 (5H, m), 3.1 - 3.2 (1H, m), 3.25 - 3.45 (3H, m), 4.38 (2H, q, J = 7Hz, 6.94 - 6.98 (2H, m), 7.24 (1H, t, J = 8Hz), 7.91 (1H, d, J = 8Hz), 8.59 (1H, s) and 9.33 (1H, br. s.).

### Example 80

### N-(5,6,8,9,10,10a-hexahydropyrrolo[2,1-f][1,6]naphthridin-2-yl)-4-methoxy-3-trifluoromethylbenzamide

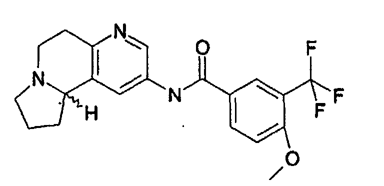

The title compound (0.008g) was prepared from amine D40 (0.07g) and 3-trifluoromethyl-4-methoxybenzoic acid (0.81g) according to the method of Example 2.
MS m/z (AP+): 392 (100%), MH⁺.
¹H NMR (MeOH, 250MHz) δ: 1.72 - 1.86 (1H, m), 1.94 - 2.02 (2H, m), 2.41 - 2.54 (1H, m), 2.60 - 2.71 (1H, m), 2.75 - 2.86 (1H, m), 2.94 - 3.01 (1H, m), 3.09 - 3.32 (3H, m), 4.01 (3H, s), 7.33 (1H, d, J = 8.4Hz), 8.02 (1H, d, J = 1.9Hz), 8.22 - 8.25 (2H, m) amnd 8.68 (1H, d, J = 1.9Hz).

### PHARMACOGICAL DATA

### 1. Binding Assay Method

WO 92/22293 (SmithKline Beecham) discloses compounds having anti-convulsant activity, including *inter alia* the compound *trans*-(+)-6-acetyl-4S-(4-fluorobenzoylamino)-3,4-dihydro-2,2-dimethyl-2H-1-benzopyran-3R-ol (hereinafter referred to as Compound A). It has been found that the compounds of WO 92/22293 bind to a novel receptor obtainable from rat forebrain tissue, as described in WO 96/18650 (SmithKline Beecham). The affinity of test compounds to the novel receptor site is assessed as follows.

### Method

Whole forebrain tissue is obtained from rats. The tissue is first homogenised in buffer (usually 50mM Tris/HCl, pH 7.4). The homogenised tissue is washed by centrifugation and resuspension in the same buffer, then stored at -70°C until used.

To carry out the radioligand binding assay, aliquots of tissue prepared as above (usually at a concentration of 1-2mg protein/ml) are mixed with aliquots of [3H]-Compound A dissolved in buffer. The final concentration of [3H]-Compound A in the mixture is usually 20nM. The mixture is incubated at room temperature for 1 hour. [3H]-Compound A bound to the tissue is then separated from unbound [3H]-Compound A by filtration through Whatman GF/B glass fibre filters. The filters are then washed rapidly with ice-cold buffer. The amount of radioactivity bound to the tissue trapped on the filters is measured by addition of liquid scintillation cocktail to the filters followed by counting in a liquid scintillation counter.

In order to determine the amount of "specific" binding of [3H]-Compound A, parallel assays are carried out as above in which [3H]-Compound A and tissue are incubated together in the presence of unlabelled Compound A (usually 3 µM). The amount of binding of [3H]-Compound A remaining in the presence of this unlabelled compound is defined as "non-specific" binding. This amount is subtracted from the total amount of [3H]-Compound A binding (i.e. that present in the absence of unlabelled compound) to obtain the amount of "specific" binding of [3H]-Compound A to the novel site.

The affinity of the binding of test compounds to the novel site can be estimated by incubating together [3H]-Compound A and tissue in the presence of a range of concentrations of the compound to be tested. The decrease in the level of specific [3H]-Compound A binding as a result of competition by increasing concentrations of the compound under test is plotted graphically, and non-linear regression analysis of the resultant curve is used to provide an estimate of compound affinity in terms of pKi value.

### Results

Compounds of this invention were active in this test with pKi values greater than 6. For example, compounds of Examples 3, 4 and 39 gave pKi values greater than 7.5.

### 2. MEST Test

The maximal electroshock seizure (MEST) threshold test in rodents is particularly sensitive for detecting potential anticonvulsant properties ¹. In this model, anticonvulsant agents elevate the threshold to electrically-induced seizures whilst proconvulsants lower the seizure threshold.

### Method for mouse model

Mice (naive male, Charles River, U.K. CD-1 strain, 25 - 30g) are randomly assigned to groups of 10 - 20 and dosed orally or intraperitoneally at a dose volume of 10 ml/kg with various doses of compound (0.3 - 300 mg/kg) or vehicle. Mice are then subjected at 30 or 60 min post dose to a single electroshock (0.1 sec, 50Hz, sine wave form) administered via corneal electrodes. The mean current and standard error required to induce a tonic seizure in 50% (CC₅₀) of the mice in a particular treatment group is determined by the 'up and down' method of Dixon and Mood (1948)². Statistical comparisons between vehicle- and drug-treated groups are made using the method of Litchfield and Wilcoxon (1949)³.

In control animals the CC₅₀ is usually 14 - 18 mA. Hence the first animal in the control group is subjected to a current of 16 mA. If a tonic seizure does not ensue, the current is increased for a subsequent mouse. If a tonic convulsion does occur, then the current is decreased, and so on until all the animals in the group have been tested.

Studies are carried out using a Hugo Sachs Electronik Constant Current Shock Generator with totally variable control of shock level from 0 to 300 mA and steps of 2 mA are usually used.

### Method for rat model

The threshold for maximal (tonic hindlimb extension) electroshock seizures in male rats (Sprague Dawley, 80 - 150g, 6 weeks old) was determined by a Hugo Sachs Electronik stimulator which delivered a constant current (0.3 sec duration; from 1-300mA in steps of 5-20mA). . The procedure is similar to that outlined above for mouse and full details are as published by Upton et al,.⁴

The percentage increase or decrease in CC₅₀ for each group compared to the control is calculated.

Drugs are suspended in 1% methyl cellulose.

### References

1. Loscher, W. and Schmidt, D. (1988). Epilepsy Res., **2,** 145-181
2. Dixon, W.J. and Mood, A.M. (1948). J. Amer. Stat. Assn., **43,** 109-126
3. Litchfield, J.T. and Wilcoxon, F.(1949). J. Pharmacol. exp. Ther., **96**, 99-113
4. N.Upton, T.P.Blackburn, C.A.Campbell, D.Cooper, M.L.Evans, H.J.Herdon, P.D.King, A.M.Ray, T.O.Stean, W.N.Chan, J.M.Evans and M.Thompson. (1997). B. J. Pharmacol., **121**, 1679-1686

### Results for rat MEST

Compounds of this invention dosed by the oral route as a suspension in methyl cellulose and tested one hour post dosing show an increase in seizure threshold. For example, at a dose of 2 mg/kg p.o. the compounds of Examples 3 and 4 showed statistically significant increases of 426 and 605 % respectively.

## Claims

1. A compound of formula (I) or salt thereof or solvate thereof: 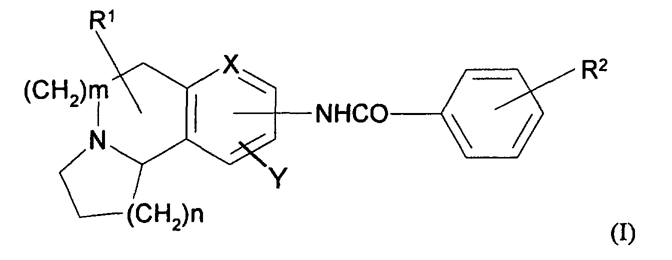 in which;
m is 1 or 2;
n is 1 or 2;
X is CH or N;
Y is selected from hydrogen, halogen, cyano, CF₃, alkyl or alkoxy;
R¹, which may be at any position within the saturated ring system, is hydrogen or up to two substituents which may be the same or different and each of which is selected from fluoro and C₁₋₆ alkyl;
R² is hydrogen or up to four substituents selected from halogen, NO₂, CN, N₃, CF₃O-, CF₃S-, CF₃CO-, C₁₋₆alkyl, C₁₋₆alkenyl, C₁₋₆alkynyl,
C₁₋₆perfluoroalkyl, C₃₋₆cycloalkyl, C₃₋₆cycloalkyl-C₁₋₄alkyl-, C₁₋₆alkylO-, C₁₋₆alkylCO-, C₃₋₆cycloalkylO-, C₃₋₆cycloalkylCO-,
C₃₋₆cycloalkyl-C₁₋₄alkylO-, C₃₋₆cycloalkyl-C₁₋₄alkylCO-, phenyl, phenoxy, benzyloxy, benzoyl, phenyl-C₁₋₄alkyl-, C₁₋₆alkylS-, C₁₋₆alkylSO₂-,
(C₁₋₄alkyl)₂NSO₂-, (C₁₋₄alkyl)NHSO₂-, (C₁₋₄alkyl)₂NCO-, oxazolyl, (C₁₋₄alkyl)NHCO-, CONH₂;
or R⁴CONH-or -NR⁴R⁵
wherein R⁴ is hydrogen or C₁₋₄ alkyl, and;
R⁵ is hydrogen, C₁₋₄alkyl, formyl, -CO₂C₁₋₄alkyl or -COC₁₋₄alkyl;
or two R² groups are linked together form a carbocyclic or heterocyclic ring that is saturated or unsaturated and unsubstituted or substituted by -OH or =O.

2. A compound of formula (I) according to claim 1 wherein:
R¹ is hydrogen, fluoro, methyl, ethyl or propyl;
R² is hydrogen or one or more of methyl, ethyl, *n*-butyl, phenyl, *iso*-propyl, *t*-butyl, methoxy, ethoxy, n-propoxy, *iso*-propoxy, *n*-butoxy, phenoxy, benzyloxy, bromo, chloro, iodo, fluoro, nitro, cyano, acetyl, pivaloyl, *iso*-butyroyl, benzoyl, trifluoromethyl, trifluoromethoxy, trifluoroacetyl, amino, acetylamino, methylthio, oxazolo, methylsulfonyl, *n*-propylsulfonyl, isopropylsulfonyl or dimethylsulfamoyl.

3. A compound of formula (I) according to claim 1 or claim 2 wherein:
R¹ is hydrogen, and;
R² is hydrogen or one or more of ethyl, methoxy, isopropoxy, trifluoromethyl, cyano, chloro, fluoro.

4. A compound according to any one of claims 1 to 3 selected from:
3-cyano-N-(1,2,3,5,6,10b-hexahydropyrrolo[2,1-a]isoquinolin-9-yl)-4-methoxybenzamide;
3-bromo-4-ethyl-N-(1,2,3,5,6,10b-hexahydropyrrolo[2,1-a]isoquinolin-9-yl)-benzamide;
N-(1,2,3,5,6,10b-hexahydropyrrolo[2,1-a]isoquinolin-9-yl)-4-methoxy-3-trifluoromethylbenzamide;
3-bromo-4-ethoxy-N-( 1,2,3,5,6,10b-hexahydropyrrolo[2,1-a]isoquinolin-9-yl)benzamide;
3-bromo-4-ethoxy-N-(1,3,4,6,7,11b-hexahydro-2H-pyrido[2,1-a]isoquinolin-10-yl)benzamide;
N-( 1,2,3,5,6,10b-hexahydropyrrolo[2,1-a]isoquinolin-9-yl)-4-methoxy-3-propionylbenzamide;
N-(1,2,3,5,6,10b-hexahydropyrrolo[2,1-a]isoquinolin-9-yl)-4-ethoxy-3-propionylbenzamide;
3-acetyl-N-(1,2,3,5,6,10b-hexahydropyrrolo[2,1-a]isoquinolin-9-yl)benzamide;
3-acetyl-N-(1,2,3,5,6,10b-hexahydropyrrolo[2,1-a]isoquinolin-9-yl)-4-methoxybenzamide;
3-fluoro-N-(1,2,3,5,6,10b-hexahydropyrrolo[2,1-a]isoquinolin-9-yl)-4-methoxybenzamide;
3,5-dichloro-4-ethoxy-N-(1,2,3,5,6,10b-hexahydropyrrolo[2,1-a]isoquinolin-9-yl)benzamide;
3-acetyl-N-(1,2,3,5,6,10b-hexahydropyrrolo[2,1-a]isoquinolin-9-yl)-4-propoxybenzamide;
3-butyryl-N-(1,2,3,5,6,10b-hexahydropyrrolo[2,1-a]isoquinolin-9-yl)-4-methoxybenzamide;
3-*iso*-butyryl-N-(1,2,3,5,6,10b-hexahydropyrrolo[2,1-a]isoquinolin-9-yl)-4-*iso*propoxybenzamide;
3-cyano-N-(1,2,3,5,6,10b-hexahydropyrrolo[2,1-a]isoquinolin-9-yl)-4-*iso*propylbenzamide;
4-oxo-chroman-6-carboxylic acid (1,2,3,5,6,10b-hexahydropyrrolo[2,1-a]isoquinolin-9-yl)amide;
N-(1,2,3,5,6,10b-hexahydropyrrolo[2,1-a]isoquinolin-9-yl)-4-isopropoxy-3-propionylbenzamide;
N-(1,2,3,5,6,10b-hexahydropyrrolo[2,1-a]isoquinolin-9-yl)-3-isobutyryl-4-methoxybenzamide;
N-(1,2,3,5,6,10b-hexahydropyrrolo[2,1-a]isoquinolin-9-yl)-4-ethoxy-3-isobutyrylbenzamide;
N-(1,2,3,5,6,10b-hexahydropyrrolo[2,1-a]isoquinolin-9-yl)-4-ethoxy-3-fluorobenzamide;
N-( 1,2,3,5,6,10b-hexahydropyrrolo[2,1-a]isoquinolin-9-yl)-4-isopropoxy-3-fluorobenzamide;
3-cyano-N-(1,2,3,5,6,10b-hexahydropyrrolo[2,1-a]isoquinolin-9-yl)-4-isopropoxybenzamide;
3-cyano-4-ethoxy-N-(1,2,3,5,6,10b-hexahydropyrrolo[2,1-a]isoquinolin-9-yl)-benzamide;
N-(1,2,3,5,6,10b-hexahydropyrrolo[2,1-a]isoquinolin-9-yl)-3-propionyl-4-propoxybenzamide;
3-acetyl-4-ethyl-N-(1,2,3,5,6,10b-hexahydropyrrolo[2,1-a]isoquinolin-9-yl)benzamide;
3-acetyl-4-chloro-N-(1,2,3,5,6,10b-hexahydropyrrolo[2,1-a]isoquinolin-9-yl)benzamide;
3-acetyl-4-bromo-N-(1,2,3,5,6,10b-hexahydropyrrolo[2,1-a]isoquinolin-9-yl)benzamide;
3-acetyl-5-bromo-N-(1,2,3,5,6,10b-hexahydropyrrolo[2,1-a]isoquinolin-9-yl)benzamide;
3-cyano-4-ethoxy-N-(1,2,3,5,6,10b-hexahydropyrrolo[2,1-a]isoquinolin-9-yl)benzamide;
3-cyano-N-(1,2,3,5,6,10b-hexahydropyrrolo[2,1-a]isoquinolin-9-yl)-4-isopropoxybenzamide;
3-acetyl-4-acetylamino-N-(1,2,3,5,6,10b-hexahydropyrrolo[2,1-a]isoquinolin-9-yl)benzamide;
3-acetyl-N-(1,2,3,5,6,10b-hexahydropyrrolo[2,1-a]isoquinolin-9-yl)-4-isopropylbenzamide;
N-(1,2,3,5,6,10b-hexahydropyrrolo[2,1-a]isoquinolin-9-yl)-3-propionyl-4-isopropoxybenzamide;
3-acetyl-4-ethoxy-N-(1,2,3,5,6,10b-hexahydropyrrolo[2,1-a]isoquinolin-9-yl)-benzamide;
3-cyano-N-(1,2,3,5,6,10b-hexahydropyrrolo[2,1-a]isoquinolin-9-yl)-4-methoxybenzamide;
N-(1,2,3,5,6,10b-hexahydropyrrolo[2,1-a]isoquinolin-9-yl)-4-methoxy-3-trifluoromethylbenzamide;
3-fluoro-N-(1,2,3,5,6,10b-hexahydropyrrolo[2,1-a]isoquinolin-9-yl)-4-methoxybenzamide;
4-ethoxy-3-fluoro-N-(1,2,3,5,6,10b-hexahydropyrrolo[2,1-a]isoquinolin-9-yl)benzamide;
3-butyryl-N-(1,2,3,5,6,10b-hexahydropyrrolo[2,1-a]isoquinolin-9-yl)-4-propoxybenzamide;
3-butyryl-N-(1,2,3,5,6,10b-hexahydropyrrolo[2,1-a]isoquinolin-9-yl)-4-*iso*propoxybenzamide;
3-acetyl-N-(1,2,3,5,6,10b-hexahydropyrrolo[2,1-a]isoquinolin-9-yl)-4-propoxybenzamide;
3-butyryl-4-ethoxy-N-(1,2,3,5,6,10b-hexahydropyrrolo[2,1-a]isoquinolin-9-yl)benzamide;
3-iso-butyryl-N-(1,2,3,5,6,10b-hexahydropyrrolo[2,1-a]isoquinolin-9-yl)-4-propoxybenzamide;
3-acetyl-N-(1,2,3,5,6,10b-hexahydropyrrolo[2,1-a]isoquinolin-9-yl)-4-*iso*propoxybenzamide;
3-chloro-N-(1,2,3,5,6,10b-hexahydropyrrolo[2,1-a]isoquinolin-9-yl)-4-*iso*propoxybenzamide;
3-cyano-N-( 1,2,3,5,6,10b-hexahydropyrrolo[2,1-a]isoquinolin-9-yl)-4-*iso*propylbenzamide;
3-bromo-N-(1,2,3,5,6,10b-hexahydropyrrolo[2,1-a]isoquinolin-9-yl)-4-*iso*propoxybenzamide;
5-acetyl-N-( 1,2,3,5,6,10b-hexahydropyrrolo[2,1-a]isoquinolin-9-yl)-2-methoxy-4-isopropoxybenzamide;
N-(1,2,3,5,6,10b-hexahydropyrrolo[2,1-a]isoquinolin-9-yl)-3-pivaloylbenzamide;
N-( 1,2,3,5,6,10b-hexahydropyrrolo[2,1-a]isoquinolin-9-yl)-2-methoxy-4-isopropyl-5-trifluoromethylbenzamide;
naphthalene-2-carboxylic acid (1,2,3,5,6,10b-hexahydropyrrolo[2,1-a]isoquinolin-9-yl)amide;
benzothiazole-5-carboxylic acid (1,2,3,5,6,10b-hexahydropyrrolo[2,1-a]isoquinolin-9-yl)amide;
2,3-dihydrobenzofuran-5-carboxylic acid (1,2,3,5,6,10b-hexahydropyrrolo[2,1-a]isoquinolin-9-yl)carboxamide;
3-acetyl-N-(1,2,3,5,6,10b-hexahydropyrrolo[2,1-a]isoquinolin-9-yl)-4-isopropoxybenzamide;
3-chloro-4-ethoxy-N-(1,2,3,5,6,10b-hexahydropyrrolo[2,1-a]isoquinolin-9-yl)benzamide;
N-3-(N, N-dimethylcarboxamido)-(1,2,3,5,6,10b-hexahydropyrrolo[2,1-a]isoquinolin-9-yl)-4-isopropoxybenzamide;
N-(6,6-dimethyl-1,2,3,5,6,10b-hexahydropyrrolo[2,1-a]isoquinolin-9-yl)-4-methoxy-3 -trifluoromethylbenzamide;
3-bromo-N-(6,6-dimethyl-1,2,3,5,6,10b-hexahydropyrrolo[2,1-a]isoquinolin-9-yl)-4-ethoxybenzamide;
3-bromo-N-(6,6-dimethyl-1,2,3,5,6,10b-hexahydropyrrolo[2,1-a]isoquinolin-9-yl)-4-*iso*propoxybenzamide;
3-cyano-N-(6,6-dimethyl-1,2,3,5,6,10b-hexahydropyrrolo[2,1-a]isoquinolin-9-yl)-4-*iso*propylbenzamide;
3-acetyl-N-(6,6-dimethyl-1,2,3,5,6,10b-hexahydropyrrolo[2,1-a]isoquinolin-9-yl)-4-*iso*propoxybenzamide;
N-(6,6-dimethyl-1,2,3,5,6,10b-hexahydropyrrolo [2,1-a]isoquinolin-9-yl)-3-fluoro-4-methoxybenzamide;
N-(6,6-dimethyl-1,2,3,5,6,10b-hexahydropyrrolo[2,1-a]isoquinolin-9-yl)-3-fluoro-4-ethoxybenzamide;
N-(6,6-dimethyl-1,2,3,5,6,10b-hexahydropyrrolo[2,1-a]isoquinolin-9-yl)-3-fluoro-4-*iso*propoxybenzamide;
3-cyano-N-(6,6-dimethyl-1,2,3,5,6,10b-hexahydropyrrolo[2,1-a]isoquinolin-9-yl)-4-methoxybenzamide;
3-acetyl-N-(6,6-dimethyl-1,2,3,5,6,10b-hexahydropyrrolo[2,1-a]isoquinolin-9-yl)-4-methoxybenzamide;
N-(6,6-dimethyl-1,2,3,5,6,10b-hexahydropyrrolo[2,1-a]isoquinolin-9-yl)-3-fluoro-4-methoxybenzamide;
N-(7-chloro-1,2,3,5,6,10b-hexahydropyrrolo[2,1-a]isoquinolin-9-yl)-4-methoxy-3 -trifluoromethylbenzamide;
10b-methyl-N-(1,2,3,5,6,10b-hexahydropyrrolo [2,1-a]isoquinolin-9-yl)-4-methoxy-3-trifluoromethylbenzamide;
3-bromo-N-4-ethoxy(6-methyl-1,2,3,5,6,10b-hexahydropyrrolo[2,1-a]isoquinolin-9-yl)-4-isopropoxybenzamide;
N-(6-methyl-1,2,3,5,6,10b-hexahydropyrrolo[2,1-a]isoquinolin-9-yl)-4-methoxy-3 -trifluoromethylbenzamide;
N-(5-methyl-1,2,3,5,6,10b-hexahydropyrrolo[2,1-a]isoquinolin-9-yl)-4-methoxy-3 -trifluoromethylbenzamide;
3-acetyl-N-(5-methyl-1,2,3,5,6,10b-hexahydropyrrolo[2,1-a]isoquinolin-9-yl)-4-isopropoxybenzamide;
3-acetyl-N-(2,3,5,6,7,11b-hexahydro-1H-benzo[c]pyrrolo[1,2-a]azepin-10-yl)-4-isopropoxybenzamide;
N-(2,3,5,6,7,11b-hexahydro-1H-benzo[c]pyrrolo[1,2-a]azepin-10-yl)-4-methoxy-3-trifluoromethylbenzamide;
4-*tert*-butyl-2-methoxy-N-( 1,2,3,5,6,10b-hexahydropyrrolo[2,1-a]isoquinolin-7-yl)benzamide;
5-cyano-2-ethoxy-4-*iso*propyl-N-(2,3,5,6,6a,10a,10b-octahydro-1H-pyrrolo[2,1-a]isoquinolin-7-yl)benzamide, and;
N-(5,6,8,9,10,10a-hexahydropyrrolo[2,1-f][1,6]naphthridin-2-yl)-4-methoxy-3-trifluoromethylbenzamide.

5. A compound of formula (I) according to claim 1 selected from:
(+) 3-Cyano-N-(1,2,3,5,6,10b-hexahydropyrrolo[2,1-a]isoquinolin-9-yl)-4-isopropoxybenzamide and
(+) 3-Cyano-4-ethoxy-N-(1,2,3,5,6,10b-hexahydropyrrolo[2,1-a]isoquinolin-9-yl)-benzamide.

6. A process for the preparation of compounds of formula (I) as defined in claim 1, or salts thereof or solvates thereof, which comprises reacting a compound of formula (II) 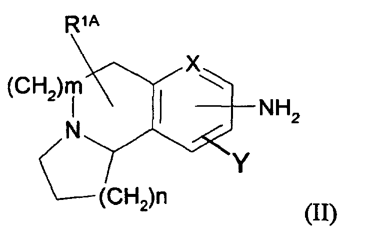 with a compound of formula (III) 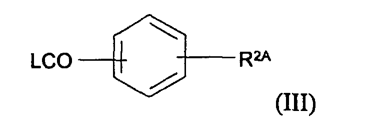 where R^{1A} and R^{2A} are R¹ and R² respectively as defined for formula (I) as defined in claim 1 or a group or groups convertible to R¹ or R² groups; and L is OH, acyloxy, or a halogen, and where required;
converting an R^{1A} or R^{2A} group to an R¹ or R² group;
converting one R¹ or R² group to another R¹ or R² group;
converting a salt product to the free base or another pharmaceutically acceptable salt;
or converting a free base product to a pharmaceutically acceptable salt.

7. A pharmaceutical composition for use in the treatment and/or prophylaxis of anxiety, mania, depression, panic disorders and/or aggression, disorders associated with a subarachnoid haemorrhage or neural shock, the effects associated with withdrawal from substances of abuse such as cocaine, nicotine, alcohol and benzodiazepines, disorders treatable and/or preventable with anti-convulsive agents, such as epilepsy including post-traumatic epilepsy, Parkinson's disease, psychosis, migraine, cerebral ischaemia, Alzheimer's disease and other degenerative diseases such as Huntingdon's chorea, schizophrenia, obsessive compulsive disorders (OCD), neurological deficits associated with AIDS, sleep disorders (including circadian rhythm disorders, insomnia & narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, multiple sclerosis (MS) and motor neurone disease, ataxias, muscular rigidity (spasticity), temporomandibular joint dysfunction, and amyotrophic lateral sclerosis (ALS) which comprises a compound of formula (I) as defined in any one of claims 1 to 5, or a pharmaceutically acceptable salt or solvate thereof, and a pharmaceutically acceptable carrier.

8. A compound of formula (I) as defined in any one of claims 1 to 5, or a pharmaceutically acceptable salt or solvate, thereof for use as a therapeutic agent, in particular for the treatment and/or prophylaxis of anxiety, mania, depression, panic disorders and/or aggression, disorders associated with a subarachnoid haemorrhage or neural shock, the effects associated with withdrawal from substances of abuse such as cocaine, nicotine, alcohol and benzodiazepines, disorders treatable and/or preventable with anti-convulsive agents, such as epilepsy including post-traumatic epilepsy, Parkinson's disease, psychosis, migraine, cerebral ischaemia, Alzheimer's disease and other degenerative diseases such as Huntingdon's chorea, schizophrenia, obsessive compulsive disorders (OCD), neurological deficits associated with AIDS, sleep disorders (including circadian rhythm disorders, insomnia & narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, multiple sclerosis (MS) and motor neurone disease, ataxias, muscular rigidity (spasticity), temporomandibular joint dysfunction, and amyotrophic lateral sclerosis (ALS).

9. Use of a compound of formula (I) as defined in any one of claims 1 to 5, or a pharmaceutically acceptable salt or solvate thereof, for the manufacture of a medicament for the treatment and/or prophylaxis of anxiety, mania, depression, panic disorders and/or aggression, disorders associated with a subarachnoid haemorrhage or neural shock, the effects associated with withdrawal from substances of abuse such as cocaine, nicotine, alcohol and benzodiazepines, disorders treatable and/or preventable with anti-convulsive agents, such as epilepsy including post-traumatic epilepsy, Parkinson's disease, psychosis, migraine, cerebral ischaemia, Alzheimer's disease and other degenerative diseases such as Huntingdon's chorea, schizophrenia, obsessive compulsive disorders (OCD), neurological deficits associated with AIDS, sleep disorders (including circadian rhythm disorders, insomnia & narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, multiple sclerosis (MS) and motor neurone disease, ataxias, muscular rigidity (spasticity), temporomandibular joint dysfunction, and amyotrophic lateral sclerosis (ALS).

## Patentansprüche

1. Verbindung der Formel (I) oder ein Salz oder Solvat davon: 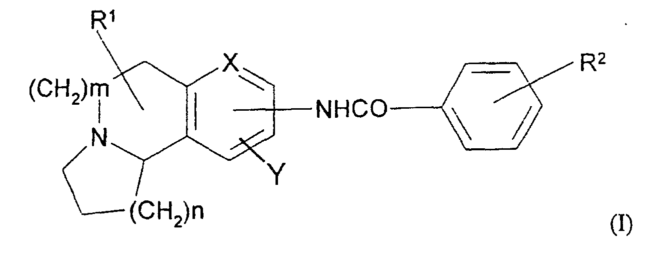 in welcher
m 1 oder 2 ist;
n 1 oder 2 ist;
X CH oder N ist;
Y ausgewählt ist aus einem Wasserstoffatom, einem Halogenatom, einer Cyanogruppe, einer Gruppe CF₃, einem Alkylrest oder einem Alkoxyrest;
R¹, welches eine beliebige Position innerhalb des gesättigten Ringsystems einnehmen kann, ein Wasserstoffatom oder bis zu zwei Substituenten darstellt, die gleichartig oder unterschiedlich sein können und jeweils ausgewählt sind aus einem Fluoralom und einem C₁₋₆-Alkylrest;
R² ein Wasserstoffatom oder bis zu vier Substituenten darstellt, die ausgewählt sind aus einem Halogenatom, einer der Gruppen NO₂, CN, N₃, CF₃O-, CF₃S-, CF₃CO-, einem C₁₋₆-Alkyl-, C₁₋₆-Alkenyl-, C₁₋₆-Alkinyl, C₁₋₆-Perfluoralky-, C₃₋₆-Cycloalkyl-, C₃₋₆-Cycloalkyl-C₁₋₄-alkylrest, einem der Reste C₁₋₆-AlkylO-, C₁₋₆-AlkylCO-, C₃₋₆-CycloalkylO-, C₃₋₆-CycloalkylCO-, C₃₋₆-Cycloalkyl-C₁₋₄-alkylO-, C₃₋₆-Cycloalkyl-C₁₋₄-alkylCO-, einer Phenyl-, Phenoxy-, Benzyloxy- oder Benzoylgruppe, einem Phenyl-C₁₋₄-alkylrest, einem der Reste C₁₋₆-AlkylS-, C₁₋₆-AlkylSO₂-, (C₁₋₄-Alkyl)₂NSO₂-, (C₁₋₄-Alkyl)NHSO₂- (C₁₋₄-Alkyl)₂NCO-, einer Oxazolylgruppe, einem Rest (C₁₋₄-Alkyl)NHCO-, einer Gruppe CONH₂;
oder einem der Reste R⁴CONH- oder -NR⁴R⁵,
wobei R⁴ ein Wasserstoffatom oder ein C₁₋₄-Alkylrest ist; und
R⁵ ein Wasserstoffatom, ein C₁₋₄-Alkylrest, eine Formylgruppe, ein Rest -CO₂C₁₋₄-Alkyl oder ein Rest -COC₁₋₄-Alkyl ist;
oder zwei Reste R² verbunden sind, um einen carbocyclischen oder heterocyclischen Ring zu bilden, der gesättigt oder ungesättigt und unsubstituiert oder durch eine Gruppe -OH oder =O substituiert ist.

2. Verbindung der Formel (I) gemäß Anspruch 1, wobei:
R¹ ein Wasserstoffatom, ein Fluoratom oder eine Methyl-, Ethyl- oder Propylgruppe ist;
R² ein Wasserstoffatom oder eine oder mehrere aus einer Methyl-, Ethyl-, *n*-Butyl-, Phenyl-, *iso*-Propyl-, *t*-Butyl-, Methoxy-, Ethoxy-, *n*-Propoxy-, *iso*-Propoxy-, *n*-Butoxy-, Phenoxy- oder Benzyloxygruppe, einem Brom-, Chlor-, Iod- oder Fluoratom, einer Nitro-, Cyano-, Acetyl-, Pivaloyl-, *iso*-Butyroyl-, Benzoyl-, Trifluormethyl-, Trifluormethoxy-, Trifluoracetyl-, Amino-, Acetylamino-, Methylthio-, Oxazolo-, Methylsulfonyl-, *n*-Propylsulfonyl-, Isopropylsulfonyl- oder Dimethylsulfamoylgruppe ist.

3. Verbindung der Formel (I) gemäß Anspruch 1 oder 2, wobei:
R¹ ein Wasserstoffatom ist; und
R² ein Wasserstoffatom oder eine oder mehrere aus einer Ethyl-, Methoxy-, Isopropoxy-, Trifluormethyl- oder Cyanogruppe, einem Chlor- oder Fluoratom ist.

4. Verbindung gemäß einem der Ansprüche 1 bis 3, ausgewählt aus:
3-Cyano-N-(1,2,3,5,6,10b-hexahydropyrrolo[2,1-a]isochinolin-9-yl)-4-methoxybenzamid;
3-Brom-4-ethyl-N-(1,2,3,5,6,10b-hexahydropyrrolo[2,1-a]isochinolin-9-yl)-benzamid; N-(1,2,3,5,6,10b-Hexahydropyrrolo[2,1-a]isochinolin-9-yl)-4-methoxy-3-trifluormethylbenzamid;
3-Brom-4-ethoxy-N-(1,2,3,5,6,10b-hexahydropyrrolo[2,1-a]isochinolin-9-yl)benzamid;
3-Brom-4-ethoxy-N-( 1,3,4,6,7,11b-hexahydro-2H-pyrido[2,1-a]isochinolin-10-yl)benzamid;
N-(1,2,3,5,6,10b-Hexahydropyrrolo[2,1-a]isochinolin-9-yl)-4-methoxy-3-propionylbenzamid;
N-(1,2,3,5,6,10b-Hexahydropyrrolo[2,1-a]isochinolin-9-yl)-4-ethoxy-3-propionylbenzamid;
3-Acetyl-N-(1,2,3,5,6,10b-hexahydropyrrolo[2,1-a]isochinolin-9-yl)benzamid;
3-Acetyl-N-(1,2,3,5,6,10b-hexahydropyrrolo[2,1-a]isochinolin-9-yl)-4-methoxybenzamid;
3-Fluor-N-(1,2,3,5,6,10b-hexahydropyrrolo[2,1-a]isochinolin-9-yl)-4-methoxybenzamid;
3,5-Dichlor-4-ethoxy-N-(1,2,3,5,6,10b-hexahydropyrrolo[2,1-a]isochinolin-9-yl)benzamid;
3-Acetyl-N-(1,2,3,5,6,10b-hexahydropyrrolo[2,1-a]isochinolin-9-yl)-4-propoxybenzamid;
3-Butyryl-N-(1,2,3,5,6,10b-hexahydropyrrolo[2,1-a]isochinolin-9-yl)-4-methoxybenzamid;
3-*iso*-Butyryl-N-(1,2,3,5,6,10b-hexahydropyrrolo[2,1-a]isochinolin-9-yl)-4-*iso*propoxybenzamid;
3-Cyano-N-(1,2,3,5,6,10b-hexahydropyrrolo[2,1-a]isochinolin-9-yl)-4-*iso*propylbenzamid;
4-Oxo-chroman-6-carbonsäure-(1,2,3,5,6,10b-hexahydropyrrolo[2,1-a]isochinolin-9-yl)amid;
N-(1,2,3,5,6,10b-Hexahydropyrrolo[2,1-a]isochinolin-9-yl)-4-isopropoxy-3-propionylbenzamid;
N-(1,2,3,5,6,10b-Hexahydropyrrolo[2,1-a]isochinolin-9-yl)-3-isobutyryl-4-methoxybenzamid;
N-(1,2,3,5,6,10b-Hexahydropyrrolo[2,1-a]isochinolin-9-yl)-4-ethoxy-3-isobutyrylbenzamid;
N-(1,2,3,5,6,10b-Hexahydropyrrolo[2,1-a]isochinolin-9-yl)-4-ethoxy-3-fluorbenzamid;
N-(1,2,3,5,6,10b-Hexahydropyrrolo[2,1-a]isochinolin-9-yl)-4-isopropoxy-3-fluorbenzamid;
3-Cyano-N-(1,2,3,5,6,10b-hexahydropyrrolo[2,1-a]isochinolin-9-yl)-4-isopropoxybenzamid;
3-Cyano-4-ethoxy-N-(1,2,3,5,6,10b-hexahydropyrrolo[2,1-a]isochinolin-9-yl)-benzamid;
N-(1,2,3,5,6,10b-Hexahydropyrrolo[2,1-a]isochinolin-9-yl)-3-propionyl-4-propoxybenzamid;
3-Acetyl-4-ethyl-N-(1,2,3,5,6,10b-hexahydropyrrolo[2,1-a]isochinolin-9-yl)benzamid;
3-Acetyl-4-chlor-N-(1,2,3,5,6,10b-hexahydropyrrolo[2,1-a]isochinolin-9-yl)benzamid;
3-Acetyl-4-brom-N-(1,2,3,5,6,10b-hexahydropyrrolo[2,1-a]isochinolin-9-yl)benzamid;
3-Acetyl-5-brom-N-(1,2,3,5,6,10b-hexahydropyrrolo[2,1-a]isochinolin-9-yl)benzamid;
3-Cyano-4-ethoxy-N-(1,2,3,5,6,10b-hexahydropyrrolo[2,1-a]isochinolin-9-yl)benzamid;
3-Cyano-N-(1,2,3,5,6,10b-hexahydropyrrolo[2,1-a]isochinolin-9-yl)-4-isopropoxybenzamid;
3-Acetyl-4-acetylamino-N-(1,2,3,5,6,10b-hexahydropyrrolo[2,1-a]isochinolin-9-yl)benzamid;
3-Acetyl-N-(1,2,3,5,6,10b-hexahydropyrrolo[2,1-a]isochinolin-9-yl)-4-isopropylbenzamid;
N-(1,2,3,5,6,10b-Hexahydropyrrolo[2,1-a]isochinolin-9-yl)-3-propionyl-4-isopropoxybenzamid;
3-Acetyl-4-ethoxy-N-(1,2,3,5,6,10b-hexahydropyrrolo[2,1-a]isochinolin-9-yl)benzamid;
3-Cyano-N-(1,2,3,5,6,10b-hexahydropyrrolo[2,1-a]isochinolin-9-yl)-4-methoxybenzamid;
N-(1,2,3,5,6,10b-Hexahydropyrrolo[2,1-a]isochinolin-9-yl)-4-methoxy-3-trifluonnethylbenzamid;
3-Fluor-N-(1,2,3,5,6,10b-hexahydropyrrolo[2,1-a]isochinolin-9-yl)-4-methoxybenzamid;
4-Ethoxy-3-fluor-N-(1,2,3,5,6,10b-hexahydropyrrolo[2,1-a]isochinolin-9-yl)benzamid;
3-Butyryl-N-(1,2,3,5,6,10b-hexahydropyrrolo[2,1-a]isochinolin-9-yl)-4-propoxybenzamid;
3-Butyryl-N-(1,2,3,5,6,10b-hexahydropyrrolo[2,1-a]isochinolin-9-yl)-4-*iso*propoxybenzamid;
3-Acetyl-N-(1,2,3,5,6,10b-hexahydropyrrolo[2,1-a]isochinolin-9-yl)-4-propoxybenzamid;
3-Butyryl-4-ethoxy-N-(1,2,3,5,6,10b-hexahydropyrrolo[2,1-a]isochinolin-9-yl)benzamid;
3-*iso*-Butyryl-N-(1,2,3,5,6,10b-hexahydropyrrolo[2,1-a]isochinolin-9-yl)-4-propoxybenzamid;
3-Acetyl-N-(1,2,3,5,6,10b-hexahydropyrrolo[2,1-a]isochinolin-9-yl)-4-*iso*propoxybenzamid;
3-Chlor-N-(1,2,3,5,6,10b-hexahydropyrrolo[2,1-a]isochinolin-9-yl)-4-*iso*propoxybenzamid;
3-Cyano-N-(1,2,3,5,6,10b-hexahydropyrrolo[2,1-a]isochinolin-9-yl)-4-*iso*propylbenzamid;
3-Brom-N-(1,2,3,5,6,10b-hexahydropyrrolo[2,1-a]isochinolin-9-yl)-4-*iso*propoxybenzamid;
5-Acetyl-N-(1,2,3,5,6,10b-hexahydropyrrolo[2,1-a]isochinolin-9-yl)-2-methoxy-4-isopropoxybenzamid;
N-(1,2,3,5,6,10b-Hexahydropyrrolo[2,1-a]isochinolin-9-yl)-3-pivaloylbenzamid;
N-(1,2,3,5,6,10b-Hexahydropyrrolo[2,1-a]isochinolin-9-yl)-2-methoxy-4-isopropyl-5-trifluormethylbenzamid;
Naphthalin-2-carbonsäure-(1,2,3,5,6,10b-hexahydropyrrolo[2,1-a]isochinolin-9-yl)amid;
Benzothiazol-5-carbonsäure-(1,2,3,5,6,10b-hexahydropyrrolo[2,1-a]isochinolin-9-yl)amid;
2,3-Dihydrobenzofuran-5-carbonsäure-(1,2,3,5,6,10b-hexahydropyrrolo[2,1-a]isochinolin-9-yl)carboxamid;
3-Acetyl-N-(1,2,3,5,6,10b-hexahydropyrrolo[2,1-a]isochinolin-9-yl)-4-isopropoxybenzamid;
3-Chlor-4-ethoxy-N-(1,2,3,5,6,10b-hexahydropyrrolo[2,1-a]isochinolin-9-yl)benzamid;
N-3-(N,N-Dimethylcarboxamido)-(1,2,3,5,6,10b-hexahydropyrrolo[2,1-a]isochinolin-9-yl)-4-isopropoxybenzamid;
N-(6,6-Dimethyl-1,2,3,5,6,10b-hexahydropyrrolo[2,1-a]isochinolin-9-yl)-4-methoxy-3-trifluormethylbenzamid;
3-Brom-N-(6,6-dimethyl-1,2,3,5,6,10b-hexahydropyrrolo[2,1-a]isochinolin-9-yl)-4-ethoxybenzamid;
3-Brom-N-(6,6-dimethyl-1,2,3,5,6,10b-hexahydropyrrolo[2,1-a]isochinolin-9-yl)-4-*iso*propoxybenzamid;
3-Cyano-N-(6,6-dimethyl-1,2,3,5,6,10b-hexahydropyrrolo[2,1-a]isochinolin-9-yl)-4-*iso*propylbenzamid;
3-Acetyl-N-(6,6-dimethyl-1,2,3,5,6,10b-hexahydropyrrolo[2,1-a]isochinolin-9-yl)-4-*iso*propoxybenzamid;
N-(6,6-Dimethyl-1,2,3,5,6,10b-hexahydropyrrolo[2,1-a]isochinolin-9-yl)-3-fluor-4-methoxybenzamid;
N-(6,6-Dimethyl-1,2,3,5,6,10b-hexahydropyrrolo[2,1-a]isochinolin-9-yl)-3-fluor-4-ethoxybenzamid;
N-(6,6-Dimethyl-1,2,3,5,6,10b-hexahydropyrrolo[2,1-a]isochinolin-9-yl)-3-fluor-4-isopropoxybenzamid;
3-Cyano-N-(6,6-dimethyl-1,2,3,5,6,10b-hexahydropyrrolo[2,1-a]isochinolin-9-yl)-4-methoxybenzamid;
3-Acetyl-N-(6,6-dimethyl-1,2,3,5,6,10b-hexahydropyrrolo[2,1-a]isochinolin-9-yl)-4-methoxybenzamid;
N-(6,6-Dimethyl-1,2,3,5,6,10b-hexahydropyrrolo[2,1-a]isochinolin-9-yl)-3-fluor-4-methoxybenzamid;
N-(7-Chlor-1,2,3,5,6,10b-hexahydropyrrolo[2,1-a]isochinolin-9-yl)-4-methoxy-3-trifluormethylbenzamid;
10b-Methyl-N-(1,2,3,5,6,10b-hexahydropyrrolo[2,1-a]isochinolin-9-yl)-4-methoxy-3-trifluormethylbenzamid;
3-Brom-N-4-ethoxy(6-methyl-1,2,3,5,6,10b-hexahydropyrrolo[2,1-a]isochinolin-9-yl)-4-*iso*propoxybenzamid;
N-(6-Methyl)-1,2,3,5,6,10b-hexahydropyrrolo[2,1-a]isochinolin-9-yl)-4-methoxy-3-trifluormethylbenzamid;
N-(5-Methyl-1,2,3,5,6,10b-hexahydropyrrolo[2,1-a]isochinolin-9-yl)-4-methoxy-3-trifluormethylbenzamid;
3-Acetyl-N-(5-methyl-1,2,3,5,6,10b-hexahydropyrrolo[2,1-a]isochinolin-9-yl)-4-isopropoxybenzamid;
3-Acetyl-N-(2,3,5,6,7,11b-hexahydro-1H-benzo[c]pyrrolo[1,2-a]azepin-10-yl)-4-isopropoxybenzamid;
N-(2,3,5,6,7,11b-Hexahydro-1H-benzo[c]pyrrolo[1,2-a]azepin-10-yl)-4-methoxy-3-trifluormethylbenzamid;
4*-tert*-Butyl-2-methoxy-N-(1,2,3,5,6,10b-hexahydropyrrolo[2,1-a]isochinolin-7-yl)benzamid;
5-Cyano-2-ethoxy-4-*iso*propyl-N-(2,3,5,6,6a,10a,10b-octahydro-1H-pyrrolo[2,1-a]isochinolin-7-yl)benzamid; und
N-(5,6,8,9,10,10a-Hexahydropyrrolo[2,1-f][1,6]naphthridin-2-yl)-4-methoxy-3-trifluormethylbenzamid.

5. Verbindung der Formel (I) gemäß Anspruch 1, ausgewählt aus:
(+)-3-Cyano-N-(1,2,3,5,6,10b-hexahydropyrrolo[2,1-a]isochinolin-9-yl)-4-isopropoxybenzamid und
(+)-3-Cyano-4-ethoxy-N-(1,2,3,5,6,10b-hexahydropyrrolo[2,1-a]isochinolin-9-yl)-benzamid.

6. Verfahren zur Herstellung von Verbindungen der Formel (I), welche die in Anspruch 1 angegebene Bedeutung haben, oder deren Salze oder Solvate, umfassend das Umsetzen einer Verbindung der Formel (II) 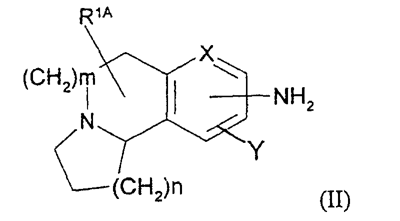 mit einer Verbindung der Formel (III) 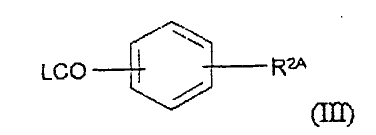 wobei R^{1A} und R^{2A} die Reste R¹ beziehungsweise R², welche die in Formel (I) in Anspruch 1 angegebene Bedeutung haben, oder ein Rest oder Reste sind, die zu R¹ oder R² umsetzbar sind; und L eine Gruppe OH, eine Acyloxygruppe oder ein Halogenatom ist;
und gegebenenfalls
das Umsetzen eines Restes R^{1A} oder R^{2A} zu einem Rest R¹ oder R²;
das Umsetzen eines Restes R¹ oder R² zu einem anderen Rest R¹ oder R²;
das Umsetzen eines Salzproduktes zu der freien Base oder einem anderen pharmazeutisch verträglichen Salz;
oder das Umsetzen eines Produkts in Form der freien Base zu einem pharmazeutisch verträglichen Salz.

7. Arzneimittel zur Verwendung in der Behandlung und/oder Prophylaxe von Angstzuständen, Manie, Depression, panischen Störungen und/oder Aggression, Störungen in Verbindung mit einer subarachnoiden Blutung oder neuralem Schock, den Effekten, die verbunden sind mit dem Entzug von zum Missbrauch geeigneten Substanzen, wie Kokain, Nikotin, Alkohol und Benzodiazepinen, Störungen, die mit Anticonvulsiva behandelbar und/oder verhütbar sind, wie Epilepsie, einschließlich post-traumatischer Epilepsie, Parkinson-Krankheit, Psychosen, Migräne, cerebraler Ischämie, Alzheimer-Krankheit und anderen degenerativen Erkrankungen wie Chorea Huntingdon, Schizophrenie, Zwangsneurosen (OCD), neurologischen Ausfällen in Verbindung mit AIDS, Schlafstörungen (einschließlich Tagesrhythmusstörungen, Schlaflosigkeit und Narkolepsie), Ticks (z.B. Giles-de-la-Tourette-Syndrom), traumatischen Gehirnverletzungen, Tinnitus, Neuralgie, insbesondere trigeminaler Neuralgie, neuropathischen Schmerzen, Zahnschmerzen, Schmerzen als Folge von Krebserkrankungen, unmäßiger neuronaler Aktivität, die zu Neurodysthesie führt, bei Erkrankungen wie Diabetes, Multipler Sklerose (MS) und Erkrankungen der motorischen Nerven, Ataxie, muskulärer Rigidität (Spastizität), Unterkiefergelenks-Dysfunktion und amyotropher Lateralsklerose (ALS), welche eine Verbindung der Formel (I), welche die in einem der Ansprüche 1 bis 5 angegebene Bedeutung hat, oder ein pharmazeutisch verträgliches Salz oder Solvat davon sowie einen pharmazeutisch verträglichen Träger umfasst.

8. Verbindung der Formel (I), welche die in einem der Ansprüche 1 bis 5 angegebene Bedeutung hat, oder ein pharmazeutisch verträgliches Salz oder Solvat davon zur Verwendung als Therapeutikum, insbesondere zur Behandlung und/oder Prophylaxe von Angstzuständen, Manie, Depression, panischen Störungen und/oder Aggression, Störungen in Verbindung mit einer subarachnoiden Blutung oder neuralem Schock, den Effekten, die verbunden sind mit dem Entzug von zum Missbrauch geeigneten Substanzen, wie Kokain, Nikotin, Alkohol und Benzodiazepinen, Störungen, die mit Anticonvulsiva behandelbar und/oder verhütbar sind, wie Epilepsie, einschließlich post-traumatischer Epilepsie, Parkinson-Krankheit, Psychosen, Migräne, cerebraler Ischämie, Alzheimer-Krankheit und anderen degenerativen Erkrankungen wie Chorea Huntingdon, Schizophrenie, Zwangsneurosen (OCD), neurologischen Ausfällen in Verbindung mit AIDS, Schlafstörungen (einschließlich Tagesrhythmusstörungen, Schlaflosigkeit und Narkolepsie), Ticks (z.B. Giles-de-la-Tourette-Syndrom), traumatischen Gehimverletzungen, Tinnitus, Neuralgie, insbesondere trigeminaler Neuralgie, neuropathischen Schmerzen, Zahnschmerzen, Schmerzen als Folge von Krebserkrankungen, unmäßiger neuronaler Aktivität, die zu Neurodysthesie führt, bei Erkrankungen wie Diabetes, Multipler Sklerose (MS) und Erkrankungen der motorischen Nerven, Ataxie, muskulärer Rigidität (Spastizität), Unterkiefergelenks-Dysfunktion und amyotropher Lateralsklerose (ALS).

9. Verwendung einer Verbindung der Formel (I), welche die in einem der Ansprüche 1 bis 5 angegebene Bedeutung hat, oder eines pharmazeutisch verträglichen Salzes oder Solvats davon für die Herstellung eines Medikaments zur Behandlung und/oder Prophylaxe von Angstzuständen, Manie, Depressionen, panischen Störungen und/oder Aggression, Störungen in Verbindung mit einer subarachnoiden Blutung oder neuralem Schock, den Effekten, die verbunden sind mit dem Entzug von zum Missbrauch geeigneten Substanzen, wie Kokain, Nikotin, Alkohol und Benzodiazepinen, Störungen, die mit Anticonvulsiva behandelbar und/oder verhütbar sind, wie Epilepsie, einschließlich post-traumatischer Epilepsie, Parkinson-Krankheit, Psychosen, Migräne, cerebraler Ischämie, Alzheimer-Krankheit und anderer degenerativer Erkrankungen wie Chorea Huntingdon, Schizophrenie, Zwangsneurosen (OCD), neurologischen Ausfällen in Verbindung mit AIDS, Schlafstörungen (einschließlich Tagesrhythmusstörungen, Schlaflosigkeit und Narkolepsie), Ticks (z.B. Giles-de-la-Tourette-Syndrom), traumatischen Gehimverletzungen, Tinnitus, Neuralgie, insbesondere trigeminaler Neuralgie, neuropathischen Schmerzen, Zahnschmerzen, Schmerzen als Folge von Krebserkrankungen, unmäßiger neuronaler Aktivität, die zu Neurodysthesie führt, bei Erkrankungen wie Diabetes, Multipler Sklerose (MS) und Erkrankungen der motorischen Nerven, Ataxie, muskulärer Rigidität (Spastizität), Unterkiefergelenks-Dysfunktion und amyotropher Lateralsklerose (ALS).

## Revendications

1. Composé de formule (I) ou un de ses sels ou produits de solvatation : 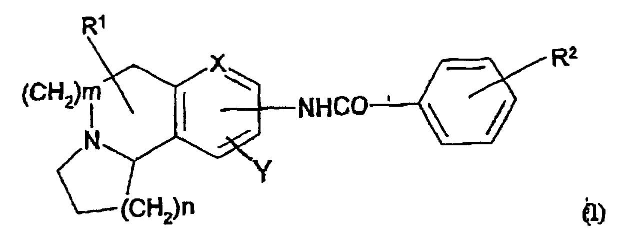 dans laquelle :
m est égal à 1 ou 2 ;
n est égal à 1 ou 2 ;
X représente un groupe CH ou N ;
Y est choisi entre un atome d'hydrogène, un atome d'halogène, un groupe cyano, CF₃, alkyle ou alkoxy ;
R¹, qui peut être à n'importe quelle position dans le système cyclique saturé, représente un atome d'hydrogène ou jusqu'à deux substituants qui peuvent être identiques ou différents, chacun étant choisi entre des substituants fluoro et alkyle en C₁ à C₆ ;
R² représente un atome d'hydrogène ou jusqu'à quatre substituants choisis entre des substituants halogéno, NO₂, CN, N₃, CF₃O-, CF₃S-, CF₃CO-, alkyle en C₁ à C₆, alcényle en C₁ à C₆, alcynyle en C₁ à C₆, perfluoralkyle en C₁ à C₆, cycloalkyle en C₃ à C₆, (cycloalkyle en C₃ à C₆) (alkyle en C₁ à C₄)-, (alkyle en C₁ à C₆)O-, (alkyle en C₁ à C₆)CO-, (cycloalkyle en C₃ à C₆)O-, (cycloalkyle en C₃ à C₆)CO-, (cycloalkyle en C₃ à C₆)(alkyle en C₁ à C₄)O-, (cycloaklyle en C₃ à C₆) (alkyle en C₁ à C₄)CO-, phényle, phénoxy, benzyloxy, benzoyle, phényl-(alkyle en C₁ à C₄)-; (alkyle en C₁ à C₆)S-, (alkyle en C₁ à C₆)SO₂-, (alkyle en C₁ à C₄)₂NSO₂-, (alkyle en C₁ à C₄)NHSO₂-, (alkyle en C₁ à C₄)₂NCO-, oxazolyle, (alkyle en C₁ à C₄)NHCO- et CONH₂ ;
ou un substituant R⁴CONH- ou -NR⁴R⁵
dans lequel R⁴ représente un atome d'hydrogène ou un groupe alkyle en C₁ à C₄ ; et
R⁵ représente un atome d'hydrogène, un groupe alkyle en C₁ à C₄, formyle, -CO₂(alkyle en C₁ à C₄) ou -CO(alkyle en C₁ à C₄) ;
ou bien deux groupes R² sont liés l'un à l'autre pour former un noyau carbocyclique ou hétérocyclique qui est saturé ou insaturé et substitué ou non substitué avec des substituants -OH ou =O.

2. Composé de formule (I) suivant la revendication 1, dans lequel :
R¹ représente un atome d'hydrogène, un groupe fluoro, méthyle, éthyle ou propyle ;
R² représente un atome d'hydrogène ou un ou plusieurs groupes méthyle, éthyle, n-butyle, phényle, isopropyle, tertio-butyle, méthoxy, éthoxy, n-propoxy, isopropoxy, n-butoxy, phénoxy, benzyloxy, bromo, chloro, iodo, fluoro, nitro, cyano, acétyle, pivaloyle, isobutyroyle, benzoyle, trifluorométhyle, trifluorométhoxy, trifluoracétyle, amino, acétylamino, méthylthio, oxazolo, méthylsulfonyle, n-propylsulfonyle, isopropylsulfonyle ou diméthylsulfamoyle.

3. Composé de formule (I) suivant la revendication 1 ou la revendication 2, dans lequel :
R¹ représente un atome d'hydrogène ; et
R² représente un atome d'hydrogène ou un ou plusieurs groupes éthyle, méthoxy, isopropoxy, trifluorométhyle, cyano, chloro ou fluoro.

4. Composé suivant l'une quelconque des revendications 1 à 3, choisi entre les suivants :
3-cyano-N-(1,2,3,5,6,10b-hexahydropyrrolo[2,1-a]isoquinoléine-9-yl)-4-méthoxybenzamide ;
3-brome-4-éthyl-N-(1,2,3,5,6,10b-hexahydropyrrolo[2,1-a]iso-quinoléine-9-yl)benzamide ;
N-(1,2,3,5,6,10b-hexahydropyrrolo[2,1-a]isoquinoléine-9-yl)-4-méthgoxy-3-trifluorométhylbenzamide ;
3-bromo-4-éthoxy-N-(1,2,3,5,6,10b-hexahydropyrrolo[2,1-a]isoquinoléine-9-yl)benzamide ;
3-bromo-4-éthoxy-N-(1,3,4,6,7,11b-hexahydro-2H-pyrido-[2,1-a]isoquinoléine-10-yl)benzamide ;
N-(1,2,3,5,6,10b-hexahydropyrrolo[2,1-a]isoquinoléine-9-yl)-4-méthoxy-3-propionylbenzamide ;
N-(1,2,3,5,6,10b-hexahydropyrrolo[2,1-a]isoquinoléine-9-yl)-4-éthoxy-3-propionylbenzamide ;
3-acétyl-N-(1,2,3,5,6,10b-hexahydropyrrolo[2,1-a]isoquinoléine-9-yl)benzamide ;
3-acétyl-N-(1,2,3,5,6,10b-hexahydropyrrolo[2,1-a]isoquinoléine-9-yl)-4-méthoxybenzamide ;
3-fluoro-N-(1,2,3,5,6,10b-hexahydropyrrolo[2,1-a]isoquinoléine-9-yl)-4-méthoxybenzamide ;
3,5-dichloro-4-éthoxy-N-(1,2,3,5,6,10b-hexahydropyrrolo[2,1-a]isoquinoléine-9-yl)benzamide ;
3-acétyl-N-(1,2,3,5,6,10b-hexahydropyrrolo[2,1-a]isoquinoléine-9-yl)-4-propoxybenzamide ;
3-butyryl-N-(1,2,3,5,6,10b-hexahydropyrrolo[2,1-a]isoquinoléine-9-yl)-4-méthoxybenzamide ;
3-isobutyryl-N-(1,2,3,5,6,10b-hexahydropyrrolo[2,1-a]-isoquinoléine-9-yl)-4-isopropoxybenzamide ;
3-cyano-N-(1,2,3,5,6,10b-hexahydropyrrolo[2,1-a]isoquinoléine-9-yl)-4-isopropylbenzamide ;
(1,2,3,5,6,10b-hexahydropyrrolo[2,1-a]isoquinoléine-9-yl)amide d'acide 4-oxo-chromane-6-carboxylique ;
N-(1,2,3,5,6,10b-hexahydropyrrolo[2,1-a]isoquinoléine-9-yl)-4-isopropoxy-3-propionylbenzamide ;
N-(1,2,3,5,6,10b-hexahydropyrrolo[2,1-a]isoquinoléine-9-yl)-3-isobutyryl-4-méthoxybenzamide ;
N-(1,2,3,5,6,10b-hexahydropyrrolo[2,1-a]isoquinoléine-9-yl)-4-éthoxy-3-isobutyrylbenzamide ;
N-(1,2,3,5,6,10b-hexahydropyrrolo[2,1-a]isoquinoléine-9-yl)-4-éthoxy-3-fluorobenzamide ;
N-(1,2,3,5,6,10b-hexahydropyrrolo[2,1-a]isoquinoléine-9-yl)-4-isopropoxy-3-fluorobenzamide ;
3-cyano-N-(1,2,3,5,6,10b-hexahydropyrrolo[2,1-a]isoquinoléine-9-yl)-4-isopropoxybenzamide ;
3-cyano-4-éthoxy-N-(1,2,3,5,6,10b-hexahydropyrrolo[2,1-a]isoquinoléine-9-yl)benzamide ;
N-(1,2,3,5,6,10b-hexahydropyrrolo[2,1-a]isoquinoléine-9-yl)-3-propionyl-4-propoxybenzamide ;
3-acétyl-4-éthyl-N-(1,2,3,5,6,10b-hexahydropyrrolo[2,1-a]isoquinoléine-9-yl)benzamide ;
3-acétyl-4-chloro-N-(1,2,3,5,6,10b-hexahydropyrrolo[2,1-a]isoquinoleine-9-yl)benzamide ;
3-acétyl-4-bromo-N-(1,2,3,5,6,10b-hexahydropyrrolo[2,1-a] isoquinoléine-9-yl)benzamide ;
3-acétyl-5-bromo-N-(1,2,3,5,6,10b-hexahydropyrrolo[2,1-a]isoquinoléine-9-yl)benzamide ;
3-cyano-4-éthoxy-N-(1,2,3,5,6,10b-hexahydropyrrolo[2,1-a]isoquinoléine-9-yl)benzamide ;
3-cyano-N-(1,2,3,5,6,10b-hexahydropyrrolo [2,1-a]-isoquinoléine-9-yl)-4-isopropoxybenzamide ;
3-acétyl-4-acétylamino-N-(1,2,3,5,6,10b-hexahydropyrrolo[2,1-a]isoquinoléine-9-yl)benzamide ;
3-acétyl-N-(1,2,3,5,6,10b-hexahydropyrrolo[2,1-a]-isoquinoléine-9-yl) -4-isopropylbenzamide ;
N-(1,2,3,5,6,10b-hexahydropyrrolo[2,1-a]isoquinoléine-9-yl)-3-propionyl-4-isopropoxybenzamids ;
3-acétyl-4-éthoxy-N-(1,2,3,5,6,10b-hexahydropyrrolo[2,1-a]isoquinoléine-9-yl)benzamide ;
3-cyano-N-(1,2,3,5,6,10b-hexahydropyrrolo[2,1-a]isoquinoléine-9-yl)-4-méthoxybenzamide ;
N-(1,2,3,5,6,10b-hexahydropyrrolo[2,1-a]isoquinoléine-9-yl)-4-méthoxy-3-trifluorométhylbenzamide ;
3-fluoro-N-(1,2,3,5,6,10b-hexahydropyrrolo[2,1-a]-isoquinoléine-9-yl)-4-méthoxybenzamide ;
4-éthoxy-3-fluoro-N-(1,2,3,5,6,10b-hexahydropyrrolo[2,1-a]isoquinoléine-9-yl)benzamide ;
3-butyryl-N-(1,2,3,5,6,10b-hexahydropyrrolo[2,1-a]-isoquinoléine-9-yl)-4-propoxybenzamide ;
3-butyryl-N-(1,2,3,5,6,10b-hexahydropyrrolo[2,1-a]-isoquinoléine-9-yl)-4-isopropoxybenzamide ;
3-acétyl-N-(1,2,3,5,6,10b-hexahydropyrrolo[2,1-a]-isoquinoléine-9-yl)-4-propoxybenzamide ;
3-butyryl-4-éthoxy-N-(1,2,3,5,6,10b-hexahydropyrrolo[2,1-a]isoquinoléine-9-yl)benzamide ;
3-isobutyryl-N-(1,2,3,5,6,10b-hexahydropyrrolo[2,1-a]isoquinoléine-9-yl)-4-propoxybenzamide ;
3-acétyl-N-(1,2,3,5,6,10b-hexahydropyrrolo[2,1-a]-isoquinoléine-9-yl)-4-isopropoxybenzamide ;
3-chloro-N-(1,2,3,5,6,10b-hexahydropyrrolo[2,1-a]-isoquinoléine-9-yl)-4-isopropoxybenzamide ;
3-cyano-N-(1,2,3,5,6,10b-hexahydropyrrolo[2,1-a]-isoquinoléine-9-yl)-4-isopropylbenzamide ;
3-bromo-N-(1,2,3,5,6,10b-hexahydropyrrolo[2,1-a]-isoquinoléine-9-yl)-4-isopropoxybenzamide ;
5-acétyl-N-(1,2,3,5,6,10b-hexahydropyrrolo[2,1-a]-isoquinoléine-9-yl)-2-méthoxy-4-isopropoxybenzamide ;
N-(1,2,3,5,6,10b-hexahydropyrrolo[2,1-a]isoquinoléine-9-yl)-3-pivaloylbenzamide ;
N-(1,2,3,5,6,10b-hexahydropyrrolo[2,1-a]isoquinoléine-9-yl)-2-méthoxy-4-isopropyl-5-trifluorométhylbenzamide ;
(1,2,3,5,6,10b-hexahydropyrrolo[2,1-a]isoquinoléine-9-yl)amide d'acide naphtalène-2-carboxylique ;
(1,2,3,5,6,10b-hexahydropyrrolo[2,1-a]isoquinoléine-9-yl)amide d'acide benzothiazole-5-carboxylique ;
(1,2,3,5,6,10b-hexahydropyrrolo[2,1-a]isoquinoléine-9-yl)carboxamide d'acide 2,3-dihydrobenzofuranne-5-carboxylique ;
3-acétyl-N-(1,2,3,5,6,10b-hexahydropyrrolo[2,1-a]-isoquinoléine-9-yl)-4-isopropoxybenzamide ;
3-chloro-4-éthoxy-N-(1,2,3,5,6,10b-hexahydropyrrolo-[2,1-a]isoquinoléine-9-yl)benzamlde ;
N-3-(N,N-diméthylcarboxamido)-(1,2,3,5,6,10b-hexahydropyrrolo[2,1-a]isoquinoléine-9-yl)-4-isopropoxybenzamide ;
N-(6,6-diméthyl-1,2,3,5,6,10b-hexahydropyrrolo[2,1-a]-isoquinoléine-9-yl)-4-méthoxy-3-trifluorométhylbenzamide ;
3-bromo-N- (6,6-diméthyl-1,2,3,5,6,10b-hexahydropyrrolo[2,1-a]isoquinoléine-9-yl)-4-éthoxybenzamide ;
3-bromo-N-(6,6-diméthyl-1,2,3,5,6,10b-hexahydropyrrolo[2,1-a]isoquinoléine-9-yl)-4-isopropoxybenzamide ;
3-cyano-N-(6,6-diméthyl-1,2,3,5,6,10b-hexahydropyrrolo[2,1-a]isoquinoléine-9-yl)-4-isopropylbenzamide ;
3-acétyl-N-(6,6-diméthyl-1,2,3,5,6,10b-hexahydropyrrolo[2,1-a]isoquinoléine-9-yl)-4-isopropoxybenzamide ;
N-(6,6-diméthyl-1,2,3,5,6,10b-hexahydropyrrolo[2,1-a]isoquinoléine-9-yl)-3-fluoro-4-méthoxybenzamide ;
N-(6,6-diméthyl-1,2,3,5,6,10b-hexahydropyrrolo[2,1-a]isoquinoléine-9-yl)-3-fluoro-éthoxybenzamide ;
N- (6,6-diméthyl-1,2,3,5,6,10b-hexahydropyrrolo[2,1-a]isoquinoléine-9-yl)-3-fluoro-4-isopropoxybenzamide ;
3-cyano-N-(6,6-diméthyl-1,2,3,5,6,10b-hexahydropyrrolo[2,1-a]isoquinoléine-9-yl)-4-méthoxybenzamide ;
3-acétyl-N-(6,6-diméthyl-1,2,3,5,6,10b-hexahydropyrrolo[2,1-a]isoquinoléine-9-yl)-4-méthoxybenzamide ;
N-(6,6-diméthyl-1,2,3,5,6,10b-hexahydropyrrolo[2,1-a]isoquinoléine-9-yl)-3-fluoro-4-méthoxybenzamide ;
N-(7-chloro-1,2,3,5,6,10b-hexahydropyrrolo[2,1-a]-isoquinoléine-9-yl)-4-méthoxy-3-trifluorométhylbenzamide ;
10b-méthyl-N-(1,2,3,5,6,10b-hexahydropyrrolo[2,1-a]-isoquinoléine-9-yl)-4-méthoxy-3-trifluorométhylbenzamide ;
3-bromo-N-4-éthoxy-(6-méthyl-1,2,3,5,6,10b-hexahydropyrrolo[2,1-a]isoquinoléine-9-yl)-4-isopropoxybenzamide ;
N-(6-méthyl-1,2,3,5,6,10b-hexahydropyrrolo[2,1-a]-isoquinoléine-9-yl)-4-méthoxy-3-trifluorométhylbenzamide ;
N-(5-méthyl-1,2,3,5,6,10b-hexahydropyrrolo[2,1-a]-isoquinoléine-9-yl)-4-méthoxy-3-trifluorométhylbenzamide ;
3-acétyl-N-(5-méthyl-1,2,3,5,6,10b-hexahydropyrrolo-[2,1-a]isoquinoléine-9-yl)-4-isopropoxybenzamide ;
3-acétyl-N-(2,3,5,6,7,11b-hexahydro-1H-benzo[c]-pyrrolo[1,2-a]azépine-10-yl)-4-isopropoxybenzamide ;
N-(2,3,5,6,7,11b-hexahydro-1H-benzo[c]pyrrolo[1,2-a]-azépine-10-yl)-4-méthoxy-3-trifluorométhylbenzamide ;
4-tertio-butyl-2-méthoxy-N-(1,2,3,5,6,10b-hexahydropyrrolo[2,1-a]isoquinoléine-7-yl)benzamide ;
5-cyano-2-éthoxy-4-isopropyl-(2,3,5,6,6a,10a,10b-octahydro-1H-pyrrolo[2,1-a]isoquinoléine-7-yl)benzamide ; et
N-(5,6,8,9,10,10a-hexahydropyrrolo[2,1-f][1,6]-naphthyridine-2-yl)-4-méthoxy-3-trifluorométhylbenzamide.

5. Composé de formule (I) suivant la revendication 1, choisi entre les suivants :
(+)-3-cyano-N-(1,2,3,5,6,10b-hexahydropyrrolo[2,1-a]-isoquinoléine-9-yl)-4-isopropoxybenzamide et
(+)-3-cyano-4-éthoxy-N-(1,2,3,5,6,10b-hexahydropyrrolo[2,1-a]isoquinoléine-9-yl)benzamide.

6. Procédé pour la préparation de composés de formule (I) répondant à la définition suivant la revendication 1, ou de leurs sels ou produits de solvatation, qui comprend la réaction d'un composé de formule (II) 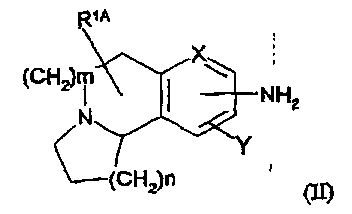 avec un composé de formule (III) 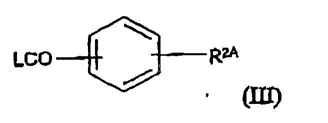 formules dans lesquelles R^{1A} et R^{2A} représentent, respectivement, des groupes R¹ et R² répondant aux définitions mentionnées pour la formule (I) telle que définie dans la revendication 1, ou bien un ou plusieurs groupes pouvant être convertis en groupes R¹ ou R² ; et L représente un groupe OH, acyloxy ou un atome d'halogène,
et, lorsque cela est requis ;
la conversion d'un groupe R^{1A} ou R^{2A} en un groupe R¹ ou R² ;
la conversion d'un groupe R¹ ou R² en un autre groupe R¹ ou R² ;
la conversion d'un produit consistant en un sel en la base libre ou un autre sel pharmaceutiquement acceptable ;
ou la conversion d'un produit consistant en base libre en un sel pharmaceutiquement acceptable.

7. Composition pharmaceutique destinée à être utilisée dans le traitement et/ou la prophylaxie de l'anxiété, de la manie, la dépression, de troubles du type de la panique et/ou de l'agression, de troubles associés à une hémorragie sous-arachnoïdienne ou un choc neural, des effets associés au sevrage de substances entraînant une dépendance telles que la cocaïne, la nicotine, l'alcool et les benzodiazépines, de troubles pouvant être traités et/ou prévenus avec des agents anticonvulsivants, tels que l'épilepsie y compris l'épilepsie post-traumatique, la maladie de Parkinson, la psychose, la migraine, l'ischémie cérébrale, la maladie d'Alzheimer et d'autres maladies dégénératives telles que la chorée de Huntington, la schizophrénie, des troubles obsessionnels compulsifs (TOC), les déficiences neurologiques associées au SIDA, des troubles du sommeil (comprenant des troubles du rythme circadien, l'insomnie et la narcolepsie), les tics (par exemple le syndrome de Gilles de la Tourette), une lésion cérébrale traumatique, les bourdonnements d'oreilles, les névralgies, notamment les névralgies du trijumeau, une douleur neuropathique, une douleur dentaire, une douleur provoquée par un cancer, une activité neuronale inappropriée ayant pour résultat des neurodysthésies dans des maladies telles que le diabète, la sclérose en plaques (SM) et une maladie neuronale motrice, des ataxies, la rigidité musculaire (spasticité), un dysfonctionnement de l'articulation temporomandibulaire et la sclérose latérale amyotrophique (ALS), qui comprend un composé de formule (I) répondant à la définition suivant l'une quelconque des revendications 1 à 5, ou un de ses sels ou produits de solvatation pharmaceutiquement acceptables, et un support pharmaceutiquement acceptable.

8. Composé de formule (I) répondant à la définition suivant l'une quelconque des revendications 1 à 5, ou un de ses sels ou produits de solvatation pharmaceutiquement acceptables, destiné à être utilisé comme agent thérapeutique, en particulier pour le traitement, et/ou la prophylaxie de l'anxiété, de la manie, la dépression, de troubles du type de la panique et/ou de l'agression, de troubles associés à une hémorragie sous-arachnoïdienne ou un choc neural, des effets associés au sevrage de substances entraînant une dépendance telles que la cocaïne, la nicotine, l'alcool et les benzodiazépines, de troubles pouvant être traités et/ou prévenus avec des agents anticonvulsivants, tels que l'épilepsie y compris l'épilepsie post-traumatique, la maladie de Parkinson, la psychose, la migraine, l'ischémie cérébrale, la maladie d'Alzheimer et d'autres maladies dégénératives telles que la chorée de Huntington, la schizophrénie, des troubles obsessionnels compulsifs (TOC), les déficiences neurologiques associées au SIDA, des troubles du sommeil (comprenant des troubles du rythme circadien, l'insomnie et la narcolepsie), les tics (par exemple le syndrome de Gilles de la Tourette), une lésion cérébrale traumatique, les bourdonnements d'oreilles, les névralgies, notamment les névralgies du trijumeau, une douleur neuropathique, une douleur dentaire, une douleur provoquée par un cancer, une activité neuronale inappropriée ayant pour résultat des neurodysthésies dans des maladies telles que le diabète, la sclérose en plaques (SM) et une maladie neuronale motrice, des ataxies, la rigidité musculaire (spasticipé), un dysfonctionnement de l'articulation temporomandibulaire et la sclérose latérale amyotrophique (ALS).

9. Utilisation d'un composé de formule (I) répondant à la définition suivant l'une quelconque des revendications 1 à 5, ou d'un de ses sels ou produits de solvatation pharmaceutiquement acceptables, pour la production d'un médicament destiné au traitement et/ou à la prophylaxie de l'anxiété, de la manie, la dépression, de troubles du type de la panique et/ou de l'agression, de troubles associés à une hémorragie sous-arachnoidienne ou un choc neural, des effets associés au sevrage de substances entraînant une dépendance telles que la cocaïne, la nicotine, l'alcool et les benzodiazépines, de troubles pouvant être traités et/ou prévenus avec des agents anticonvulsivants, tels que l'épilepsie y compris l'épilepsie post-traumatique, la maladie de Parkinson, la psychose, la migraine, l'ischémie cérébrale, la maladie d'Alzheimer et d'autres maladies dégénératives telles que la chorée de Huntington, la schizophrénie, des troubles obsessionnels compulsifs (TOC), les déficiences neurologiques associées au SIDA, des troubles du sommeil (comprenant des troubles du rythme circadien, l'insomnie et la narcolepsie), les tics (par exemple le syndrome de Gilles de la Tourette), une lésion cérébrale traumatique, les bourdonnements d'oreilles, les névralgies, notamment les névralgies du trijumeau, une douleur neuropathique, une douleur dentaire, une douleur provoquée par un cancer, une activité neuronale inappropriée ayant pour résultat des neurodysthésies dans des maladies telles que le diabète, la sclérose en plaques (SM) et une maladie neuronale motrice, des ataxies, la rigidité musculaire (spasticité), un dysfonctionnement de l'articulation temporomandibulaire et la sclérose latérale amyotrophique (ALS).
